# EUROPEAN PATENT SPECIFICATION

(11) **EP 0 846 114 B1**
(45) Date of publication and mention of the grant of the patent: **05.12.2001**
(21) Application number: 96914786.7
(22) Date of filing: 28.05.1996
(51) Int. Cl.: C07D 401/12, A61K 31/44

(54) **3-PYRIDYLOXYALKYL HETEROCYCLIC ETHER COMPOUNDS USEFUL IN CONTROLLING CHEMICAL SYNAPTIC TRANSMISSION**
3-PYRIDYLOXYALKYL HETEROZYKLISCHE ETHER-VERBINDUNGEN, VERWENDBAR ZUR STEUERUNG VON CHEMISCHER SYNAPTISCHER TRANSMISSION
COMPOSES D'ETHER HETEROCYCLIQUE DE 3-PYRIDYLOXYALKYLE UTILES POUR REGULER LA TRANSMISSION SYNAPTIQUE CHIMIQUE

(30) Priority: 07.06.1995 US 474873; 07.06.1995 US 485537
(43) Date of publication of application: 10.06.1998
(73) Proprietor: Abbott Laboratories, Abbott Park, Illinois 60064-3500 (US)
(72) Inventor: HOLLADAY, Mark, W., Libertyville, IL 60048 (US); LIN, Nan-Horng, Mundelein, IL 60060 (US); RYTHER, Keith, B., Round Lake Park, IL 60073 (US); HE, Yun, San Diego, CA 92121 (US)
(74) Representative: Modiano, Guido, Dr.-Ing.
(86) International application number: US9607804
(87) International publication number: WO9640682

(56) References cited:
- WO-A-94/08992

## Description

### Technical Field

This invention relates to 3-pyridyloxymethyl heterocyclic ether compounds which control chemical synaptic transmission; to therapeutically-effective pharmaceutical compositions of these compounds; and to the use of said compositions to selectively control synaptic transmission.

### Background of the Invention

Compounds that selectively control chemical synaptic transmission offer therapeutic utility in treating disorders that are associated with dysfunctions in synaptic transmission. This utility may arise from controlling either pre-synaptic or post-synaptic chemical transmission. The control of synaptic chemical transmission is, in turn, a direct result of a modulation of the excitability of the synaptic membrane. Presynaptic control of membrane excitability results from the direct effect an active compound has upon the organelles and enzymes present in the nerve terminal for synthesizing, storing, and releasing the neurotransmitter, as well as the process for active re-uptake. Postsynaptic control of membrane excitability results from the influence an active compound has upon the cytoplasmic organelles that respond to neurotransmitter action.

An explanation of the processes involved in chemical synaptic transmission will help to illustrate more fully the potential applications of the invention. (For a fuller explanation of chemical synaptic transmission refer to Hoffman *et al.,* "Neurotransmission: The autonomic and somatic motor nervous systems." In: Goodman and Gilman's, The Pharmacological Basis of Therapeutics, 9th ed., J.G. Hardman, L.E. Limbird, P.B. Molinoff, R.W. Ruddon, and A. Goodman Gilman, eds., Pergamon Press, New York, **1996**, pp. 105-139).

Typically, chemical synaptic transmission begins with a stimulus that depolarizes the transmembrane potential of the synaptic junction above the threshold that elicits an all-or-none action potential in a nerve axon. The action potential propagates to the nerve terminal where *ion fluxes* activate a mobilization process leading to neurotransmitter secretion and "transmission" to the postsynaptic cell. Those cells which receive communication from the central and peripheral nervous systems in the form of neurotransmitters are referred to as "excitable cells." Excitable cells are cells such as nerves, smooth muscle cells, cardiac cells and glands. The effect of a neurotransmitter upon an excitable cell may be to cause either an excitatory or an inhibitory postsynaptic potential (EPSP or IPSP, respectively) depending upon the nature of the postsynaptic receptor for the particular neurotransmitter and the extent to which other neurotransmitters are present. Whether a particular neurotransmitter causes excitation or inhibition depends principally on the ionic channels that are opened in the postsynaptic membrane (i.e., in the excitable cell).

EPSPs typically result from a local depolarization of the membrane due to a generalized increased permeability to cations (notably Na⁺ and K⁺), whereas IPSPs are the result of stabilization or hyperpolarization of the membrane excitability due to a increase in permeability to primarily smaller ions (including K⁺ and Cl-). For example, the neurotransmitter acetylcholine excites at skeletal muscle junctions by opening permeability channels for Na⁺ and K⁺. At other synapses, such as cardiac cells, acetylcholine can be inhibitory, primarily resulting from an increase in K⁺ conductance.

The biological effects of the compounds of the present invention result from modulation of a particular subtype of acetylcholine receptor. It is, therefore, important to understand the differences between two receptor subtypes. The two distinct subfamilies of acetylcholine receptors are defined as nicotinic acetylcholine receptors and muscarinic acetylcholine receptors. (See Goodman and Gilman's, The Pharmacological Basis of Therapeutics, *op*. *cit*.).

The responses of these receptor subtypes are mediated by two entirely different classes of second messenger systems. When the nicotinic acetylcholine receptor is activated, the response is an increased flux of specific extracellular ions (e.g. Na⁺, K⁺ and Ca⁺⁺) through the neuronal membrane. In contrast, muscarinic acetylcholine receptor activation leads to changes in intracellular systems that contain complex molecules such as G-proteins and inositol phosphates. Thus, the biological consequences of nicotinic acetylcholine receptor activation are distinct from those of muscarinic receptor activation. In an analogous manner, inhibition of nicotinic acetylcholine receptors results in still other biological effects, which are distinct and different from those arising from muscarinic receptor inhibition.

As indicated above, the two principal sites to which drug compounds that affect chemical synaptic transmission may be directed are the presynaptic nerve terminal and the postsynaptic membrane. Actions of drugs directed to the presynaptic site may be mediated through presynaptic receptors that respond to the neurotransmitter which the same secreting structure has released (i.e., through an autoreceptor), or through a presynaptic receptor that responds to another neurotransmitter (i.e., through a heteroreceptor). Actions of drugs directed to the postsynaptic membrane mimic the action of the endogenous neurotransmitter or inhibit the interaction of the endogenous neurotransmitter with a postsynaptic receptor.

Classic examples of drugs that modulate postsynaptic membrane excitability are the neuromuscular blocking agents which interact with nicotinic acetylcholine-gated channel receptors on skeletal muscle, for example, competitive (stabilizing) agents, such as curare, or depolarizing agents, such as succinylcholine.

In the central nervous system, postsynaptic cells can have many neurotransmitters impinging upon them. This makes it difficult to know the precise net balance of chemical synaptic transmission required to control a given cell. Nonetheless, by designing compounds that selectively affect only one pre- or postsynaptic receptor, it is possible to modulate the net balance of all the other inputs. Obviously, the more that is understood about chemical synaptic transmission in CNS disorders, the easier it would be to design drugs to treat such disorders.

Knowing how specific neurotransmitters act in the CNS allows one to speculate about the disorders that may be treatable with certain CNS-active drugs. For example, dopamine is widely recognized as an important neurotransmitter in the central nervous systems in humans and animals. Many aspects of the pharmacology of dopamine have been reviewed by Roth and Elsworth, "Biochemical Pharmacology of Midbrain Dopamine Neurons", In: Psychopharmacology: The Fourth Generation of Progress, F.E. Bloom and D.J. Kupfer, Eds., Raven Press, NY, **1995**, pp 227-243). Patients with Parkinson's disease have a primary loss of dopamine containing neurons of the nigrostriatal pathway, which results in profound loss of motor control. Therapeutic strategies to replace the dopamine deficiency with dopamine mimetics, as well as administering pharmacologic agents that modify dopamine release and other neurotransmitters have been found to have therapeutic benefit ("Parkinson's Disease", In: Psychopharmacology: The Fourth Generation of Progress, *op. cit*, pp 1479-1484).

New and selective neurotransmitter controlling agents are still being sought, in the hope that one or more will be useful in important, but as yet poorly controlled, disease states or behavior models. For example, dementia, such as is seen with Alzheimer's disease or Parkinsonism, remains largely untreatable. Symptoms of chronic alcoholism and nicotine withdrawal involve aspects of the central nervous system, as does the behavioral disorder Attention-Deficit Disorder (ADD). Specific agents for treatment of these and related disorders are few in number or non-existent

A more complete discussion of the possible utility as CNS-active agents of compounds with activity as cholinergic ligands selective for neuronal nicotinic receptors, (*i.e*., for controlling chemical synaptic transmission) may be found in U.S. Patent 5,472,958, to Gunn et al., issued Dec. 5, 1995.

Existing acetylcholine agonists are therapeutically sub-optimal in treating the conditions discussed above. For example, such compounds have unfavorable pharmacokinetics (e.g., arecoline and nicotine), poor potency and lack of selectivity (e.g., nicotine), poor CNS penetration (e.g., carbachol) or poor oral bioavailability (e.g., nicotine). In addition, other agents have many unwanted central agonist actions, including hypothermia, hypolocomotion and tremor and peripheral side effects, including miosis, lachrymation, defecation and tachycardia (Benowitz *et al.,* in: Nicotine Psychopharmacology, S. Wonnacott, M.A.H. Russell, & I.P. Stolerman, eds., Oxford University Press, Oxford, **1990,** pp. 112-157; and M. Davidson, *et al*., in Current Research in Alzheimer Therapy, E. Giacobini and R. Becker, ed.; Taylor & Francis: New York, **1988**; pp 333-336).

Various heterocyclic 2-pyrrolidinyloxy-substituted compounds with analgesic and hypotensive activities have been disclosed by Scheffler *et al.* (U.S. Patent 4,643,995) and Tomioka *et al.* (*Chem*. *Pharm. Bull,* 38:2133-5, **1990**).

Certain other 2-pyridyloxy-substituted compounds are disclosed *inter alia* by Engel *et al.* in U.S. Patent 4,946,836 as having analgesic activity.

Various other compounds having a pyrrolidine or azetidine moiety substituted at the 3-position with a heterocycloxy group have also been disclosed (*cf*. U.S.Patents 4,592,866 to A.D. Cale; 4,705,853 to A.D. Cale; 4,956,359 to Taylor *et al*.; and 5,037,841 to Schoehe *et al.* and European patent application EP296560A2, to Sugimoto *et al*.).

Certain nicotine-related compounds having utility in enhancing cognitive function have been reported by Lin in U.S. Patent 5,278,176, issued Jan. 11, 1994. Also, 2-(nitro)phenoxy compounds with similar function have been reported by Gunn *et al.,* U.S. Patent 5,472,958, issued Dec. 5, 1995.

In the PCT Patent Application WO94 08992 of Abreo *et al.,* published April 28, 1994, are disclosed, *inter alia,* various 3-pyridyloxy-heterocyclic compounds that are either unsubstituted or mono-substituted on the pyridine ring with groups such as Br, Cl, F, hydroxyl, C₁-C₃-alkyl or C₁-C₃-alkoxy, such compounds also described as having utility in enhancing cognitive function.

### Summary of the Invention

It has been found, in accordance with the present invention, that a class of 3-pyridyloxyalkyl heterocyclic ether compounds are selective and potent neuronal nicotinic cholinergic compounds useful in controlling synaptic transmission.

In its principal aspect, the present invention provides a compound or a pharmaceutically acceptable salt or ester thereof having the formula **I** 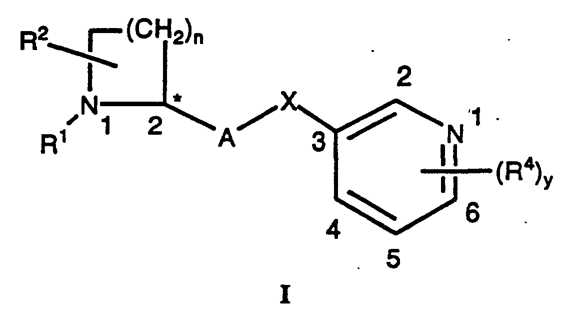 where the asterisk denotes a chiral center, and n is an integer selected from 1, 2, or 3.

**X** is oxygen or sulfur, and **R**^{**1**} is selected from the group consisting of hydrogen, allyl, and C₁-C₆-alkyl.

The substituent **R**^{**2**} is hydrogen or, when n=2, is also a single substituent selected from the group consisting of -CH₂OH, -CH₂F, -CH₂CN, -CH₂OCH₃, -Br, -Cl, -F, -OH, -CN, -(C₁-C₃ alkoxyl), -OCOCH₃, and O-methanesulfonyl, with the proviso that when R² is substituted at the 3-position or the 5-position of the pyrrolidinyl ring, it is a C₁-C₃-alkyl group.

The group A is selected from the group consisting of 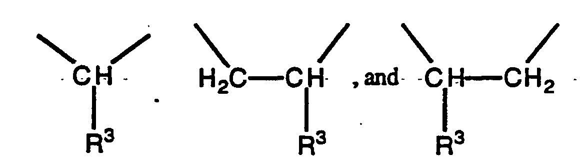 where R³ is H or C₁-C₆-alkyl;
y is 2 and R⁴ is substituted at the 2,5-, 2,6- or 5,6- positions of the pyridine ring wherein the 2-position substituent is selected from the group consisting of -Br, -Cl, -F, -OH, -(C₁-C₄ alkyl) and -(C₁-C₃ alkoxy) and the substituents at the 5- or 6-positions of the pyridine ring are selected from the group consisting of -Br, -Cl, -F, -OH, -(C₁-C₄ alkyl), -CN, -CF₃, -NO₂, -CH₂OH, -CH₂CN, -(C₁-C₃ alkoxy), -NH₂, -NH-CHO, -NHCO(C₁-C₃ alkyl), -N(C₁-C₃ alkyl)CO(C₁-C₃ alkyl), -NH-(C₁-C₃ alkyl), N(C₁-C₃ alkyl)₂, -COOH, -COO(C₁-C₃ alkyl), -CONH₂, -CONH-(C₁-C₃ alkyl), -CONHbenzyl, and -OCO(C₁-C₃ alkyl).

In another aspect, the present invention provides a pharmaceutical composition for selectively controlling synaptic transmission comprising a pharmaceutically acceptable carrier and a therapeutically effective amount of a compound of Formula (I) below.

In still another aspect, the present invention provides a method for selectively controlling synaptic transmission comprising administering to a mammal in need of such treatment a therapeutically effective amount of a compound of Formula (I) below.

### Detailed Description of the Invention

In one preferred embodiment, compounds of the present invention comprise a class of substituted azetidine compounds of Formula (I) above wherein n=1.

In another preferred embodiment, compounds of the present invention comprise a class of substituted pyrrolidine compounds of Formula (I) above wherein n=2.

In yet another preferred embodiment, compounds of the present invention comprise a class of substituted piperidine compounds of Formula (I) above wherein n=3.

In a further preferred embodiment of the invention, the group X in compounds of Formula (I) above is oxygen.

In yet another preferred embodiment of the present invention, the group X in compounds of Formula (I) above is sulfur.

In still further preferred embodiments of the present invention, the group A in compounds of Formula I above is selected from the group consisting of 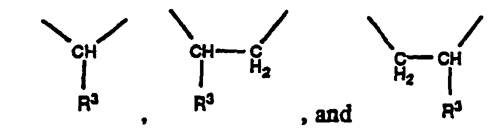 and R³ is as defined above.

In a particularly preferred embodiment of the present invention, n=0 or 1,
X=oxygen,
A is 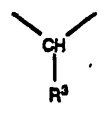 and R³ is H in compounds of Formula (I) above.

Representative substituted azetidine compounds falling within the scope of the present invention where n in the generic formula **I** is equal to one include:
5-bromo-6-chloro-3-(2-(S)-azetidinylmethoxy)pyridine;
5-bromo-6-chloro-3-(1-methyl-2-(S)-azetidinylmethoxy)pyridine;
5,6-dichloro-3-(2-(S)-azetidinylmethoxy)pyridine;
5,6-dichloro-3-(1-methyl-2-(S)-azetidinylmethoxy)pyridine;
5,6-dichloro-3-(2-(R)-azetidinylmethoxy)pyridine;
5,6-dichloro-3-(1-methyl-2-(R)-azetidinylmethoxy)pyridine;
5-ethyl-6-chloro-3-(2-(S)-azetidinylmethoxy)pyridine;
5-cyano-6-chloro-3-(2-(S)-azetidinylmethoxy)pyridine;
5-cyano-6-chloro-3-(1-methyl-2-(S)-azetidinylmethoxy)pyridine;
5-carbamoyl-6-chloro-3-(1-methyl-2-(S)-azetidinylmethoxy)pyridine;
6-fluoro-5-methyl-3-(2-(S)-azetidinylmethoxy)pyridine;
6-chloro-5-methyl-3-(2-(S)-azetidinylmethoxy)pyridine;
5-bromo-6-fluoro-3-(2-(S)-azetidinylmethoxy)pyridine;
5-methoxy-6-chloro-3-(2-(S)-azetidinylmethoxy)pyridine;
5-ethoxy-6-chloro-3-(2-(S)-azetidinylmethoxy)pyridine;
5-nitro-6-methyl-3-(2-(S)-azetidinylmethoxy)pyridine;
5,6-dimethyl-3-(2-(S)-azetidinylmethoxy)pyridine;
5-nitro-6-methyl-3-(2-(R)-azetidinylmethoxy)pyridine;
5-nitro-6-methyl-3-(1-methyl-2-(S)-azetidinylmethoxy)pyridine;
5-nitro-6-methyl-3-(1-methyl-2-(R)-azetidinylmethoxy)pyridine;
5-amino-6-methyl-3-(2-(S)-azetidinylmethoxy)pyridine;
5-amino-6-methyl-3-(2-(R)-azetidinylmethoxy)pyridine;
5-amino-6-methyl-3-(1-methyl-2-(S)-azetidinylmethoxy)pyridine;
5-amino-6-methyl-3-(1-methyl-2-(R)-azetidinylmethoxy)pyridine;
5-bromo-6-methyl-3-(2-(S)-azetidinylmethoxy)pyridine;
5-bromo-6-methyl-3-(2-(R)-azetidinylmethoxy)pyridine;
5-bromo-6-methyl-3-(1-methyl-2-(R)-azetidinylmethoxy)pyridine;
5-bromo-6-fluoro-3-(2-(R)-azetidinylmethoxy)pyridine;
5-bromo-6-fluoro-3-(1-methyl-2-(S)-azetidinylmethoxy)pyridine;
5-bromo-6-fluoro-3-(1-methyl-2-(R)-azetidinylmethoxy)pyridine;
5-Bromo-6-chloro-3-(2-(R)-azetidinylmethoxy)pyridine;
5-Bromo-6-chloro-3-(1-methyl-2-(R)-azetidinylmethoxy)pyridine;
5-cyano-6-methyl-3-(2-(S)-azetidinylmethoxy)pyridine;
5-cyano-6-methyl-3-(2-(R)-azetidinylmethoxy)pyridine;
5-cyano-6-methyl-3-(1-methyl-2-(S)-azetidinylmethoxy)pyridine;
5-cyano-6-methyl-3-(1-methyl-2-(R)-azetidinylmethoxy)pyridine;
5-cyano-6-fluoro-3-(2-(S)-azetidinylmethoxy)pyridine;
5-cyano-6-fluoro-3-(2-(R)-azetidinylmethoxy)pyridine;
5-cyano-6-fluoro-3-(1-methyl-2-(S)-azetidinylmethoxy)pyridine;
5-cyano-6-fluoro-3-(1-methyl-2-(R)-azetidinylmethoxy)pyridine;
5-cyano-6-chloro-3-(2-(R)-azetidinylmethoxy)pyridine;
5-cyano-6-chloro-3-(1-methyl-2-(R)-azetidinylmethoxy)pyridine;
5-carbamoyl-6-methyl-3-(2-(S)-azetidinylmethoxy)pyridine;
5-carbamoyl-6-methyl-3-(2-(R)-azetidinylmethoxy)pyridine;
5-carbamoyl-6-methyl-3-(1-methyl-2-(S)-azetidinylmethoxy)pyridine;
5-carbamoyl-6-methyl-3-(1-methyl-2-(R)-azetidinylmethoxy)pyridine;
5-carbamoyl-6-fluoro-3-(2-(S)-azetidinylmethoxy)pyridine;
5-carbamoyl-6-fluoro-3-(2-(R)-azetidinylmethoxy)pyridine;
5-carbamoyl-6-fluoro-3-(1-methyl-2-(S)-azetidinylmethoxy)pyridine;
5-carbamoyl-6-fluoro-3-(1-methyl-2-(R)-azetidinylmethoxy)pyridine;
5-carbamoyl-6-chloro-3-(2-(S)-azetidinylmethoxy)pyridine;
5-carbamoyl-6-chloro-3-(2-(R)-azetidinylmethoxy)pyridine;
5-carbamoyl-6-chloro-3-(1-methyl-2-(R)-azetidinylmethoxy)pyridine;
5-amino-6-fluoro-3-(2-(S)-azetidinylmethoxy)pyridine;
5-amino-6-fluoro-3-(2-(R)-azetidinylmethoxy)pyridine;
5-amino-6-fluoro-3-(1-methyl-2-(S)-azetidinylmethoxy)pyridine;
5-amino-6-fluoro-3-(1-methyl-2-(R)-azetidinylmethoxy)pyridine;
5-amino-6-chloro-3-(2-(S)-azetidinylmethoxy)pyridine;
5-amino-6-chloro-3-(2-(R)-azetidinylmethoxy)pyridine;
5-amino-6-chloro-3-(1-methyl-2-(S)-azetidinylmethoxy)pyridine;
5-amino-6-chloro-3-(1-methyl-2-(R)-azetidinylmethoxy)pyridine;
5-formamido-6-methyl-3-(2-(S)-azetidinylmethoxy)pyridine;
5-formamido-6-methyl-3-(2-(R)-azetidinylmethoxy)pyridine;
5-formamido-6-methyl-3-(1-methyl-2-(S)-azetidinylmethoxy)pyridine;
5-formamido-6-methyl-3-(1-methyl-2-(R)-azetidinylmethoxy)pyridine;
5-acetamido-6-fluoro-3-(2-(S)-azetidinylmethoxy)pyridine;
5-acetamido-6-fluoro-3-(2-(R)-azetidinylmethoxy)pyridine;
5-acetamido-6-fluoro-3-(1-methyl-2-(S)-azetidinylmethoxy)pyridine;
5-acetamido-6-fluoro-3-(1-methyl-2-(R)-azetidinylmethoxy)pyridine;
5-acetamido-6-chloro-3-(2-(S)-azetidinylmethoxy)pyridine;
5-acetamido-6-chloro-3-(2-(R)-azetidinylmethoxy)pyridine;
5-acetamido-6-chloro-3-(1-methyl-2-(S)-azetidinylmethoxy)pyridine;
5-acetamido-6-chloro-3-(1-methyl-2-(R)-azetidinylmethoxy)pyridine;
5-methylamino-6-methyl-3-(2-(S)-azetidinylmethoxy)pyridine;
5-methylamino-6-methyl-3-(2-(R)-azetidinylmethoxy)pyridine;
5-methylamino-6-methyl-3-(1-methyl-2-(S)-azetidinylmethoxy)pyridine;
5-methylamino-6-methyl-3-(1-methyl-2-(R)-azetidinylmethoxy)pyridine;
5-ethylamino-6-fluoro-3-(2-(S)-azetidinylmethoxy)pyridine;
5-ethylamino-6-fluoro-3-(2-(R)-azetidinylmethoxy)pyridine;
5-ethylamino-6-fluoro-3-(1-methyl-2-(S)-azetidinylmethoxy)pyridine;
5-ethylamino-6-fluoro-3-(1-methyl-2-(R)-azetidinylmethoxy)pyridine;
5-ethylamino-6-chloro-3-(2-(S)-azetidinylmethoxy)pyridine;
5-ethylamino-6-chloro-3-(2-(R)-azetidinylmethoxy)pyridine;
5-ethylamino-6-chloro-3-(1-methyl-2-(S)-azetidinylmethoxy)pyridine;
5-ethylamino-6-chloro-3-(1-methyl-2-(R)-azetidinylmethoxy)pyridine;
5-ethyl-6-methyl-3-(2-(S)-azetidinylmethoxy)pyridine;
5-ethyl-6-methyl-3-(2-(R)-azetidinylmethoxy)pyridine;
5-ethyl-6-methyl-3-(1-methyl-2-(S)-azetidinylmethoxy)pyridine;
5-ethyl-6-methyl-3-(1-methyl-2-(R)-azetidinylmethoxy)pyridine;
5-ethyl-6-fluoro-3-(2-(S)-azetidinylmethoxy)pyridine;
5-ethyl-6-fluoro-3-(2-(R)-azetidinylmethoxy)pyridine;
5-ethyl-6-fluoro-3-(1-methyl-2-(S)-azetidinylmethoxy)pyridine;
5-ethyl-6-fluoro-3-(1-methyl-2-(R)-azetidinylmethoxy)pyridine;
5-propyl-6-chloro-3-(2-(S)-azetidinylmethoxy)pyridine;
5-propyl-6-chloro-3-(2-(R)-azetidinylmethoxy)pyridine;
5-propyl-6-chloror-3-(1-methyl-2-(S)-azetidinylmethoxy)pyridine;
5-propyl-6-chloro-3-(1-methyl-2-(R)-azetidinylmethoxy)pyridine;
5-methyl-6-fluoro-3-(2-(R)-azetidinylmethoxy)pyridine;
5-methyl-6-fluoro-3-(1-methyl-2-(S)-azetidinylmethoxy)pyridine;
5-methyl-6-fluoro-3-(1-methyl-2-(R)-azetidinylmethoxy)pyridine;
5-methyl-6-chloro-3-(2-(R)-azeddinylmethoxy)pyridine;
5-methyl-6-chloro-3-(1-methyl-2-(S)-azetidinylmethoxy)pyridine;
5-methyl-6-chloro-3-(1-methyl-2-(R)-azetidinylmethoxy)pyridine;
5,6-dimethyl-3-(2-(R)-azetidinylmethoxy)pyridine;
5,6-dimethyl-3-(1-methyl-2-(S)-azetidinylmethoxy)pyridine;
5,6-dimethyl-3-(1-methyl-2-(R)-azetidinylmethoxy)pyridine;
6-fluoro-5-methoxy-3-(2-(S)-azetidinylmethoxy)pyridine;
6-fluoro-5-methoxy-3-(2-(R)-azetidinylmethoxy)pyridine;
6-fluoro-5-methoxy-3-(1-methyl-2-(S)-azetidinylmethoxy)pyridine;
6-fluoro-5-methoxy-3-(1-methyl-2-(R)-azetidinylmethoxy)pyridine;
6-fluoro-5-ethoxy-3-(2-(S)-azetidinylmethoxy)pyridine;
6-fluoro-5-ethoxy-3-(2-(R)-azetidinylmethoxy)pyridine;
6-fluoro-5-ethoxy-3-(1-methyl-2-(S)-azetidinylmethoxy)pyridine; and
6-fluoro-5-ethoxy-3-(1-methyl-2-(R)-azetidinylmethoxy)pyridine; or a pharmaceutically acceptable salt or ester thereof.

Preferred substituted azetididine compounds of the present invention include:
5-bromo-6-chloro-3-(2-(S)-azetidinylmethoxy)pyridine;
5-bromo-6-chloro-3-(1-methyl-2-(S)-azetidinylmethoxy)pyridine;
5,6-dichloro-3-(2-(S)-azetidinylmethoxy)pyridine;
5,6-dichloro-3-(1-methyl-2-(S)-azetidinylmethoxy)pyridine;
5,6-dichloro-3-(2-(R)-azetidinylmethoxy)pyridine;
5,6-dichloro-3-(1-methyl-2-(R)-azetidinylmethoxy)pyridine;
5,6-Dichloro-3-(2-(S)-azetidinylmethoxy)pyridine; and
5,6-Dichloro-3-(1-methyl-2-(S)-azetidinylmethoxy)pyridine;
5-ethyl-6-chloro-3-(2-(S)-azetidinylmethoxy)pyridine;
5-cyano-6-chloro-3-(2-(S)-azetidinylmethoxy)pyridine;
5-cyano-6-chloro-3-(1-methyl-2-(S)-azetidinylmethoxy)pyridine;
5-carbamoyl-6-chloro-3-(1-methyl-2-(S)-azetidinylmethoxy)pyridine;
5-bromo-6-methyl-3-(2-(S)-azetidinylmethoxy)pyridine;
   and
6-fluoro-5-methyl-3-(2-(S)-azetidinylmethoxy)pyridine; or a pharmaceutically acceptable salt or ester thereof.

Representative substituted pyrrolidine compounds falling within the scope of the present invention where n in the generic formula **I** above is equal to two include:
5-bromo-6-chloro-3-(2-(S)-pyrrolidinylmethoxy)pyridine;
5-bromo-6-chloro-3-(1-methyl-2-(S)-pyrrolidinylmethoxy)pyridine;
5-bromo-6-chloro-3-(2-(R)-pyrrolidinylmethoxy)pyridine;
5-bromo-6-chloro-3-(1-methyl-2-(R)-pyrrolidinylmethoxy)pyridine;
5,6-dichloro-3-(2-(R)-pyrrolidinylmethoxy)pyridine;
5,6-dichloro-3-(1-methyl-2-(R)-pyrrolidinylmethoxy)pyridine;
5,6-dichloro-3-(2-(S)-pyrrolidinylmethoxy)pyridine;
5,6-dichloro-3-(1-methyl-2-(S)-pyrrolidinylmethoxy)pyridine;
5-ethyl-6-chloro-3-(2-(S)-pyrrolidinylmethoxy)pyridine;
5-ethyl-6-chloro-3-(1-methyl-2-(S)-pyrrolidinylmethoxy)pyridine;
5-ethyl-6-chloro-3-(2-(R)-pyrrolidinylmethoxy)pyridine;
5-nitro-6-methyl-3-(2-(S)-pyrrolidinylmethoxy)pyridine;
5-nitro-6-methyl-3-(2-(R)-pyrrolidinylmethoxy)pyridine;
5-nitro-6-mahyl-3-(1-methyl-2-(S)-pyrrolidinylmethoxy)pyridine;
5-nitro-6-methyl-3-(1-methyl-2-(R)-pyrrolidinylmethoxy)pyridine;
5-amino-6-methyl-3-(2-(S)-pyrrolidinylmethoxy)pyridine;
5-amino-6-melyl-3-(2-(R)-pyrrolidinylmethoxy)pyridine;
5-amino-6-methyl-3-(1-methyl-2-(S)-pyrrolidinylmethoxy)pyridine;
5-amino-6-methyl-3-(1-methyl-2-(R)-pyrrolidinylmethoxy)pyridine;
5-bromo-6-methyl-3-(2-(S)-pyrrolidinylmethoxy)pyridine;
5-bromo-6-methyl-3-(2-(R)-pyrrolidinylmethoxy)pyridine;
5-bromo-6-methyl-3-(1-methyl-2-(S)-pyrrolidinylmethoxy)pyridine;
5-bromo-6-methyl-3-(1-methyl-2-(R)-pyrrolidinylmethoxy)pyridine;
5-bromo-6-fluoro-3-(2-(S)-pyrrolidinylmethoxy)pyridine;
5-bromo-6-fluoro-3-(2-(R)-pyrrofidinylmethoxy)pyridine;
5-bromo-6-fluoro-3-(1-methyl-2-(S)-pyrrolidinylmethoxy)pyridine;
5-bromo-6-fluero-3-(1-methyl-2-(R)-pyrrolidinylmethoxy)pyridine;
5-cyano-6-methyl-3-(2-(S)-pyrrolidinylmethoxy)pyridine;
5-cyano-6-methyl-3-(2-(R)-pyrrolidinylmethoxy)pyridine;
5-cyano-6-methyl-3-(1-methyl-2-(S)-pyrrolidinylmethoxy)pyridine;
5-cyano-6-methyl-3-(1-methyl-2-(R)-pyrrolidinylmethoxy)pyridine;
5-cyano-6-fluoro-3-(2-(S)-pyrrolidinylmethoxy)pyridine;
5-cyano-6-fluoro-3-(2-(R)-pyrrolidinylmethoxy)pyridine;
5-cyano-6-fluoro-3-(1-methyl-2-(S)-pyrrolidinylmethoxy)pyridine;
5-cyano-6-fluoro-3-(1-methyl-2-(R)-pyrrolidinylmethoxy)pyridine;
5-cyano-6-chloro-3-(2-(S)-pyrrolidinylmethoxy)pyridine;
5-cyano-6-chloro-3-(2-(R)-pyrrolidinylmethoxy)pyridine;
5-cyano-6-chloro-3-(1-methyl-2-(S)-pyrrolidinylmethoxy)pyridine;
5-cyano-6-chloro-3-(1-methyl-2-(R)-pyrrolidinylmethoxy)pyridine;
5-carbamoyl-6-methyl-3-(2-(S)-pyrrolidinylmethoxy)pyridine;
5-carbamoyl-6-methyl-3-(2-(R)-pyrrolidinylmethoxy)pyridine;
5-carbamoyl-6-methyl-3-(1-methyl-2-(S)-pyrrolidinylmethoxy)pyridine;
5-carbamoyl-6-methyl-3-(1-methyl-2-(R)-pyrrolidinylmethoxy)pyridine;
5-carbamoyl-6-fluoro-3-(2-(S)-pyrrolidinylmethoxy)pyridine;
5-carbamoyl-6-fluoro-3-(2-(R)-pyrrolidinylmethoxy)pyridine;
5-carbamoyl-6-fluoro-3-(1-methyl-2-(S)-pyrrolidinylmethoxy)pyridine;
5-carbamoyl-6-fluoro-3-(1-methyl-2-(R)-pyrrolidinylmethoxy)pyridine;
5-carbamoyl-6-chloro-3-(2-(S)-pyrrolidinylmethoxy)pyridine;
5-carbamoyl-6-chloro-3-(2-(R)-pyrrolidinylmethoxy)pyridine;
5-carbamoyl-6-chloro-3-(1-methyl-2-(R)-azetidinylmethoxy)pyridine;
5-carbamoyl-6-chloro-3-(1-methyl-2-(S)-pyrrolidinylmethoxy)pyridine;
5-carbamoyl-6-chloro-3-(1-methyl-2-(R)-pyrrolidinylmethoxy)pyridine:
5-amino-6-fluoro-3-(2-(S)-pyrrolidinylmethoxy)pyridine;
5-amino-6-fluoro-3-(2-(R)-pyrrolidinylmethoxy)pyridine;
5-amino-6-fluoro-3-(1-methyl-2-(S)-pyrrolidinylmethoxy)pyridine;
5-amino-6-fluoro-3-(1-methyl-2-(R)-pyrrolidinylmethoxy)pyridine;
5-amino-6-chloro-3-(2-(S)-pyrrolidinylmethoxy)pyridine;
5-amino-6-chloro-3-(2-(R)-pyrrolidinylmethoxy)pyridine;
5-amino-6-chloro-3-(1-methyl-2-(S)-pyrrolidinylmethoxy)pyridine;
5-amino-6-chloro-3-(1-methyl-2-(R)-pyrrolidinylmethoxy)pyridine;
5-formamido-6-methyl-3-(2-(S)-pyrrolidinylmethoxy)pyridine;
5-formamido-6-methyl-3-(2-(R)-pyrrolidinylmethoxy)pyridine;
5-formamido-6-methyl-3-(1-methyl-2-(S)-pyrrolidinylmethoxy)pyridine;
5-formamido-6-methyl-3-(1-methyl-2-(R)-pyrrolidinylmethoxy)pyridine;
5-acetamido-6-fluoro-3-(2-(S)-pyrrolidinylmethoxy)pyridine;
5-acetamido-6-fluoro-3-(2-(R)-pyrrolidinylmethoxy)pyridine;
5-acetamido-6-fluoro-3-(1-methyl-2-(S)-pyrrolidinylmethoxy)pyridine;
5-acetamido-6-fluoro-3-(1-methyl-2-(R)-pyrrolidinylmethoxy)pyridine;
5-acetamido-6-chloro-3-(2-(S)-pyrrolidinylmethoxy)pyridine;
5-acetamido-6-chloro-3-(2-(R)-pyrrolidinylmethoxy)pyridine;
5-acetamido-6-chloro-3-(1-methyl-2-(S)-pyrrolidinylmethoxy)pyridine;
5-acetamido-6-chloro-3-(1-methyl-2-(R)-pyrrolidinylmethoxy)pyridine;
5-methylamino-methyl-3-(2-(S)-pyrrolidinylmethoxy)pyridine;
5-methylamino-6-methyl-3-(2-(R)-pyrrolidinylmethoxy)pyridine;
5-methylamino-6-methyl-3-(1-methyl-2-(S)-pyrrolidinylmethoxy)pyridine;
5-methylamino-6-methyl-3-(1-methyl-2-(R)-pyrrolidinylmethoxy)pyridine;
5-ethylamino-6-fluoro-3-(2-(S)-pyrrolidinylmethoxy)pyridine;
5-ethylamino-6-fluoro-3-(2-(R)-pyrrolidinylmethoxy)pyridine;
5-ethylamino-6-fluoro-3-(1-methyl-2-(S)-pyrrolidinylmethoxy)pyridine;
5-ethylamino-6-fluoro-3-(1-methyl-2-(R)-pyrrolidinylmethoxy)pyridine;
5-ethylamino-6-chloro-3-(2-(S)-pyrrolidinylmethoxy)pyridine;
5-ethylamino-6-chloro-3-(2-(R)-pyrrolidinylmethoxy)pyridine;
5-ethylamino-6-chloro-3-(1-methyl-2-(S)-pyrrolidinylmethoxy)pyridine;
5-ethylamino-6-chloro-3-(1-methyl-2-(R)-pyrrolidinylmethoxy)pyridine;
5-ethyl-6-methyl-3-(2-(S)-pyrrolidinylmethoxy)pyridine;
5-ethyl-6-methyl-3-(2-(R)-pyrrolidinylmethoxy)pyridine;
5-ethyl-6-methyl-3-(1-methyl-2-(S)-pyrrolidinylmethoxy)pyridine;
5-ethyl-6-methyl-3-(1-methyl-2-(R)-pyrrolidinylmethoxy)pyridine;
5-ethyl-6-fluoro-3-(2-(S)-pyrrolidinylmethoxy)pyridine;
5-ethyl-6-fluoro-3-(2-(R)-pyrrolidinylmethoxy)pyridine;
5-ethyl-6-fluoro-3-(1-methyl-2-(S)-pyrrolidinylmethoxy)pyridine;
5-ethyl-6-fluoro-3-(1-methyl-2-(R)-pyrrolidinylmethoxy)pyridine;
5-propyl-6-chloro-3-(2-(S)-pyrrolidinylmethoxy)pyridine;
5-propyl-6-chloro-3-(2-(R)-pyrrolidinylmethoxy)pyridine;
5-propyl-6-chloro-3-(1-methyl-2-(S)-pyrrolidinylmethoxy)pyridine;
5-propyl-6-chloro-3-(1-methyl-2-(R)-pyrrolidinylmethoxy)pyridine;
5-methyl-6-fluoro-3-(2-(S)-pyrrolidinylmethoxy)pyridine;
5-methyl-6-fluoro-3-(2-(R)-pyrrolidinylmethoxy)pyridine;
5-methyl-6-fluoro-3-(1-methyl-2-(S)-pyrrolidinylmethoxy)pyridine;
5-methyl-6-fluoro-3-(1-methyl-2-(R)-pyrrolidinylmethoxy)pyridine;
5-methyl-6-chloro-3-(2-(S)-pyrrolidinylmethoxy)pyridine;
5-methyl-6-chloro-3-(2-(R)-pyrrolidinylmethoxy)pyridine;
5-methyl-6-chloro-3-(1-methyl-2-(S)-pyrrolidinylmethoxy)pyridine;
5-methyl-6-chloro-3-(1-methyl-2-(R)-pyrrolidinylmethoxy)pyridine;
5,6-dimethyl-3-(2-(S)-pyrrolidinylmethoxy)pyridine;
5,6-dimethyl-3-(2-(R)-pyrrolidinylmethoxy)pyridine;
5,6-dimethyl-3-(1-methyl-2-(S)-pyrrolidinylmethoxy)pyridine;
5,6-dimethyl-3-(1-methyl-2-(R)-pyrrolidinylmethoxy)pyridine;
6-fluoro-5-methoxy-3-(2-(S)-pyrrolidinylmethoxy)pyridine;
6-fluoro-5-methoxy-3-(2-(R)-pyrrolidinylmethoxy)pyridine;
6-fluoro-5-methoxy-3-(1-methyl-2-(S)-pyrrolidinylmethoxy)pyridine;
6-fluoro-5-methoxy-3-(1-methyl-2-(R)-pyrrolidinylmethoxy)pyridine;
6-fluoro-5-ethoxy-3-(2-(S)-pyrrolidinylmethoxy)pyridine;
6-fluoro-5-ethoxy-3-(2-(R)-pyrrolidinylmethoxy)pyridine;
6-fluoro-5-ethoxy-3-(1-methyl-2-(S)-pyrrolidinylmethoxy)pyridine; and
6-fluoro-5-ethoxy-3-(1-methyl-2-(R)-pyrrolidinylmethoxy)pyridine; or a pharmaceutically acceptable salt thereof.

Preferred substituted pyrrolidinyl compounds of the present invention include:
5-bromo-6-chloro-3-(2-(S)-pyrrolidinylmethoxy)pyridine;
5-bromo-6-chloro-3-(1-methyl-2-(S)-pyrrolidinylmethoxy)pyridine;
5-bromo-6-chloro-3-(2-(R)-pyrrolidinylmethoxy)pyridine;
5-bromo-6-chloro-3-(1-methyl-2-(R)-pyrrolidinylmethoxy)pyridine;
5,6-dichloro-3-(2-(R)-pyrrolidinylmethoxy)pyridine;
5,6-dichloro-3-(1-methyl-2-(R)-pyrrolidinylmethoxy)pyridine;
5,6-dichloro-3-(2-(S)-pyrrolidinylmethoxy)pyridine;
5,6-dichloro-3-(1-methyl-2-(S)-pyrrolidinylmethoxy)pyridine;
5-ethyl-6-chloro-3-(2-(S)-pyrrolidinylmethoxy)pyridine;
5-ethyl-6-chloro-3-(1-methyl-2-(S)-pyrrolidinylmethoxy)pyridine; and
5-ethyl-6-chloro-3-(2-(R)-pyrrolidinylmethoxy)pyridine; or a pharmaceutically acceptable salt thereof.

### Definitions

The terms "C₁-C₃-alkyl," "C₁-C₄-alkyl,"or "C₁-C₆-alkyl" as used herein refer to saturated, straight- or branched-chain hydrocarbon radicals containing between one and three or one and six carbon atoms, respectively. Examples of C₁-C₄-alkyl radicals include, but are not limited to, methyl, ethyl, propyl, isopropyl, *n*-butyl and *t*-butyl. Examples of C₁-C₃ alkyl radicals include methyl, ethyl, propyl and isopropyl, examples of C₁-C₆-alkyl radicals include, but are not limited to, methyl, ethyl, propyl, isopropyl, *n*-butyl, *t*-butyl, neopentyl, n-hexyl.

The term "C₁-C₃-alkoxy" as used herein refers to an C₁-C₃-alkyl group, as previously defined, attached to the parent molecular moiety through an oxygen atom. Examples of C₁-C₃-alkoxy, but are not limited to, methoxy, ethoxy, propoxy and isopropoxy.

One or more asymmetric centers may exist in the compounds of the present invention. Except where otherwise noted, the present invention contemplates the various stereoisomers and mixtures thereof.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, *et al*. describe pharmaceutically acceptable salts in detail in J. Pharmaceutical Sciences. 66: 1-19 (1977), incorporated herein by reference. The salts can be prepared *in situ* during the final isolation and purification of the compounds of the invention, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, *p*-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

As used herein, the term "pharmaceutically acceptable ester" refers to esters which hydrolyze *in vivo* and include those that break down headily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters includes formates, acetates, propionates, butryates, acrylates and ethylsuccinates.

### Abbreviations

Abbreviations which have been used in the descriptions of the scheme and the examples that follow are: BOC for t-butyloxycarbonyl; Et₂O for diethyl ether; EtOAc for ethyl acetate; DEAD for diethylazodicarboxylate; DMAP for 4-dimethylaminopyridine; DMF for dimethyl formamide; DPPA for diphenylphosphoryl azide; EDC for 1-(3-dimethylaminopropyl)-3-ethylcarbodiimide HCl; EtOAc for ethyl acetate; MeOH for methanol; NaN(TMS)₂ for sodium bis(trimethylsilyl)amide; NMMO for N-methylmorpholine N-oxide; Ph for phenyl; TEA for triethylamine; TFA for trifluoroacetic acid; THF for tetrahydrofuran; TPP for triphenylphosphine.

### Pharmaceutical Compositions

The pharmaceutical compositions of the present invention comprise a therapeutically effective amount of a compound of the present invention formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar, buffering agents such as magnesium hydroxide and aluminum hydroxide; alginic acid; pyrogen-free water, isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents; sweetening, flavoring and perfuming agents, preservatives and antioxidants can also be present in the composition, according to the judgment of the formulator. The pharmaceutical compositions of this invention can be administered to humans and other animals orally, rectally, parenterally, intracisternally, intravaginally, intraperitoneally, topically (as by powders, ointments, or drops), bucally, or as an oral or nasal spray.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Solid compositions of a similar type may also be employed as fillers in soft and hard-filled gelatin capsules using such excipients as lactose or milk sugar as well as high molecular weight polyethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

According to the methods of treatment of the present invention, disorders in synaptic transmission are treated or prevented in a patient such as a human or lower mammal by administering to the patient a therapeutically effective amount of a compound of the invention, in such amounts and for such time as is necessary to achieve the desired result. By a "therapeutically effective amount" of a compound of the invention is meant a sufficient amount of the compound to treat disorders in synaptic transmission, at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

### Therapeutic Administration

The total daily dose of the compounds of this invention administered to a human or other mammal in single or in divided doses can be in amounts, for example, from 0.001 to 50 mg/kg body weight or more usually from 0.01 to 25 mg/kg body weight. Single dose compositions may contain such amounts or submultiples thereof to make up the daily dose. In general, treatment regimens according to the present invention comprise administration to a patient in need of such treatment from about 1 mg to about 1000 mg of the compound(s) of this invention per day in single or multiple doses.

### Synthetic Methods

The compounds and processes of the present invention will be better understood in connection with the following synthetic schemes which illustrate the methods by which the compounds of the invention may be prepared. The groups n, y, R¹, R², R³ and R⁴ are as defined above unless otherwise noted.

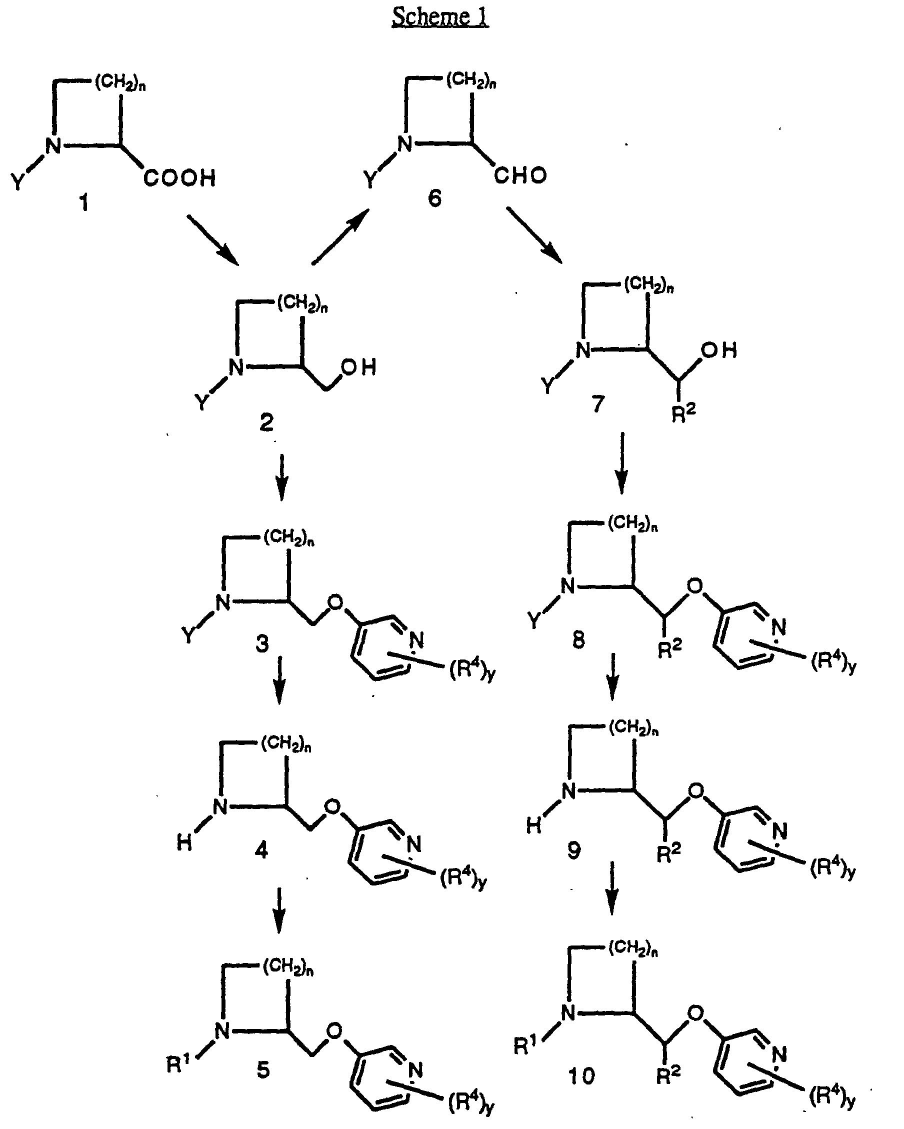

In accordance with reaction Scheme 1, compounds of Formula (I) are prepared by reacting a 2-carboxyl-substituted azacycloalkyl compound (1), wherein n is as described above, R³ is H and Y is a C₁-C₃-alkyl or a suitable protecting group, such as BOC or CBZ, for example, which may subsequently be removed and replaced with H, allyl, or C₁-C₃-alkyl, is converted to the hydroxymethyl compound of formula (2) with a suitable reducing agent, such as Red-Al®, borane/THF, borane/methyl sulfide or LiAlH₄, for example. Compound (2) is reacted with an appropriately substituted 3-hydroxypyridine compound, wherein R⁴ is as described above or is an appropriately protected R⁴ group (wherein the protecting group may be removed after the coupling reaction), in the presence of triphenylphosphine and DEAD as described by O. Mitsunobu (*Synthesis*, **1981**: 1) to form the pyridine compound of formula (3). This compound is then treated with a reagent suitable for removing the N-protecting group, such as trifluoroacetic acid for BOC removal, HCl in ethanol for CBZ removal, HBr in acetic acid, or hydrogenolysis with hydrogen in the presence of a noble metal catalyst, to form the unprotected compound (4), which is a compound of Formula (I) above. The ring nitrogen may then be alkylated, for example, by treatment with alkyl halide in the presence of a base, formaldehyde in formic acid, or with an aldehyde and sodium cyanoborohydride in an alcohol solvent, such as methanol, ethanol or isopropanol, or by reaction with the appropriate alkylating reagent, such as, for example, allyl bromide, to give the desired compound (5), wherein R¹ is as described above.

Alternately, compound (2) may be oxidized using a suitable mild oxidizing agent such as DMSO/pyridine•SO₃, pyridinium chlorochromate or DMSO/oxalyl chloride, for example, to afford the aldehyde (6). The aldehyde is then reacted with a suitable organometallic nucleophile, for example, a Grignard reagent, to afford the alcohol (7), wherein R² is as described above. The alcohol is then reacted as described above with a 3-hydroxypyridine and carried through the same series of reactions as described above, starting with compound (2) and leading to compounds (3), (4) and (5), to give the compounds (8), (9) and (10), wherein R¹ is as described above.

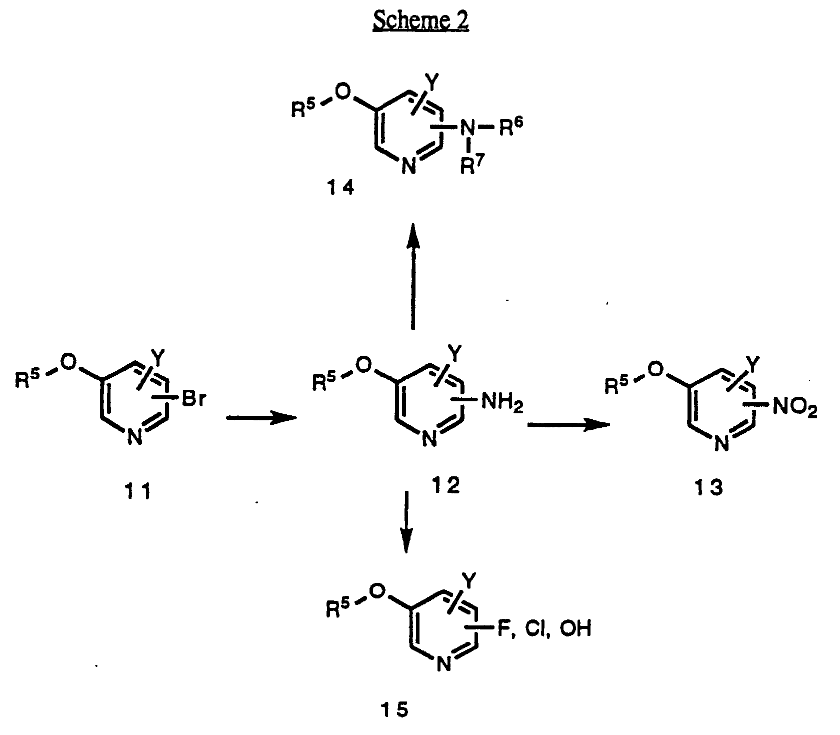

In accordance with Scheme 2, compounds (11), which may be either protected pyridine compounds wherein R⁵ is a protecting group or the conjugated compounds of formulas (5) or (10) above, and Y may be hydrogen or a suitable group selected by one skilled in the art, may be utilized as starting materials to prepare various compounds of Formula (1) wherein R⁴ represents different chemical moieties. For example, compound (11) may be reacted with NH₃ by heating in an alcoholic solvent, such as methanol, ethanol, propanol, i-propanol, t-butanol or the like, preferably in the presence of Cu²⁺ as a catalyst to give the compound (12).
In turn, compound (12) may be used to generated additional functionally substituted compounds. For example, compound (12) may be treated with potassium persulfate and sulfuric acid to oxidize the amino group to a nitro group and prepare compound (13). Or, compound (12) may be treated with R⁶-X or R⁷-X, wherein R⁶ and R⁷ are independently -C₁-C₃-alkyl or -CO-C₁-C₃-alkyl and X is Cl, to give the appropriately derivatized compounds (14). Alternately, compound (12) may be treated with nitrous acid under diazotizing conditions, then the diazo compound may be reacted with an M-X compound, wherein M is sodium or copper, and X is F or Cl to give selected compounds (15). The intermediate diazo compound may instead be treated with aqueous sulfic acid to prepare a hydroxy-substituted compound (15). If R⁵ is a protecting group, deprotection followed by conjugation as in Scheme 1 gives compounds of Formula (I).

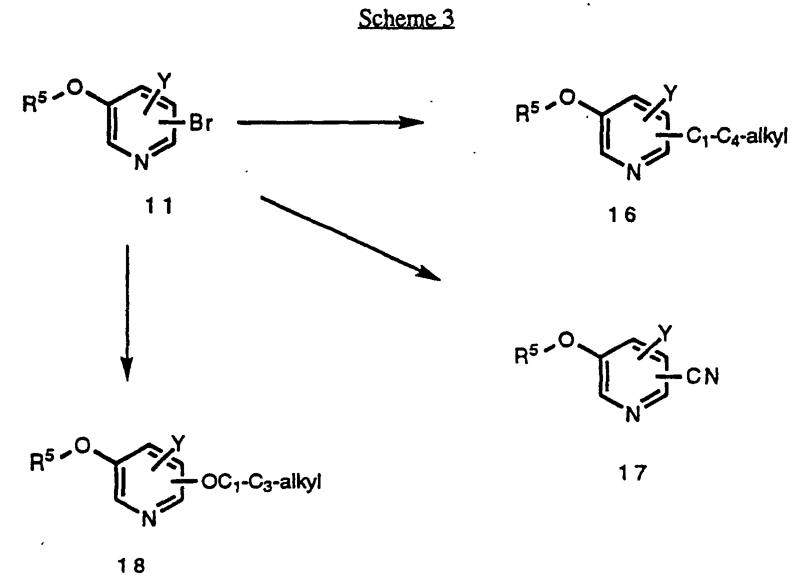

In accordance with Scheme 3 compounds (11), which may be either protected pyridine compounds wherein R⁵ is a protecting group or the conjugated compounds of formulas (5) or (10) above, and Y may be hydrogen or a suitable group selected by one skilled in the art, may be utilized as starting materials to prepare various compounds of Formula (I) wherein R⁴ is represented by additional chemical moieties. For example, compound (11) may be reacted with a C₁-C₃-alkyl-Cu-halide, or alternately, a C₁-C₃-alkyl-Mg-halide in the presence of a nickel or copper catalyst, or in certain cases, the anion of diethyl malonate followed by hydrolysis and decarboxylation, to give the alkyl-substituted compound (16). Compound (11) may be treated with Zn(CN)₂ in the presence of a Pd catalyst (according to the procedure given by D. M. Tschaen *et al*., *Synth*. *Commun*., 24:887 (**1994**)) to afford the cyano compound (17). In the event that alkoxy substituent is desired, compound (11) may be reacted with C₁-C₃-alkyl-O⁻ M⁺ salt in heated DMF or a similar dipolar aprotic solvent or in the corresponding C₁-C₃-alkanol solvent to give the compound (18).

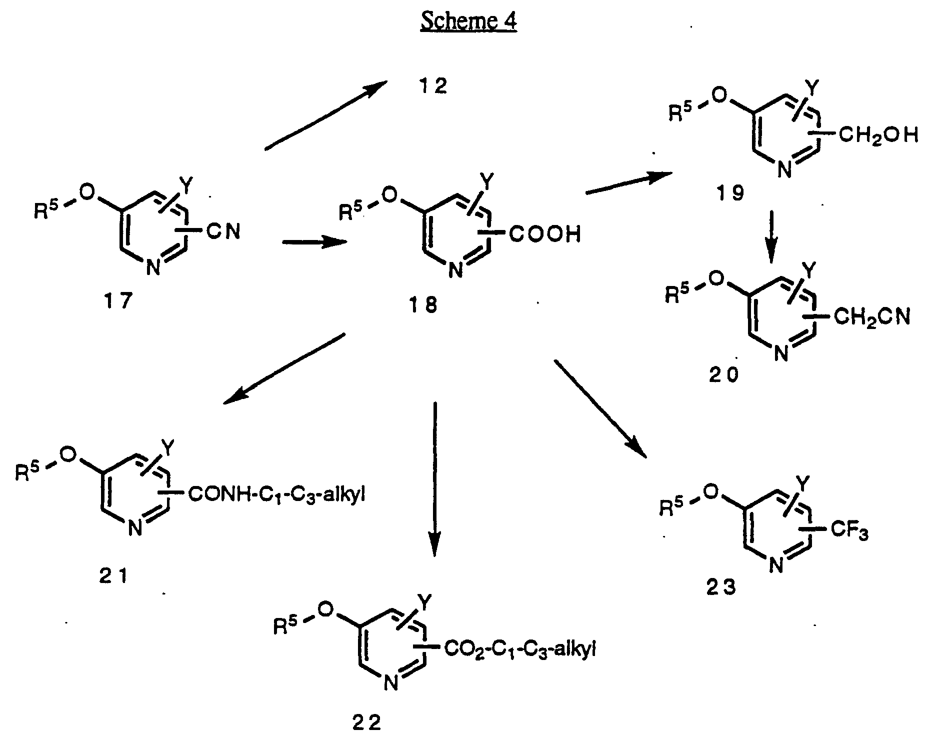

In accordance with Scheme 4 the cyano compounds (17), which may be either protected pyridine compounds wherein R⁵ is a protecting group or the conjugated compounds of formulas (5) or (10) above, and Y may be hydrogen or a suitable group selected by one skilled in the art, may be utilized as starting materials to prepare various compounds of Formula (I) wherein R⁴ is represented by additional chemical moieties. For example, compound (17) may be reacted with H₃O⁺ and the intermediate amide subjected to a Hofmann rearrangement to provide an alternate route to compounds (12). Also, compound (17) may be converted with H₃O⁺ and heat or NaOH and H₂O₂ to the carboxylic acid compound (18). Reduction of compound (18) with BH₃ or Red-Al™, for example, gives compound (19). By treating compound (19) with a reagent to convert the OH to a leaving group, such as a halo or sulfonate group, then replacing the leaving group with CN in a standard nucleophilic reaction, the cyanomethyl compound (20) may be prepared. Alternately, treating the acid (18) with an C₁-C₃-alkylamine under dehydrating conditions allows for preparation of the amide compound (21). Or, reaction of the acid (18) with a suitable alcohol, acid catalyst and heat, the ester compound (22) may be prepared. Another reagent MoF₆ may be reacted with compound (18) by a process similar to that described by Shustov *et al., J. Gen. Chem. USSR* (Engl. Transl.) 53:85-86 (**1983**) to prepare compound (23).

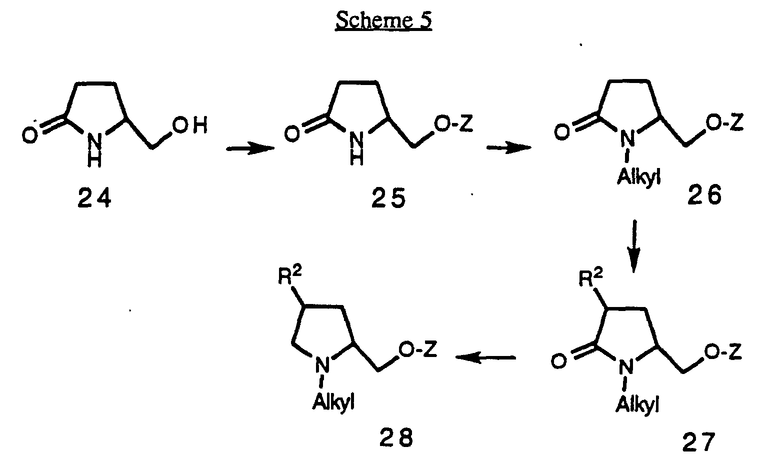

In accordance with Scheme 5, the compound (24) may be reacted according to the alternate reaction pathways shown for compounds (2) or (7) in Scheme 1 to give the desired compound (25). In compound (25) Z may represent the pyridine moiety shown in Schemes 1 or may represent a protecting group. Those skilled in the art will recognized that the protecting group may be removed and replaced by the "B" moiety at alternate positions in the synthetic scheme. Such variations in the process will be obvious to skilled practitioners, and exhaustive explanations of the synthetic options are not deemed necessary. The N-alkyl group, wherein the alkyl group is C₁-C₆-alkyl, is added to compound (25) by reaction with NaH and the appropriate alkyl iodide in anhydrous THF to give compound (26). Compound (26) is treated with LDA and an electrophile such as an alkyl halide, oxaziridine, formaldehyde, a halomethyl ether, N-halo-succinimide, or molecular halogen, for example, to give the substituted compound (27). In addition, compound (27) wherein R² is hydroxy or carboxy may be further reacted with an alkyl halide under standard Williamson ether conditions, acetic anhydride, or methanesulfonyl chloride to give additional precursors to compounds of Formula (I). The methanesulfonate from above (27, R=OMs or CH₂OMs may be further reacted with CN- or F- anions to produce additional precursors to compounds of Formula (I). B reducing the lactam moiety of (27) with BH₃ followed by treatment with CsF, the desired compound (28) is obtained. Conventional use of nitrogen or oxygen protecting groups for achieving selective reactions are included within the processes discussed herein.

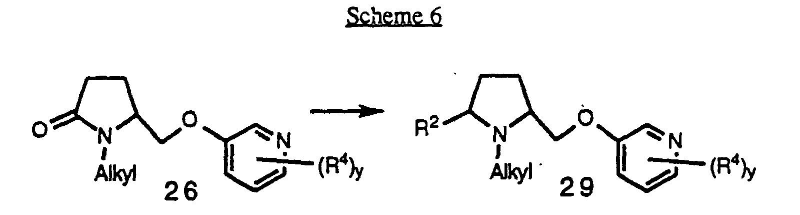

In accordance with Scheme 6 are synthesized the compounds of formula (I) wherein R² therein represents the appropriate 5-substituent group as described above, compound (26) is reacted with an C₁-C₃-alkyl Grignard reagent followed by NaBH₃CN to give the compound (29).

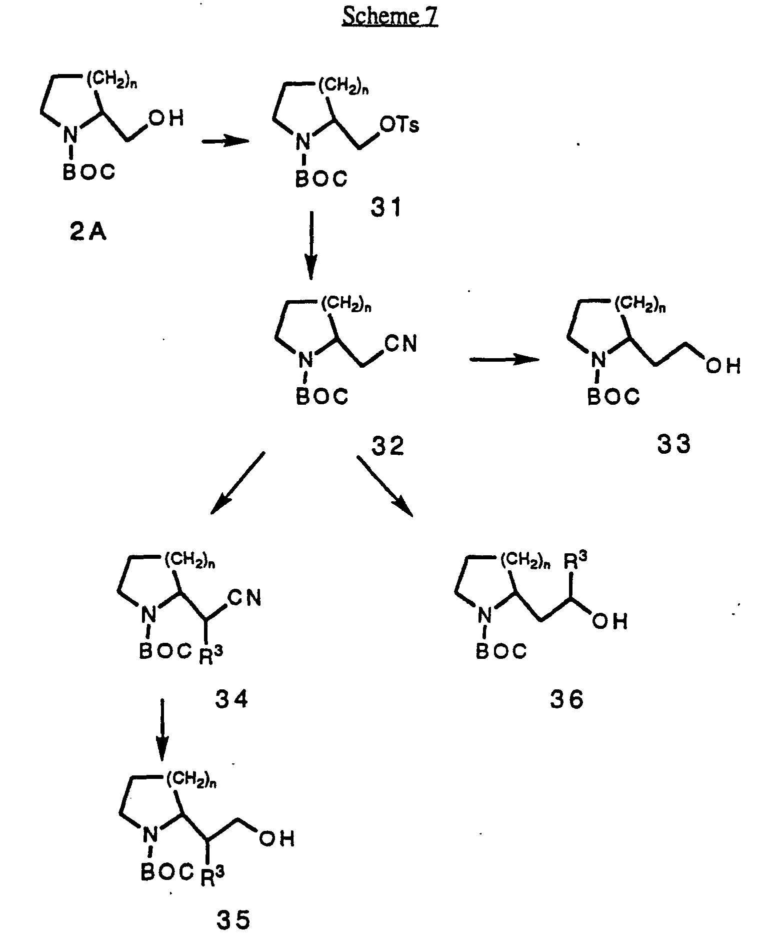

In accordance with Scheme 7 are prepared compounds of Formula (I) wherein A is 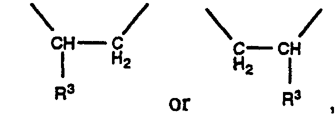 wherein R³ is as defined above. Compound (2A) (compound 2 of Scheme 1 wherein Y is BOC) is treated with tosyl chloride under standard conditions to prepare the tosylate compound (31). Compound (31) is treated with a cyanide salt in a standard nucleophilic reaction to give the compound (32). Compound (32) may be treated first with methanol and strong acid to hydrolyze the CN group to a methyl ester and the resulting ester is reduced with Li BH₄ or LiAlH₄, for example, to prepare the alcohol compound (33). Compound (33) may then be substituted for compound (2) or (7) of Scheme 1 and carried forward as desired. Alternately, compound (32) may be treated with LDA and R³-X, wherein X is a halogen or other suitable leaving group, to prepare compound (34). Compound (34) may be treated first with methanol and strong acid to convert the CN group to a methyl ester and the resulting ester is reduced with LiBH₄ or LiAlH₄, for example, to prepare the alcohol compound (35). In yet another alternate process, compound (32) may be treated with Dibal-H™, H₃O⁺, and R³-Mg-X in sequence to prepare the desired alcohol compound (36). Compounds (35) or (36) may then be substituted for compound (2) or (7) of Scheme 1 and carried forward as desired. Compounds (33), (35) and (36) present opportunities for substitution, in accord with the appropriate Schemes above, thus allowing one skilled in the art to prepare other selected compounds of the invention.

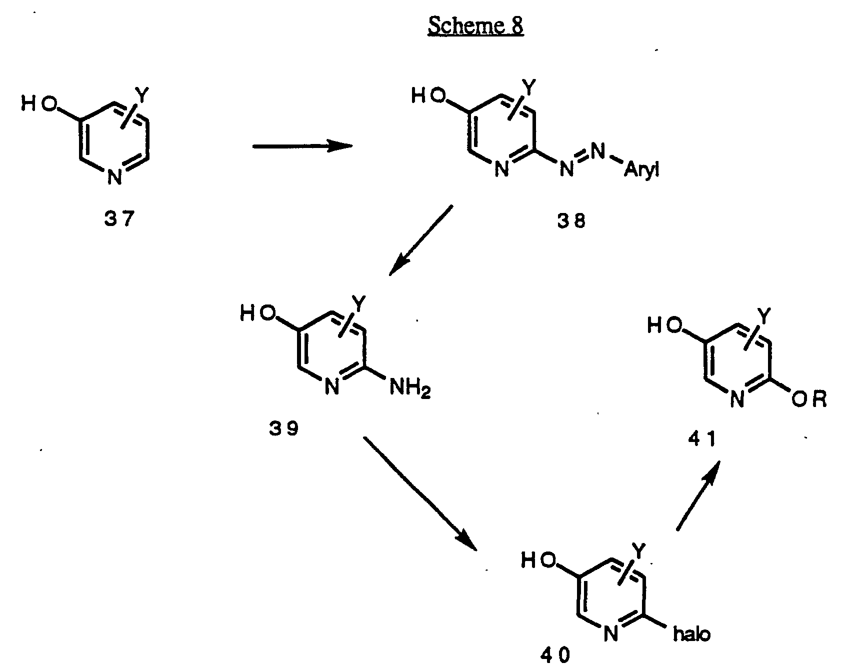

In accordance with Scheme 8 are prepared compounds that may be utilized as starting materials to provide for additional substituted pyridine moieties in selected compounds of the invention. Appropriate substituted compounds (37), wherein Y is a pre-existing group selected from C₁-C₃ alkyl, C₁-C₃ alkoxy, halo, -CN, -CF₃, -NO₂, -CH₂OH, -CH₂CN, -NH-CHO,-NHCO(C₁-C₃ alkyl), -N(C₁-C₃ alkyl)-CO(C₁-C₃ alkyl), -COOH, -COO(C₁-C₃ alkyl), -CONH₂, -CONH-(C₁-C₃ alkyl), -CONHbenzyl, and -OCOC₁-C₃ alkyl), may be treated with an aryldiazonium compound in the presence of aqueous base to give the diazo compounds (38). Compounds (38) may be reduced by treatment with H₂ and a noble metal catalyst or SnCl₂ to prepare the amino compounds (39). Alternately, treatment of compound (39) with a nucleophilic halide, in the presence of Cu⁺ when necessary, allows the preparation of compounds (40). Treatment of compound (40) with a nucleophilic alcohol ROH, allows the preparation of compounds (41).

### In vitro Determination of Neuronal Nicotinic Receptor Binding Potencies, Selectivity and Functionality

For the purpose of identifying compounds as cholinergic agents which are capable of interacting with cholinergic channel receptors in the brain, a ligand-receptor binding assay was carried out as the initial screen. Compounds of the present invention were effective at interacting with neuronal nicotinic cholinergic receptors as assayed *in vitro* for their ability to displace radioligand from neuronal nicotinic cholinergic channel receptors labeled with [³H]-cytisine ([³H]-CYT) (Protocol A below).

For the purpose of directly evaluating the ability of test compounds to functionally activate or inhibit certain subtypes of neuronal nicotinic cholinergic channels, an assay to determine ⁸⁶Rb⁺ efflux in IMR-32 cells was employed (Protocol B below).

### A. Protocol For Determination of Nicotinic Cholinergic Channel Receptor Binding Potencies of Ligands

Binding of [³H]-cytisine ([³H]-CYT) to nicotinic receptors was accomplished using crude synaptic membrane preparations from whole rat brain (Pabreza *et al., Molecular Pharmacol*., **1990,** 39:9). Washed membranes were stored at -80°C prior to use. Frozen aliquots were slowly thawed and resuspended in 20 volumes of buffer (containing: 120 *mM* NaCl, 5 *mM* KCl, 2 *mM* MgCl₂, 2 *mM* CaCl₂ and 50 *mM* Tris-Cl, pH 7.4 @4°C). After centrifuging at 20,000x g for 15 minutes, the pellets were resuspended in 30 volumes of buffer. Homogenate (containing 125-150 µg protein) was added to triplicate tubes containing concentrations of test compound and [³H]-CYT (1.25 *nM*) in a final volume of 500 µL. Samples were incubated for 60 minutes at 4°C, then rapidly filtered through Whatman GF/B filters presoaked in 0.5% polyethyleneimine using 3 x 4 mL of ice-cold buffer. The filters are counted in 4 mL of Ecolume® (ICN). Nonspecific binding was determined in the presence of 10 µM (-)-nicotine and values were expressed as a percentage of total binding. IC₅₀ values were determined with the RS-1 (BBN) nonlinear least squares curve-fitting program and IC₅₀ values were converted to Ki values using the Cheng and Prusoff correction (Ki=IC₅₀/(1+[ligand]/Kd of ligand). Alternately, data were expressed as a percentage of the total specific binding. The binding data (shown in Table 1) suggest that the compounds of the present invention have high affinity for the neuronal nicotinic cholinergic channel receptor.

### B. Protocols for the Determination of Functional Effects of Cholinergic Channel Receptor Ligands on Synaptic Transmission

Cells of the IMR-32 human neuroblastoma clonal cell line (ATCC, Rockville, MD) were maintained in a log phase of growth according to established procedures (Lukas, 1993). Experimental cells were seeded at a density of 500,000 cells/mL into a 24-well tissue culture dish. Plated cells were allowed to proliferate for at least 48 hours before loading with 2 µCi/mL of ⁸⁶Rb⁺ (35 Ci/mmol) overnight at 37°C. The ⁸⁶Rb⁺ efflux assays were performed according to previously published protocols (Lukas, R.J., *J. Pharmacol. Exp. Ther*., 265: 294-302, **1993)** except serum-free Dulbecco's Modified Eagle's Medium was used during the ⁸⁶Rb⁺ loading, rinsing, and agonist-induced efflux steps.

EC₅₀ data and maximal responses (reported as percent relative to the response elicited by 100 µM (S)-nicotine) are shown for selected compounds of the invention. The inhibition data (given for other selected compounds) reflect inhibition of the efflux elicited by 100 µM (S)-nicotine for either a single dose (% inhibition at 1 or 10 µM) or over a range of doses (IC₅₀ of inhibition). The results (also shown in Table 1) suggest that selected compounds of the present invention either activate or inhibit the initial ion flux aspects of synaptic transmission mediated by neuronal nicotinic acetylcholine receptors. This finding is in agreement with the results of others who have linked dopamine release, which is dependent upon the ion flux in synaptic transmission, to binding at nicotinic receptors (cf., for example, Lippiello and Caldwell, U.S.Patent 5,242,935, issued Sept. 7, 1993; Caldwell and Lippiello, U.S.Patent 5,248,690, issued Sept. 28, 1993; and Wonnacott *et al*., Prog. Brain Res., 79: 157-163 (**1989**)).

**Table 1**

| Binding to Neuronal Nicotinic Receptors and Activation or Inhibition of Neuronal Nicotinic Cholinergic Channels in IMR-32 Cells | | | | | | |
|---|---|---|---|---|---|---|
| Ex. No | Binding (nM) | IMR-32 EC₅₀ (µM) | IMR-32 maximal response | % Inhibition @ 1 µM | % Inhibition @ 10 µM | Inhibition IC₅₀ (µM) |
| 2 | 0.02 | 0.1 | 120 | | | |
| 3 | 0.38 | | | 55 | | |
| 5 | 0.35 | >10,000 | 12 | 12 | 22 | |
| 7 | 0.099 | 4.9 | 70 | | | |
| 8 | 13 | >10,000 | 10.6 | 28 | 60 | |
| 9 | 0.03 | 1.6 | 67 | | | |
| 10 | 20 | >10,000 | 3.3 | 18 | 90 | |
| 11 | 0.029 | 0.6 | 93 | | | |
| 12 | 0.11 | >10,000 | 16 | 5 | 8 | |
| 15 | 0.024 | 0.3 | 152 | | | |
| 16 | 0.7 | 0.5 | 37 | 35 | | |
| 17 | 0.06 | 0.2 | 108 | | | |
| 18 | 2.4 | >10,000 | 22.5 | 0 | 18 | |
| 45 | 0.04 | 0.4 | 137 | | | |
| 46 | 0.6 | >10,000 | 11 | | | |
| 47 | 0.065 | 22 | 152 | | | |
| 48 | 0.027 | 56 | 129 | | | |
| 49 | 0.097 | 0.29 | 158 | | | |
| 50 | 0.88 | 10 | 35 | | | |
| 51 | 0.19 | 16 | 64 | | | |
| 52 | | 1.6 | 124 | | | |

### Examples

The present invention will be better understood by reference to the following examples, which are intended as an illustration of, and not a limitation upon, the scope of the invention as it is defined by the appended claims

### Preparations of Starting Materials

Several starting materials are used repeatedly throughout the examples that follow. 1-Methyl-2-(S)-pyrrolidinemethanol was obtained from Aldrich Chemical Co. 1-Methyl-2-(R)-pyrrolidinemethanol was obtained from Fluka.

In the PCT Patent Application WO94 08992 of Abreo *et al.,* published April 28, 1994, and in Abreo, *et al. J. Med Chem.,* 3:, 817-825 **(1996),** are disclosed methods for preparing, *inter alia,* the (R) and (S) 1-BOC-2-(S)-pyrrolidinemethanol compounds, the (R) and (S) 1-BOC-2-(S)-azetidinemethanol compounds, and l-Cbz-2-(R)-azetidinemethanol. 1-Cbz-2-(S)-azetidinemethanol is prepared from 2-(S)-azetidinecarboxylic acid using analogous procedures.

### Example 2

### 5-Bromo-6-chloro-3-(2-(S)-azetidinylmethoxy)pyridine hydrochloride

### Step 2a, 5-Bromo-6-chloro-3-(1-BOC-2-(S)-azetidinylmethoxy)pyridine

To a solution of diethyl azodicarboxylate (1.52 mL, 9.6 mmol) in THF (56 mL) was added triphenylphosphine (2.52 g, 9.6 mmol) at 0°C, and the reaction mixture was stirred for 0.5 hour. 1-BOC-2-(S)-azetidinemethanol (1.44 g, 7.7 mmol) and 5-bromo-6-chloropyridin-3-ol (1.4 g, 6.4 mmol; prepared from 2-hydroxy-5-nitropyridine according to V. Koch and S. Schnatterer, *Synthesis* **1990,** 499-501) were then added. The reaction mixture was allowed to warm slowly to room temperature and stir overnight. Solvent was removed, and the residue was chromatographed on a silica gel column, eluting with chloroform:methanol (100:1) to afford the title compound. MS (DCI/NH₃) m/z 377,379 (M+H)⁺.

### Step 2b, 5-Bromo-6-chloro-3-(2-(S)-azetidinylmethoxy)pyridine hydrochloride

To 5-bromo-6-chloro-3-(1-BOC-2-(S)-azetidinylmethoxy)pyridine from step 2a (360 mg, 0.95 mmol) was added TFA in methylene chloride at 0°C, and the mixture was stirred for 30 minutes. The volatile components were then removed under vacuum. The residue was neutralized with NaHCO₃, then extracted with methylene chloride, which was dried over MgSO₄ and concentrated. The residue was chromatographed on a silica gel column, eluting with methylene chloride:methanol:NH₄OH 10:1:0.1 to afford to give the free base of the title compound. The base was converted to the salt by treatment with hydrogen chloride in ether to give the title compound (224 mg). mp 168-169°C. MS (DCI/NH₃) m/z 277, 279 (M+H)⁺. ¹H NMR (D₂O, 300 MHz) δ 2.69 (dd, J=7.0, 8.5, 2H), 4.06-4.20 (m, 3H), 4.43 (d, J=4.5, 2H), 4.95 (m, 1H), 7.94 (d, J=3.0, 1H), 8.17 (d, J=3.0, 1H). Anal. Calcd. for C₉H₁₀N₂OBrCl•0.9 HCl: C, 34.83; H, 3.54; N, 9.03. Found: C, 34.85; H, 3.56; N, 8.82. [α]²⁵_{D}=-4.81° (c 0.13, MeOH).

### Example 3

### 5-Bromo-6-chloro-3-(1-methyl-2-(S)-azetidinylmethoxy)pyridine hydrochloride

A sample of 5-bromo-6-chloro-3-(2-(S)-azetidinylmethoxy)pyridine (from Example 2b, 140 mg, 0.51 mmol) was stirred with excess paraformaldehyde (0.5 mL) in ethanol (3 mL), on ice and under nitrogen at pH 5 (pH adjusted with acetic acid). The reaction was stirred for 15 minutes and sodium cyanoborohydride (94 mg, 1.5 mmol) was added. A very small amount of bromocresol green was added directly to the reaction mixture as indicator. The reaction was stirred 18 hours, allowed to warm to room temperature, and additional formaldehyde (0.25 mL) and sodium cyanoborohydride (20 mg, 0.32 mmol) were added. The reaction mixture was then acidified to pH 1 with 10% solution of potassium hydrogen sulfate, and the volatile components were evaporated. The aqueous phase was washed with ethyl acetate (3x10 mL), basified with sodium carbonate (pH 9.5), and the products were extracted with ethyl acetate (5x15 mL). These extracts were dried (MgSO₄), filtered, and concentrated *in vacuo.* The residual oil was purified by flash silica gel chromatography (CHCl₃:MeOH:NH₄OH/10:1:0.1), yielding the pure free base (101 mg, 68% yield), which was dissolved in ethanol and converted to the hydrochloride salt by treatment with hydrogen chloride in ethanol to give the title compound. mp 155-157°C (dec). MS (DCI/NH₃) m/z 291, 293 (M+2H)⁺. ¹H NMR (D₂O, 300 MHz) δ 2.54-2.77 (m, 2H), 2.98 (s, 3H), 3.99 (m, 1H), 4.27 (m, 1H), 4.37-4.55 (m, 2H), 7.93 (d, J=3 Hz, 1H), 8.17 (s, J=3 Hz, 1H). Anal. Calcd. for C₁₀H₁₂N₂OBrCl•1HCl•0.8 H₂O·: C, 35.07; H, 4.30; N, 8.18. Found: C, 35.08; H, 4.12; N, 8.08. [α]²⁵_{D}=-13.1° (c 0.16, MeOH).

### Example 4

### 5-Bromo-6-chloro-3-(2-(S)-pyrrolidinylmethoxy)pyridine dihydrochloride

### Step 4a, 5-Bromo-6-chloro-3-(1-BOC-2-(S)-pyrrolidinylmethoxy)pyridine

To a solution of diethyl azodicarboxylate (1.89 mL, 12.0 mmol) in THF (30 mL) was added triphenylphosphine (3.15 g, 12.0 mmol) at 0°C, and the reaction mixture was stirred for 0.5 hour. 1-BOC-(S)-pyrrolidinemethanol (2.41 g, 12.0 mmol) and 5-bromo-6-chloropyridine-3-ol (2.09 g, 10.0 mmol) were then added. The reaction mixture was allowed to warm to room temperature overnight. The solvent was removed, and the residue was chromatographed on a silica gel column, eluting with EtOAc/hexane (1:5 and 1:2) to afford an oil (3.80 g, 97%). MS (CI/NH₃) m/z 391,393 (M+H)⁺. ¹H NMR (DMSO-d₆, 300 MHz) δ 1.65-2.05 (m, 4H), 3.20-3.35 (m, 2H), 3.95-4.15 (m, 3H), 7.98 (d, J = 2.9 Hz, 1H), 8.21 (d, J = 2.9 Hz, 1H).

### Step 4b, 5-Bromo-6-chloro-3-(2-(S)-pyrrolidinylmethoxy)pyridine dihydrochloride

To 5-bromo-6-chloro-3-(1-BOC-2-(S)-pyrrolidinylmethoxy)pyridine from step 4a (150 mg, 0.38 mmol) was added hydrogen chloride (4.0 M in 1,4-dioxane, 3.0 mL), and the reaction mixture was allowed to stir at room temperature for two days. A precipitate formed which was filtered, washed with Et₂O and vacuum-dried to afford the title compound (119 mg, 94%). mp 264-268°C. MS (CI/NH₃) m/z 291,293 (M+H)⁺, 308,310 (M+NH₄)⁺. ¹H NMR (D₂O, 300 MHz) δ 1.90-2.35 (m, 4H), 3.38-3.46 (m, 2H), 4.12 (m, 1H), 4.27 (m, 1H), 4.47 (m, 1H), 7.90 (d, J = 2.9 Hz, 1H), 8.13 (d, J = 2.9 Hz, 1H). Anal. Calcd. for C₁₀H₁₂N₂OBrCl•1.0 HCl: C, 36.61; H, 3.99; N, 8.54. Found: C, 36.61; H, 3.95; N, 8.42. [α]²⁵_{D}=+9.2° (c 0.90, MeOH).

### Example 5

### 5-Bromo-6-chloro-3-(1-methyl-2-(S)-pyrrolidinylmethoxy)pyridine dihydrochloride

### Step 5a, 5-(Bromo-6-chloro-3-(1-methyl-2-(S)-pyrrolidinylmethoxy)pyridine (50722-139)

To 5-bromo-6-chloro-3-(1-BOC-2-(S)-pyrrolidinylmethoxy)pyridine (from Example 4a, 300 mg, 0.77 mmol) was added formalin (38%, 3.0 mL) and formic acid (88%, 1.5 mL). The mixture was refluxed for 5 hours and cooled to room temperature. Hydrochloric acid (36%, 0.3 mL) was added, and the mixture was extracted with Et₂O (3 x 8 mL). The aqueous layer was concentrated to dryness under vacuum. Water (2 mL) was added, and solid sodium bicarbonate was added until the aqueous layer was saturated. The mixture was extracted with EtOAc, which was dried over MgSO4, filtered and concentrated. The residue was chromatographed on a silica gel column, eluting with CHCl₃/MeOH 10:1 to afford an oil (214 mg, 91%). MS (CI/NH₃) m/z 305,307 (M+H)⁺. ¹H NMR (CDCl₃, 300 MHz) δ 1.60-2.11 (m, 4H), 2.33 (m, 1H), 2.48 (s, 3H), 2.67 (m, 1H), 3.11 (m, 1H), 3.89-4.02 (m, 2H), 7.52 (d, J = 3.0 Hz, 1H), 8.04 (d, J = 3.0 Hz, 1H).

### Step 5b, 5-Bromo-6-chloro-3-(1-methyl-2-(S)-pyrrolidinylmethoxy)pyridine dihydrochloride

To 5-bromo-6-chloro-3-(1-methyl-2-(S)-pyrrolidinylmethoxy)pyridine from step 5a (100 mg, 0.33 mmol) in THE (3.0 mL) was added hydrogen chloride (1.0 M in Et₂O, 0.75 mL, 0.75 mmol). A precipitate formed which was filtered, washed (Et₂O) and vacuum-dried to afford the title compound (85 mg, 76%). mp 189-191°C. MS (CI/NH₃) m/z 305,307 (M+H)⁺. ¹H NMR (D₂O, 300 MHz) δ 2.02-2.47 (m, 4H), 3.03 (s, 3H), 3.28 (m, 1H), 3.73 (m, 1H), 3.94 (m, 1H), 4.36 (m, 1H), 4.51 (m, 1H), 7.92 (d, J = 3.0 Hz, 1H), 8.14 (d, J = 3.0 Hz, 1H). Anal. Calcd. for C₁₁H₁₄N₂OBrCl•1.0 HCl: C, 38.63; H, 4.51; N, 8.19. Found: C, 38.67; H, 4.49; N, 8.13. [α]²⁵_{D}=-3.8° (c 0.50, MeOH).

### Example 7

### 5-bromo-6-chloro-3-(2-(R)-pyrrolidinylmethoxy)pyridine dihydrochloride

Following the procedure of Example 4, except substituting 1-BOC-(R)-pyrrolidinemethanol for the 1-BOC-(S)-pyrrolidinemethanol thereof, the 5-bromo-6-chloro-3-(1-BOC-2-(R)-pyrrolidinylmethoxy)pyridine was prepared. MS (CI/NH₃) m/z 391,393 (M+H)⁺. ¹H NMR (CDCl₃, 300 MHz) δ 1.65-2.05 (m, 4H), 3.20-3.35 (m, 2H), 3.95-4.15 (m, 3H), 7.55 (d, J = 2.9 Hz, 1H), 8.12 (d, J = 2.9 Hz, 1H). The 5-bromo-6-chloro-3-(1-BOC-2-(R)-pyrrolidinylmethoxy)pyridine (150 mg) was stirred with 4N HCl in dioxane (3 mL) at room temperature for 16 hours . The precipitate formed was triturated with ether, and the solid was dried under high vacuum to afford the title compound (99.2 mg). mp 230°C. MS (DCI/NH₃) m/z 291,293 (M+H)⁺. ¹H NMR (D₂O, 300 MHz) δ 1.89-2.02 (m, 1H), 2.05-2.21 (m, 2H), 2.28 (m, 1H), 3.42 (t, J=7.2 Hz, 2H), 4.12 (m, 1H)), 4.25 (dd, J=7.8, 10.5 Hz, 1H), 4.47 (dd, J=3.2, 10.5 Hz, 1H), 7.90 (d, J=2.7 Hz, 1H), 8.13 (d, J=2.7 Hz, 1H). Anal. Calcd. for C₁₀H₁₂N₂OBrCl•2 HCl: C, 36.61; H, 3.99; N, 8.54. Found: C, 36.69; H, 3.91; N, 8.41. [α]²⁵_{D}=-14.80° (c 0.25, MeOH).

### Example 8

### 5-bromo-6-chloro-3-(1-methyl-2-(R)-pyrrolidinylmethoxy)pyridine hydrochloride

To 5-bromo-6-chloro-3-(1-BOC-2-(R)-pyrrolidinylmethoxy)pyridine from Example 7 (210 mg, 0.54 mmol) was added formalin (37%, 7 mL) and formic acid (3.5 mL), and the mixture was heated at 70°C for 2.5 hours. The solvent was concentrated, and excess solid NaHCO₃ was added to the residue. The mixture was extracted with methylene chloride, which was dried over MgSO₄ and concentrated. The residue was chromatographed on a silica gel column, eluting with methylene chloride:methanol 100:5-100:10 to give the free base of the title compound (110 mg, 67% yield). MS (DCI/NH₃) m/z 305, 307 (M+H)⁺. ¹H NMR (CDCl₃, 300 MHz) δ 1.68 (m, 3H), 2.02 (m, 1H), 2.32 (m, 1H), 2.48 (s, 3H), 2.67 (m, 1H), 3.12 (m, 1H), 3.96 (m, 2H), 7.52 (m, 1H), 8.06 (m, 1H). The base was converted to the salt by treatment with hydrogen chloride in THF to give the title compound. MS (DCI/NH₃) m/z 305, 307 (M+H)^{+ 1}H NMR (D₂O, 300 MHz) δ 2.20-2.17 (m, 2H), 2.24 (m, 1H), 2.39 (m, 1H), 3.03 (s, 3H), 3.27 (m, 1H), 3.73 (m, 1H), 3.96 (m, 1H), 4.36 (dd, J=6.1, 11.2 Hz, 1H), 4.53 (dd, J=3.1. 11.2 Hz, 1H), 7.91 (d, J=2.7 Hz, 1H), 8.13 (d, J=2.7 Hz, 1H). Anal. Calcd. for C₁₁H₁₄N₂OBrCl•1.1 HCl: C, 38.22; H, 4.40; N, 8.10. Found: C, 37.95; H, 4.81; N, 7.76. [α]²⁵_{D}=+11.06° (c 0.24, MeOH).

### Example 9

### 5,6-Dichloro-3-(2-(R)-pyrrolidinylmethoxy)pyridine hydrochloride

### Step 9a, 5,6-Dichloro-3-(1-BOC-2-(R)-pyrrolidinylmethoxy)pyridine

To a solution of triphenylphosphine (2.6 g, 9.88 mmol) in THF (30 mL) was added diethyl azodicarboxylate (1.56 mL, 9.88 mmol) at 0°C, and the reaction mixture was stirred for 0.5 hour. 1-BOC-(R)-pyrrolidinemethanol (2.0 g, 9.88 mmol) and 5,6-dichloropyridine-3-ol (1.35 g, 8.23 mmol; prepared from 2-hydroxy-5-nitropyridine according to the procedure of V. Koch and S. Schnatterer, *Synthesis* **1990,** 499-501) were then added. The reaction mixture was slowly warmed up to room temperature overnight. The solvent was removed, and the residue was chromatographed on a silica gel column, eluting with EtOAc/hexane 1:6 to afford an oil (2.16 g). MS (CI/NH₃) m/z 347, 349 (M+H)⁺, 364, 366 (M+NH₄)⁺. ¹H NMR (CDCl₃, 300 MHz) δ 1.47 (s, 9H), 1.85-2.05 (m, 4H), 3.35-3.45 (m, 2H), 3.85-4.2 (m, 3H), 7.35-7.45 (br d, 1H), 8.02 (d, J = 2.5 Hz, 1H).

### Step 9b, 5,6-Dichloro-3-(2-(R)-pyrrolidinylmethoxy)pyridine hydrochloride

To 5,6-dichloro-3-(1-BOC-2-(R)-pyrrolidinylmethoxy)pyridine from step 9a (9.88 mmol) was added trifluoroacetic acid in methylene chloride (1:1, 20 mL) at 0°C, and the mixture was stirred for 30 minutes. The residue was neutralized with saturated KHCO₃ solution, then extracted with methylene chloride, which was dried over MgSO₄ and concentrated to afford the free base of the title compound (1.24 g, oil). The base (800 mg, 3.2 mmol) was converted to the salt by treatment with saturated hydrogen chloride in ether to give the title compound (398 mg). mp 250-252°C. MS (CI/NH₃) m/z 247, 249, 251 (M+H)⁺, 264, 266 (M+NH₄)⁺. ¹H NMR (D₂O, 300 MHz) δ 1.90-2.35 (m, 4H), 3.40-3.45 (m, 2H), 4.13 (m, 1H), 4.27 (m, 1H), 4.47 (m, 1H), 7.74 (d, J = 2.7 Hz, 1H), 8.07 (d, J = 2.7 Hz, 1H). Anal. Calcd. for C₁₀H₁₂N₂OCl₂•1.0 HCl: C, 42.35; H, 4.62; N, 9.88. Found: C, 42.41; H, 4.49; N, 9.79. [α]²⁵_{D}=-11.1° (c 1.0, MeOH).

### Example 10

### 5,6-Dichloro-3-(1-methyl-2-(R)-pyrrolidinylmethoxy)pyridine hydrochloride

### Step 10a, (5,6-Dichloro-3-(1-methyl-2-(R)-pyrrolidinylmethoxy)pyridine (50722-139)

To 5,6-dichloro-3-(1-BOC-2-(R)-pyrrolidinylmethoxy)pyridine (from Example 9b, 438 mg, 1.8 mmol) was added formalin (37%, excess) and formic acid (88%, excess). The mixture was treated by a procedure similar to that of Example 5a to afford a solid (338 mg, 72%). mp 38-39°C. MS (CI/NH₃) m/z 261/263 (M+H)⁺. [α]²⁵_{D}=+59.3° (c 1.00, CHCl₃).

### Step 10b, 5,6-Dichloro-3-(1-methyl-2-(R)-pyrrolidinylmethoxy)pyridine dihydrochloride

To 5,6-dichloro-3-(1-methyl-2-(R)-pyrrolidinylmethoxy)pyridine from step 10a (313 mg) in ether was added saturated HCl in ether. A precipitate formed which was washed with Et₂O, then recrystallized from methanol/ether, and vacuum dried to afford the title compound (272 mg). mp 187-189°C. MS (CI/NH₃) m/z 262, 263, 265 (M+H)⁺. ¹H NMR (D₂O, 300 MHz) δ 2.02-2.45 (m, 4H), 3.03 (S, 3H), 3.28 (m, 1H), 3.73 (m, 1H), 3.94 (m, 1H), 4.36 (m, 1H), 4.51 (m, 1H), 7.76 (d, J = 2.7 Hz, 1H), 8.10 (d, J = 2.7 Hz, 1H). Anal. Calcd. for C₁₁H₁₄N₂OCl₂•1.0 HCl•0.1 H₂O: C, 44.13; H, 5.12; N, 9.36. Found: C, 43.89; H, 5.21; N, 9.19. [α]²⁵_{D}=+6.8° (c 1.0, MeOH).

### Example 11

### 5,6-Dichloro-3-(2-(S)-pyrrolidinylmethoxy)pyridine hydrochloride

### Step 11a, 5,6-Dichloro-3-(1-BOC-2-(S)-pyrrolidinylmethoxy)pyridine

To a solution of triphenylphosphine (2.9 g, 11.0 mmol) in THF (30 mL) was added diethyl azodicarboxylate (1.7 mL, 11.0 mmol) at 0°C, and the reaction mixture was stirred for 15 minutes. 1-BOC-(S)-pyrrolidinemethanol (2.21 g, 11.0 mmol) and 5,6-dichloropyridine-3-ol (1.5 g, 9.2 mmol) were then added. The reaction mixture was slowly warmed up to room temperature overnight. The solvent was removed, and the residue was chromatographed on a silica gel column, eluting with EtOAc/hexane (1:6) to afford an oil (3.5 g). MS (CI/NH₃) m/z 347, 349 (M+H)⁺, 364/366 (M+NH₄)⁺. ¹H NMR (CDCl₃, 300 MHz) δ 1.47 (s, 9H), 1.85-2.05 (m, 4H), 3.35-3.45 (m, 2H), 3.85-4.2 (m, 3H), 7.40 (m, 1H), 8.02 (d, J = 2.5 Hz, 1H).

### Step 11b, 5,6-Dichloro-3-(2-(S)-pyrrolidinylmethoxy)pyridine hydrochloride

To 5,6-dichloro-3-(1-BOC-2-(S)-pyrrolidinylmethoxy)pyridine from step 11a (3.5 g) was added trifluoroacetic acid in methylene chloride (1:1, 30 mL) at 0°C, and the mixture was stirred for 30 minutes. The residue was partitioned between saturated K₂CO₃ solution and methylene chloride, then the organic layer was dried over MgSO₄ and concentrated. The residue was flash chromatographed on silica gel eluting with 10% methanol/chloroform to afford the free base of the title compound (2.5 g, oil). The base (1.32 g, 5.3 mmol) was converted to the salt by treatment with saturated HCl in ether to give the title compound (224 mg). mp 253-254°C. MS (CI/NH₃) m/z 247, 249, 251 (M+H)⁺, 264, 266 (M+NH₄)⁺. ¹H NMR (D₂O, 300 MHz) δ 1.90-2.36 (m, 4H), 3.37-3.49 (m, 2H), 4.13 (m. 1H), 4.27 (m, 1H), 4.48 (dd, J=3.5, 10.5, 1H), 7.74 (d, J = 2.7 Hz, 1H), 8.07 (d, J = 2.7 Hz, 1H). Anal. Calcd. for C₁₀H₁₂N₂OCl₂•1.0 HCl: C, 42.35; H, 4.62; N, 9.88. Found: C, 42.41; H, 4.55; N, 9.73. [α]²⁵_{D}=+12.2° (c 1.0, MeOH).

### Example 12

### 5,6-Dichloro-3-(1-methyl-2-(S)-pyrrolidinylmethoxy)pyridine hydrochloride

### Step 12a, 5-(5,6-Dichloro-3-(1-methyl-2-(S)-pyrrolidinylmethoxy)pyridine (50722-139)

To 5,6-dichloro-3-(1-BOC-2-(S)-pyrrolidinylmethoxy)pyridine (from Example 11a, 1.06 g, 4.29 mmol) was added formalin (37%, excess) and formic acid (88%, excess). The mixture was heated for 2 hours, then hydrochloric acid (12 N) was added, and the mixture was washed with Et₂O. The aqueous layer was basified with 15% NaOH, and the mixture was extracted with methylene chloride, which was dried over MgSO₄, filtered and concentrated. The residue was chromatographed on a silica gel column, eluting with CHCl₃/MeOH (20:1) to afford an oil (360 mg). MS (CI/NH₃) m/z 261,263,265 (M+H)⁺. [α]²⁵_{D}=-48.8° (c 1.00, CHCl₃).

### Step 12b, 5,6-Dichloro-3-(1-methyl-2-(S)-pyrrolidinylmethoxy)pyridine hydrochloride

To 5,6-dichloro-3-(1-methyl-2-(S)-pyrrolidinylmethoxy)pyridine from step 12a (360 mg) in ether was added saturated HCl in ether. A precipitate formed which was washed with Et₂O and dried under vacuum to afford the title compound (240 mg). mp 192-193°C. MS (CI/NH₃) m/z 261, 263, 265 (M+H)⁺. ¹H NMR (D₂O, 300 MHz) δ 2.05-2.45 (m, 4H), 3.03 (S, 3H), 3.28 (m, 1H), 3.72 (m, 1H), 3.96 (m, 1H), 4.36 (dd, J=6.1, 11.2, 1H), 4.53 (dd, J=3,11.2 Hz, 1H), 7.76 (d, J = 2.7 Hz, 1H), 8.10 (d, J = 2.7 Hz, 1H). Anal. Calcd. for C₁₁H₁₄N₂OCl₂•1.0 HCl: C, 44.39; H, 5.08; N, 9.41. Found: C, 44.13; H, 5.05; N, 9.29. [α]²⁵_{D}=-5.3° (c 1.0, MeOH).

### Example 15

### 5,6-dichloro-3-(2-(S)-azetidinylmethoxy)pyridine hydrochloride

### Step 15a, 5,6-dichloro-3-(1-BOC-2-(S)-azetidinylmethoxy)pyridine

A solution of triphenylphosphine (2.6 g, 9.94 mmol) and diethyl azodicarboxylate (1.6 mL, 9.94 mmol) in THF (16 mL) was stirred at 0°C for 15 minutes. 1-BOC-2-(S)-azetidinemethanol (1.55 g, 8.28 mmol) and 5,6-dichloro-3-pyridinol (1.5 g, 9.1 mmol) were then added. The reaction mixture was allowed to warm slowly to room temperature and stir overnight. Solvent was removed, and the residue was redissolved in methylene chloride. The solution was washed with saturated aqueous K₂CO₃ and brine, dried over MgSO₄ and concentrated. The residue was chromatographed on a silica gel column, eluting with ethyl acetate:hexane (1:5) to afford the title compound (1.08 g). MS (DCI/NH₃) m/z 333 (M+H)⁺. ¹H NMR (CDCl₃, 300 MHz) δ 1.42 (s, 9H), 2.22-2.42 (m, 2H), 3.85-3.92 (m, 2H), 4.12 (dd, J=2.7, 10.1 Hz, 1H), 4.30-4.40 (m, 1H), 4.48-4.56 (m, 1H), 7.41 (d, J=2.8 Hz, 1H), 7.97 (d, J =2.8 Hz, 1H).

### Step 15b, 5,6-dichloro-3-(2-(S)-azetidinylmethoxy)pyridine hydrochloride

To 5,6-dichloro-3-(1-BOC-2-(S)-azetidinylmethoxy)pyridine from step 15a (1.06 g, 3.11 mmol) was added TFA (10 mL) in methylene chloride (10 mL) at 0°C, and the mixture was stirred for 30 minutes. The solution was allowed to warm to room temperature while stirring for an additional 45 minutes. The volatile components were then removed under vacuum. The residue was treated with saturated K₂CO₃ solution, then extracted with methylene chloride, which was dried over MgSO₄ and concentrated. The residue was chromatographed on a silica gel column, eluting with MeOH:CHCl₃:NH₄OH (1:10:0 to 1:10:0.05) to afford the free base of the title compound (475 mg, 64% yield). mp 59-60°C. MS (DCI/NH₃) m/z 233 (M+H)⁺. ¹H NMR (CDCl₃, 300 MHz) δ 2.21-2.44 (m, 2H), 3.45 (m, 1H), 3.73 (dd, J=8.4, 15.8 Hz, 1H), 3.98-4.08 (m, 2H), 4.28 (m, 1H), 7.37 (d, J=2.8 Hz, 1H), 8.01 (d, J=2.8 Hz, 1H). The base (336 mg) was slurried in ether and converted to the salt by treatment with saturated HCl in ether to give the title compound. Recrystallization from methanol/ether gave the title compound (317 mg, 81% yield). mp 181-182°C. MS (DCI/NH₃) m/z 233 (M+H)⁺. ¹H NMR (D₂O, 300 MHz) δ 2.65-2.74 (m, 2H), 4.03-4.21 (m, 2H), 4.44 (d, J=4.4 Hz, 1H), 4.95 (m, 1H), 7.79 (d, J=2.9 Hz, 1H), 8.13 (d, J=2.9 Hz, 1H). Anal. Calcd. for C₉H₁₁N₂OCl₂•1.0 HCl: C, 40.10; H, 4.11; N, 10.39. Found: C, 39.89; H, 4.08; N, 10.25.

### Example 16

### 5,6-dichloro-3-(1-methyl-2-(S)-azetidinylmethoxy)pyridine hydrochloride

A sample of 5,6-dichloro-3-(2-(S)-azetidinylmethoxy)pyridine (from Example 15b, 126 mg, 0.54 mmol) was slurried in 4 mL of H₂O and acetic acid was added (ca 3 mL) until the solution became homogeneous. An excess of formalin was added, followed by sodium cyanoborohydride until tlc showed consumption of starting material. The reaction mixture was then acidified to pH 1 with HCl, and the mixture was extracted with ether. The aqueous layer was made basic with solid K₂CO₃ and 15% NaOH, and this solution was extracted with methylene chloride. The extract was dried (MgSO₄), filtered, and concentrated *in vacuo.* The residue was purified by flash silica gel chromatography (CHCl₃:MeOH, 20:1), yielding the free base as an oil (105 mg, 79% yield). MS (DCI/NH₃) m/z 247 (M+H)⁺. ¹H NMR (CDCl₃, 300 MHz) δ 2.04-2.12 (m, 2H), 2.39 (m, 3H), 2.87 (m, 1H), 3.33-3.49 (m, 2H), 4.00 (d, J=4.8 Hz, 2H), 7.36 (d, J=2.7 Hz, 1H), 8.01 (d, J=2.7 Hz, 1H). The base was dissolved in ether and converted to the hydrochloride salt by treatment with saturated HCl in ether to give the title compound (75 mg, 66% yield). The salt was crystallized from methanol/ether. mp 144-145°C. MS (DCI/NH₃) m/z 247 (M+H)⁺. ¹H NMR (D₂O, 300 MHz) δ 2.57-2.75 (m, 2H), 2.99 (s, 3H), 3.97-4.08 (m, 1H), 4.22-4.30 (m, 2H), 4.43-4.53 (m, 2H), 4.83 (m, 1H), 7.78 (d, J=2.7 Hz, 1H), 8.13 (d, J=2.7 Hz, 1H). Anal. Calcd. for C₁₀H₁₃N₂OCl₂•1.1HCl·: C, 42.09; H, 4.66; N, 9.82. Found: C, 41.86; H, 4.57; N, 9.62.

### Example 17

### 5,6-dichloro-3-(2-(R)-azetidinylmethoxy)pyridine hydrochloride

Following the procedures of Example 15, replacing the 1-BOC-2-(S)-azetidinemethanol thereof with 1-BOC-2-(R)-azetidinemethanol (3 mmol), the title compound was prepared (212 mg, 83% yield). mp 166-168°C. MS (DCI/NH₃) m/z 233, 235, 237 (M+H)⁺. ¹H NMR (D₂O, 300 MHz) δ 2.65-2.74 (m, 2H), 4.03-4.20 (m, 2H), 4.44 (d, J=4.4 Hz, 1H), 4.91-5.00 (m, 1H), 7.79 (d, J=2.7 Hz, 1H), 8.13 (d, J=2.7 Hz, 1H). Anal. Calcd. for C₉H₁₁N₂OCl₂•1.0 HCl: C, 40.10; H, 4.11; N, 10.39. Found: C, 40.01; H, 4.02; N, 10.33. [α]²⁵_{D}=+9.5° (c 0.55, MeOH).

### Example 18

### 5,6-dichloro-3-(1-methyl-2-(R)-azetidinylmethoxy)pyridine hydrochloride

Following the procedures of Example 16, replacing the 5,6-dichloro-3-(2-(S)-azetidinylmethoxy)pyridine thereof with a sample of 5,6-dichloro-3-(2-(R)-azetidinylmethoxy)pyridine (98.6 mg, from Example 17), the title compound was prepared (45.6 mg, 74% yield). mp 136-137°C. MS (DCI/NH₃) m/z 247/249/251 (M+H)⁺. ¹H NMR (D₂O, 300 MHz) δ 2.58-2.70 (m, 2H), 2.96 (s, 3H), 3.95-4.05 (m, 1H), 4.18-4.26 (m, 2H), 4.42-4.52 (m, 2H), 4.77 (m, 1H), 7.78 (d, J=2.7 Hz, 1H), 8.13 (d, J=2.7 Hz, 1H). Anal. Calcd. for C₁₀H₁₃N₂OCl ₂•1.0 HCl·•0.1 H₂O: C, 42.09; H, 4.66; N, 9.82. Found: C, 41.70; H, 4.49; N, 9.48.

### Example 41 (not part of the invention)

### 5-nitro-3-(2-(S)-pyrrolidinylmethoxy)pyridine hydrochloride

### Step 41a. 3-benzyloxy-5-bromopyridine

NaH (60% in mineral oil) (40.9 g 1.0225 mol) in 800 mL of DMF was cooled to 0°C, and benzyl alcohol (105 mL 1.014 mol) was added slowly. The reaction mixture was stirred for 1 hour at 20°C, then 3,5-dibromopyridine (200.4 g, 846 mmol) was added and the mixture was stirred for 16 hours. The mixture was quenched with saturated NH₄Cl (500 mL), diluted with 400 mL water and extracted with Et₂O (5 x 300 mL). The combined Et₂O extracts were washed with 50 % brine (6x 300 mL) and dried (MgSO₄). The solvent was evaporated *in vacuo* and the crude product was recrystallized from Et₂O to afford 161 g (72 %) of the title product, mp 63-68 °C. ¹H NMR (CDCl₃, 300 MHz) δ 8.37-8.27 (m, 2H), 7.5-7.35 (m, 6H), 5.1 (s, 1H). MS (DCI/NH₃) m/z 264, 266 (M+H)⁺.

### Step 41b. 3-amino-5-benzyloxypyridine

The product of Example 41a (41.3 g 156 mmol), copper(I) bromide (22.43 g 156 mmol), MeOH (275 mL), and liquid NH₃ (50 mL) were combined in a stainless steel reactor and heated to 130° C for 24 hours. The mixture was allowed to cool to ambient temperature, then concentrated. The residue was suspended in 300 mL of saturated aqueous Na₂CO₃ and extracted with CH₂Cl₂ (4 x 500 mL). The combined CH₂Cl₂ extracts were washed with brine, dried (MgSO₄ ), and concentrated. The crude product was chromatographed (silica gel; hexane/ EtOAc, 9:1 to 7:3) to afford 15.6 g (50 %) of the title compound. ¹H NMR (CDCl₃, 300 MHz) δ 8.21-8.29 (m, 2H), 7.44-1.26 (m, 6H), 5.10 (s, 2H). MS (DCI/NH₃) m/z 201 (M+H)⁺.

### Step 41c. 3-amino-5-hydroxypyridine

The product of Example 41b (15.47 g, 77.25 mmol) in MeOH (25 mL) was stirred under an atmosphere of H₂ in the presence of 5% Pd/C (100 mg) for 48 hours. The mixture was filtered and concentrated, then the crude product was chromatographed (silica gel; CHCl₃/MeOH, 9:1) to afford 4.5 g (53 %) of the title compound MS (DCI/NH₃) m/z 111 (M+H)⁺, 128 (M+NH₄)⁺. ¹H NMR (CDCl₃, 300 MHz) δ 7.4 (d, J = 3 Hz, 1H), 7.3 (d, J = 2.5 Hz, 1H), 6.33 (dd, J = 2.6 Hz, 1H).

### Step 41d. 3-hydroxy-5-nitropyridine

Potassium persulfate (56.8 g 210 mmol) was ground into 31.5 mL of concd sulfuric acid, and the solution was added to a solution of the product of Example 41c (2.75 g 25 mmol) in concd sulfuric acid (27 mL). The mixture was allowed to stand for 72 hours, then was poured over ice and adjusted to pH 6 with concd NH₄OH. The solution was extracted with EtOAc (4 x 100 mL), then the EtOAc extracts were dried (MgSO₄) and concentrated. The crude product was chromatographed (silica gel; CHCl₃/ MeOH, 99:1 to 9:1) to afford 1.65 g (47 %) of the title compound. ¹H NMR (CDCl₃, 300 MHz) δ 8.81(d, J = 3 Hz, 1H), 8.51 (d, H = 3 Hz, 1H), 7.82 (dd, J = 2.5 Hz, 1H). MS (DCI/NH₃) m/z 141 (M+H)⁺, 158 (M+NH₄)⁺.

### Step 41e. 5-nitro-3-(2-(1-BOC-2-(S)-pyrrolidinyl)methoxy)pyridine

The procedures of Example 2a were followed, substituting 3-hydroxy-5-nitropyridine for 5-bromo-6-chloro-3-hydroxypyridine. Yield: 23 %. ¹H NMR (DMSO-*d*_{*6*}, 120° C, 300 MHz) δ 8.94 (d, J= 1.8 Hz, 1H), 8.68 (d, J= 2.5 Hz, 1H), 8.08 (t, J= 2.4 Hz, 1H), 4.31-4.28 (dd, J= 9.3 Hz, 1H).4.24 (dd, J=10.4 Hz, 1H), 4.09 (m 1H), 3.38 (m, 1H), 3.28 (m, 1H), 2.05 (m, 1H) 1.98-1.92 (m, 2H) 1.82 (m, 1H), 1.41 (s, 9H). MS (DCI/NH₃) m/z 324 (M+H)⁺, 341 (M+NH₄)⁺.

### Step 41f. 5-nitro-3-(2-(S)-pyrrolidinyl)methoxy)pyridine

The procedures of Example 2b were followed, replacing 5-bromo-6-chloro-3-(2-(1-BOC-2-(S)-pyrrolidinyl)methoxy)pyridine with 5-nitro-3-(2-(1-BOC-2-(S)-pyrrolidinyl)methoxy)pyridine. Yield 54%. ¹H NMR (D₂O, 300 MHz) δ 9.02 (d, J= 2 Hz, 1H), 8.66 (d, J= 2.7 Hz, 1H), 8.27 (dd, J= 2.72, 1H), 4.58 (dd, J=0.9, 1H), 4.37 (dd, J=10.9, 1H), 4.16 (m, 1H), 3.84-3.4 (m, 2H), 2.30 (m, 1H), 2.07 (m, 3H). MS (DCI/NH₃) m/z 224 (M+H)⁺, 241 (M+NH₄ )⁺. Anal. Calcd. for C₁₀H₁₃N₃O₃•1.2 HCl: C, 4.99; H 5.36; N, 15.74. Found: C, 45.08: H, 5.43; N, 15.66.

### Example 43 (not part of the invention)

### 6-Cyano-3-(2(S)-azetidinylmethoxy)pyridine hydrochloride

### Step 43a. 3-amino-6-bromopyridine

A mixture of 2-bromo-5-nitropyridine (30.75 g, 151.5 mmol), water (250 mL), and acetic acid (110 mL) was heated to 45°C. Iron powder (24.5 g, 439 mmol) was added at a rate which kept the temperature below 53°C, then the mixture was stirred at 48°C ± 5°C for one hour. The mixture was cooled to room temperature and filtered through diatomaceous earth filter aid, washing with ethyl acetate. The layers were separated and the aqueous phase was extracted with ethyl acetate. The combined organic fractions were washed with saturated Na₂CO₃ (4X 50 mL) and brine (50 mL), dried over MgSO₄, and the solvent was removed *in vacuo*. The residue was chromatographed [silica gel, hexane:EtOAc, 100:0 to 50:50) to give 20.4 g of the title compound. MS (CI/NH₃) m/e: 173 (M+H)⁺, 190 (M+NH₄)⁺· ¹H NMR (CDCl₃ 300 MHz) δ 6.86-6.90 (dd, 1H, J=8.5, 2.4 Hz) 7.21-7.23 (d, 1H, J=8.2 Hz) 7.85-7.86 (d, 1H, J=3 Hz).

### Step 43b. 3-acetoxy-6-bromopyridine.

To 25.6 mL of boron trifluoride etherate (208 mmol, Aldrich) cooled to -15°C under N₂ was added 18 g (104 mmol) of 3-amino-6-bromopyridine (from step 28a above) dissolved in 35mL of dimethoxyethane. Then *t*-Butyl nitrite (14.7 mL, 125 mmol, Aldrich) was added at a rate which kept the temperature below 0°C. Dimethoxyethane (65 mL) and methylene chloride (60 mL) were then added to aid stirring. After 10 minutes at -10°C the mixture was allowed to warm to 5°C and stirred for 30 min. Pentane (400 mL) was then added to the reaction mixture, the solid was collected by suction filtration, washed with cold ether, air dried, and dissolved in 125 mL of acetic anhydride. The resulting solution was heated to 100°C ± 5°C for 1 hour. The solvent was removed *in vacuo,* and the residue was suspended in saturated aqueous Na₂CO₃, and extracted with ethyl ether. The ether solution was dried over MgSO₄, the solvent was removed *in vacuo,* and the residue was chromatographed on silica gel, eluting with 100:0 to 60:40 hexane:ethyl acetate to give 13.6 g of the title compound. ¹H NMR (CDCl₃ 300 MHz) δ 8.20 (m, 1H).7.51 (d, J = 8.5 Hz 1H),7.38 (dd, J = 2.9, 7.5 Hz, 1H), 2.35 (s, 3H). MS (CI/NH₃) m/e: 216 (M+H)⁺, 233 (M+NH₄)⁺.

### Step 43c. 2-Bromo-5-hydroxypyridine.

The product of Example 43b (12.8 g, 60 mmol was dissolved in 15% aqueous NaOH (50 mL) at 0°C, and the solution was allowed to warm to room temperature and stirred for 60 minutes. After complete consumption of the starting material the solution was neutralized by addition of HCl The aqueous mixture was extracted with ethyl acetate (3 X 200 mL). The organic extracts were washed with water (2 X 50 mL) and brine (4 X 50 mL), then dried (MgSO₄), and the solvent was evaporated to yield 9.8 g of the title compound. ¹H NMR (CDCl₃, 300 MHz) δ 7.12-7.16 (dd, 1H, J=3.2 Hz),7.36-7.39 (d, 1H, J=8.5Hz), 8.04-8.05 (d, 1H, J=2.4 Hz). MS m/e: 174 (M+H)⁺

### Step 43d. 6-Bromo-3-(1-BOC-2-(S)-azetidinylmethoxy)pyridine

The product of Example 43c was coupled to 1-BOC-2-(S)-azetidinemethanol using the procedure described in Example 2a. ¹H NMR (CDCl₃, 300 MHz): 1.42 (s, 9H), 2.20-2.43 (m, 2H), 4.88 (t, J=8.0 Hz, 2H), 4.17 (dd, J=3.0, 9.0 Hz, 1H), 4.30-4.39 (m, 1H), 4.43-4.58 (m,1H), 7.42 (t, J=2.0 Hz, 1H), 8.25-8.32 (m, 2H). MS (DCI/NH₃) m/e: 343 (M+H)⁺, 360 (M+NH₄)⁺.

### Step 43e. 6-Cyano-3-(1-BOC-2-(S)-azetidinylmethoxy)pyridine

To the product of Example 43d (1.22g, 3.60mmol) in degassed DMF (10 mL) were added zinc cyanide (0.295 g, 2.50 mmol) and tetrakis(triphenylphosphine)palladium(0) (0.249 g, 0.20mmol) and the mixture was heated at 80°C for 5 hours. The mixture was cooled to room temperature and poured into saturated sodium bicarbonate. The aqueous layer was extracted with EtOAc (400 mL), dried (MgSO₄) and concentrated in vacuo. The crude product was chromatographed (silica gel; EtOAC/hexane 1/1) to afford a colorless oil (0.784 g, 75%).
¹H NMR (CDCl₃, 300 MHz) δ 1.42 (s, 9H), 2.22-2.42 (m, 2H), 3.82-3.87 (m, 2H), 3.18 (dd, J=3.0, 9.0 Hz, 1H), 4.38-4.45 (m, 1H), 4.48-4.60 (m, 1H), 7.32-7.58 (m, 1H), 7.62 (d, J=11.5 Hz, 1H), 8.42 (d, J=4.0 Hz, 1H). MS (DCI/NH₃) m/e: 290 (M+H)⁺ 307 (M+NH₄)⁺.

### Step 43f. 6-Cyano-3-(2(S)-azetidinylmethoxy)pyridine hydrochloride

The product of Example 43e was deprotected and converted to the hydrochloride salt according to the procedure described in Example 2b. ¹H NMR (CDCl₃) δ 2.66-2.74 (m, 2H), 4.02-4.19 (m, 2H), 4.50 (d, 2H, J=4.4 Hz), 4.84-4.99 (m, 1H), 7.63 (dd, 1H, J=3.0, 11.5 Hz), 7.97 (d, 1H, J=8.8 Hz), 8.48 (d, 1H, J=3.0 Hz). MS (CI/NH3): m/z 190.00 (M+H⁺), 207.00 (M+NH₄⁺). Anal Calcd. for C₁₀H₁₁N₃O•1.0 HCl·0.1 Et₂O•0.1 H₂O: C, 53.18; H, 5.66; N, 17.89. Found: C, 53.07; H, 5.46; N, 17.87.

### Example 44 (not part of the invention)

### 5-cyano-3-(2-(S)-azetidinylmethoxy)pyridine hydrochloride

### Step 44a. 3-Bromo-5-hydroxypyridine.

3-Benzyloxy-5-bromopyridine from Example 41a was heated at reflux with 48% HBr/HOAc (60 mL) for 16 hours. The reaction was quenched with excess NaHCO₃, the basic mixture was extracted with ethyl acetate, and the extract was dried over Na₂SO₄. The solvent was removed, and the residue was chromatographed (silica gel; MeOH/CCl₄, 1/10) to afford the title compound. ¹H NMR (CDCl₃, 300 MHz) δ 8.27 (d, J=1.8 Hz, 1H), 8.23 (d, J=2.6 Hz, 1H), 7.44 (dd, J=1.8, 2.6 Hz, 1H). MS (DCI/NH3) m/z 174, 176 (M+H)⁺, 191, 193 (M+NH₄)⁺.

### Step 44b. 5-Bromo-3-(2-(1-BOC-2-(S)-azetidinyl)methoxy)pyridine

Triphenylphosphine (4.01 g, 15.3 mmol) and DEAD (2.43 mL, 15.3 mmol) were dissolved in 30 mL of THF at 0 °C, and the mixture was stirred for 10 minutes. Samples of 1-BOC-2-(S)-azetidinemethanol (2.86 g, 15.3 mmol) and the product of Example 44a (1.505 g, 10.2 mmol) were added, and the mixture was stirred for 40 hours at room temperature. The volatile components were removed under vacuum, and the residue was triturated with hexane. The separated hexane fraction was concentrated, and the residue was chromatographed on a silica gel column, eluting with hexane/ether (10:1 to 10:2) to afford the title compound as a colorless oil (1.669 g). ¹H NMR (CDCl₃, 300 MHz) δ 1.42 (s, 9H), 2.31 (m, 2H), 3.89 (m, 2H), 4.12 (m, 1H), 4.322 (m, 1H), 4.52 (m, 1H), 7.43 (m, 1H), 8.29 (m, 2H). MS (CI/NH₃) m/z 344 (M+H)⁺.

### Step 44c. 5-Cyano-3-(2-(1-BOC-2-(S)-azetidinyl)methoxy)pyridine

The procedure of Example 43d was followed, replacing the product of Example 43c with the product of Example 44b. The crude product was chromatographed (silica gel; CH₂Cl₂/ MeOH/concd NH₄OH, 10:1:0.1) to afford 1.3 g (86%) of the title compound. ¹H NMR (CDCl₃, 300 MHz): δ 1.42 (s, 9H), 2.23-2.43 (m, 2H), 3.85-3.87 (m, 2H), 3.18 (dd, J=3.0, 9.0 Hz, 1H), 3.35-3.42 (m, 1H), 4.48-4.58 (m, 1H), 7.45-7.50 (m, 1H), 8.50 (d, J=1.5 Hz, 1H), 8.55 (d, J=4.0 Hz, 1H).
MS (DCI/NH₃) m/e: 290 (M+H)⁺, 307(M+NH₄)⁺.

### Step 44d. 5-Cyano-3-(2-(S)-azetidinylmethoxy)pyridine hydrochloride

The product of Example 44c was deprotected and converted to the hydrochloride salt according to the procedure described in Example 2b. ¹H NMR (D₂O) δ 2.63-2.78 (m, 2H), 4.03-4.22 (m, 2H), 4.48 (d, 2H, J=4.0 Hz), 4.95-5.03 (m, 1H), 7.90-7.95 (m, 1H), 8.58 (d, 1H, J=2.0 Hz), 8.62 (d, 1H, J=3.0 Hz). MS (CI/NH3): m/z 190.00 (M+H⁺), 207.00 (M+NH₄⁺). Anal. Calcd. for C₁₀H₁₁N₃O•1.1 HCl•0.10 H₂O: C, 51.97; H, 5.36; N, 18.18. Found: C, 52.11; H, 5.27; N, 17.95.

### Example 45

### 5-Ethyl-6-chloro-3-(2-(S)-azetidinylmethoxy)pyridine hydrochloride

### Step 45a. 5-Vinyl-6-chloro-3-(1-BOC-2-(S)-azetidinylmethoxy)pyridine

To 5-bromo-6-chloro-3-(1-BOC-2-(S)-azetidinylmethoxy)pyridine from Example 2a (1.00g, 2.65mmol) in toluene (30mL) was added tetrakis(triphenylphosphine) palladium (0) (93mg, 0.081mmo) and vinyltributyltin (0.93mL, 3.18mmol). The mixture was stirred and heated at 95° C overnight, then the solvent was evaporated. The residue was chromatographed (silica gel; CH₂Cl₂/MeOH, 100:2) to afford an oil (720mg, 84%).¹H NMR (CDCl₃, 300MHz) δ 1.42 (s, 9H), 2.33 (m, 2H), 3.89 (t, J=8.5 Hz, 2H), 4.14 (m, 1H), 4.36 (m, 1H), 4.52 (m, 1H), 5.50 (d, J=10.9 Hz, 1H), 5.80 (d, J=17 Hz, 1H), 6.98 (dd, J=17.6 Hz, J=11.2 Hz, 1H), 7.44 (d, J=2.7 Hz, 1H), 8.02 (d, J=2.7 Hz, 1H). MS (CI/NH₃) m/e: 325 (M+H)⁺.

### Step 45b. 5-Ethyl-6-chloro-3-(1-BOC-2-(S)-azetidinylmethoxy)pyridine

The product from Example 45a (440 mg, 1.36 mmol) in MeOH (10mL) was stirred overnight under 1 atm of H₂ in the presence of 5% Pt-C (440mg). The mixture was filtered and the filtrate was concentrated to afford a colorless oil (219mg, 51%). NMR (CDCl₃, 300 MHz) δ 1.25 (t, J=7.46Hz, 3H), 1.42 (s, 9H), 2.32 (m, 2H), 2.71 (q, J=7.5Hz, 2H), 3.89 (t, J=7.8 Hz, 2H), 4.12 (dd, J=3.0, 9.8 Hz, 1H), 4.30 (m, 1H), 4.50 (m, 1H), 7.16 (d, J=3.1 Hz, 1H), 7.94 (d, J=3.0 Hz, 1H). MS (CI/NH₃) m/z 327 (M+H)⁺.

### Step 45c. 5-Ethyl-6-chloro-3-(2-(S)-azetidinylmethoxy)pyridine hydrochloride

To the product from Example 45b (216 mg, 0.66 mmol) in CH₂Cl₂ (2 mL) at 0 °C was added trifluoroacetic acid (1.8 mL). The solution was stirred and allowed to warm to room temperature, then adjusted to pH 11 with aqueous 10% NaOH, and extracted with CH₂Cl₂. The organic layer was dried over MgSO₄ and concentrated. The residue was chromatographed (silica gel; CH₂Cl₂/MeOH, 100:3 to 100:15) to afford an oil (60mg, 40%). NMR (CDCl₃, 300 MHz) δ 1.22(m 3H), 2.38 (m, 2H), 2.71 (q, J=7.5Hz, 2H), 3.57 (m, 1H), 3.80 (m, 1H), 4.08 (m, 2H), 4.38 (m, 1H), 7.15 (d, J=2.4 Hz, 1H), 7.92 (d, J=3.0 Hz, 1H). MS (CI/NH₃) m/e: 227 (M+H)⁺. The free base was dissolved in THF and treated with 1 M HCl in Et₂O to afford the salt, which was triturated with Et₂O and dried under vacuum. mp 102-104 °C . ¹H NMR (D₂O) δ 1.24 (t, 3H, J=7.5 Hz), 2.71 (m, 4H), 4.11 (m, 2H), 4.42 (d, 2H, J=4 Hz), 4.95 (m, 1H), 7.51 (d, 1H, J=3 Hz), 8.00 (d, 1H, J=3 Hz). Anal. Calcd. for C₁₁H₁₅N₂OCl•1.1 HCl: C, 49.52; H, 6.08; N, 10.50. Found: C, 49.63; H, 5.89; N, 10.20.

### Example 46

### 5-Ethyl-6-chloro-3-(2-(S)-pyrrolidinylmethoxy)pyridine hydrochloride

### Step 46a. 5-Vinyl-6-chloro-3-(1-BOC-2-(S)-pyrrolidinylmethoxy)pyridine

5-Bromo-6-chloro-3-(1-BOC-2-(S)-pyrrolidinylmethoxy)pyridine from Example 4a (0.95 g, 2.4 mmol) and tetrakis(triphenylphosphine)palladium(0) (0.14g, 0.12 mmol) were dissolved in toluene (50 mL) and the solution was purged with nitrogen gas for 5 minutes. Vinyl tributyltin (0.78 mL, 2.67 mmol) was added and the mixture was heated to 90°C for 1 day. The solvent was evaporated and the residue was chromatographed (silica gel; hexanes/EtOAc, 4:1) to provide 0.69 g (85%) of the title compound. TLC R_{f} 0.3 (4:1 hexanes/EtOAc). MS (DCI/NH3) m/z 339 (M+H)⁺. Continued elution provided a minor amount of 5,6-divinyl-3-(1-BOC-2-(S)-pyrrolidinylmethoxy)pyridine (0.044g, 5.5%). TLC R_{f} 0.25 (4:1 hexanes/EtOAc). MS (DCI/NH3) m/z 331 (M+H)⁺.

### Step 46b. 5-Ethyl-6-chloro-3-(1-BOC-2-(S)-pyrrolidinylmethoxy)pyridine

A solution of the compound of Example 46a (0.33 g, 0.97 mmol) in MeOH (20 mL) was stirred under 1 atm of hydrogen in the presence of 10% Pd/C for 4 days. The mixture was filtered and the solvent was evaporated. The residue was chromatographed (silica gel ; hexanes/EtOAc, 4:1) to provide 0.24 g (72%) of the title compound. TLC R_{f} 0.5 (2:1 hexanes/EtOAc). MS (DCI/NH3) m/z 341 (M+H)⁺.

### Step 46c. 5-Ethyl-6-chloro-3-(2-(S)-pyrrolidinylmethoxy)pyridine hydrochloride

The product of example 46b (115 mg, 0.34 mmol) was treated with 4N HCl (10 mL) in dioxane at 0 °C. The solution was allowed to warm to ambient temperature for 30 minutes, then ether was added and the resulting solid was collected, rinsed with fresh ether and dried in vacuo to provide 85 mg (quantitative yield) of the title compound, mp 144-1455 °C . [α]_{D} -4.5° (c 0.44, MeOH). ¹H NMR (CD₃OD) δ 1.26 (t, 3H, J=7 Hz), 2.03-2.27 (m, 3H), 2.34-2.44 (m, 1H), 2.77 (q, 2H, J=7 Hz), 3.06 (s, 3H), 3.72 (br s, 1H), 3.91 (br s, 1H), 4.32 (dd, 1H, J=7,11 Hz), 4.50 (dd, 1H, J=4,11 Hz), 7.49 (d, 1H, J=3 Hz), 8.03 (d, 1H, J=3 Hz). MS (CI/NH3): m/z 255 (M+H⁺). Anal. Calcd. for C₁₃H₁₉ClN₂O•HCl: C, 53.62; H, 6.92; N, 9.62. Found: C, 53.58; H, 6.72; N, 9.51.

### Example 47

### 5-Ethyl-6-chloro-3-(1-methyl-2-(S)-pyrrolidinylmethoxy)pyridine hydrochloride

The product of Example 45b was treated with formalin (6 mL) and formic acid (3 mL) at 80°C for 4 hours. The mixture was cooled to ambient temperature and poured into saturated sodium bicarbonate solution. The solution was extracted with EtOAc, and the organic layer was washed with water and dried (MgSO₄). The solvent was evaporated and the crude product was dissolved in ether and treated with 1M HCl in ether (1 mL). The resulting solid was filtered and washed with fresh ether to provide 41 mg (0.14 mmol, 38%) of the title compound. An additional 44 mg (42%) of the product was recovered by similar treatment of the mother liquors. mp 188-9 °C. [α]_{D} +15° (c 0.3, MeOH). ¹H NMR (CD₃OD) δ 1.42 (t, 3H, J=7 Hz), 2.02-2.13 (m, 1H), 2.20-2.33 (m, 2H), 2.39-2.49 (m, 1H), 2.91 (q, 2H, J=7 Hz), 3.53 (bt, 2H, J=7 Hz), 4.15-4.25 (m, 1H), 4.34 (dd, 1H, J=8,10 Hz), 4.58 (dd, 1H, J=3,10 Hz), 7.62 (d, 1H, J=3 Hz), 8.13 (d, 1H, J=3 Hz). MS (CI/NH3): m/z 241 (M+H⁺). Anal. Calcd. for C₁₂H₁₇ClN₂O•1.1 HCl: C, 51.32; H, 6.50; N, 9.97. Found: C, 51.47; H, 6.45; N, 9.77.

### Example 48

### 5-Ethyl-6-chloro-3-(2-(R)-pyrrolidinylmethoxy)pyridine hydrochloride

### Step 48a. 5-Vinyl-6-chloro-3-(1-BOC-2-(R)-pyrrolidinylmethoxy)pyridine

The procedure of Example 45a was followed, replacing the starting material 5-bromo-6-chloro-3-(1-BOC-2-(S)-azetidinylmethoxy)pyridine with 5-bromo-6-chloro-3-(1-BOC-2-(R)-pyrrolidinylmethoxy)pyridine from Example 7. Yield: 71%. ¹H NMR (CDCl₃, 300 MHz) δ 1.47 (s, 9H), 1.88 (m, 2H), 2.02 (m 2H), 3.40 (m, 2H), 4.00 (m, 1H), 4.18 (m, 2H), 5.50 (d, J=11.2 Hz, 1H), 5.85 (m, 1H), 6.98 (dd, J=10.8, 17.3 Hz, 1H), 7.58 (br s, 1H), 7.99 (d, J=3.0 Hz, 1H). MS (CI/NH₃) m/e: 339 (M+H)⁺.

### Step 48b. 5-Ethyl-6-chloro-3-(1-BOC-2-(R)-methoxy)pyridine

The product of Example 48a was treated under the conditions of Example 45b. Yield: 57%. NMR (CDCl₃, 300 MHz) δ 1.25 (t, J=7.5 Hz, 3H), 1.47 (s, 9H), 1.88 (m, 2H), 2.02 (m, 2H), 2.69 (q, J=7.5 Hz, 2H), 3.38 (m, 2H), 3.90 (m, 1H), 4.15 (m, 2H), 7.17 (m, 1H), 7.92 (d, J=2.4 Hz, 1H). MS (CI/NH₃) m/e: 341 (M+H)⁺.

### Step 48c. 5-Ethyl-6-chloro-3-(2-(R)-pyrrolidinylmethoxy)pyridine hydrochloride

The product of Example 48b was treated under the conditions of Example 45c. Yield of free base: 100%. ¹H NMR (CDCl₃, 300MHz) δ 1.22 (t, J=7.5 Hz, 3H), 1.85 (m, 1H), 2.04 (m, 2H), 2.15 (m, 1H), 2.68 (q, J=7.4 Hz, 2H), 3.19 (m, 2H), 3.84 (m, 1H), 4.11 (m, 2H), 7.12 (d, J=3.0 Hz, 1H), 7.89 (d, J=2.7 Hz, 1H). MS (CI/NH₃) m/z : 241 (M+H)⁺. The free base was converted to the hydrochloride salt as described in Example 45c: mp 185-187 °C. ¹H NMR (D₂O) δ 1.23 (t, 3H, J=7.5 Hz), 1.95 (m, 1H), 2.13 (m, 2H), 2.74 (q, 2H, J=7.5 Hz), 3.43 (t, 2H, J=7.2 Hz), 4.12 (m, 1H), 4.24 (dd, 2H, J=7.8, 10.5 Hz), 4.46 (dd, 1H, J=3.4, 10.5 Hz), 7.45 (d, 1H, J=3.0 Hz), 7.94 (d, 1H, J=3.0 Hz). MS (CI/NH3): m/z 241 (M+H⁺), 258 (M+NH₄⁺). Anal. Calcd. for C₁₂H₁₇N₂OCl•HCl: C, 52.00; H, 6.55; N, 10.11. Found: C, 51.73; H, 6.44; N, 9.82.

### Example 49

### 5-Cyano-6-chloro-3-(2-(S)-azetidinylmethoxy)pyridine hydrochloride

### Step 49a. 5-Cyano-6-chloro-3-(1-BOC-2-(S)-azetidinylmethoxy)pyridine

The procedure of Example 43d was followed, replacing the product of Example 43c with 5-Bromo-6-chloro-3-(1-BOC-2-(S)-azeridinylmethoxy)pyridine from Example 2 . The crude product was chromatographed (silica gel; CH₂Cl₂/ MeOH/concd NH₄OH, 10:1:0.1) to afford 0.61 g (39%) of the title compound: ¹H NMR (CDCl₃, 300 MHz) δ 1.41 (s, 9H), 2.01-2.23 (m, 2H), 3.78-3.95 (m, 2H), 4.17 (dd, J=3.0, 11.0 Hz, 1H), 4.35-4.45 (m, 1H), 4.47-4.58 (m, 1H). 7.58 (d, J = 5.0 Hz, 1H), 8.35 (d, J = 3.5 Hz, 1H). MS (DCI/NH₃) m/e: 324 (M+H)⁺, 341 (M+NH₄)⁺.

### Step 49b. 5-Cyano-6-chloro-3-(2-(S)-azetidinylmethoxy)pyridine hydrochloride

The product of Example 49a was deprotected and converted to the hydrochloride salt according to the procedure of Example 2b. ¹H NMR (D₂O) δ 2.65-2.73 (m, 2H), 4.02-4.20 (m, 2H), 4.46 (d, 1H, J=6.0 Hz), 4.93-4.99 (m, 1H), 8.00 (d, 1H, J=3.0 Hz), 8.42 (d, 2H, J=3 Hz). MS (CI/NH3): m/z 224 (M+H⁺), 241 (M+NH₄⁺). Anal. Calcd. for C₁₀H₁₀N₃OCl•HCl: C, 46.17; H, 4.26; N, 16.15. Found: C, 46.28; H, 4.18; N, 15.93.

### Example 50

### 5-Cyano-6-chloro-3-(1-methyl-2-(S)-azetidinylmethoxy)pyridine hydrochloride

To the product of Example 49b (0.160 g, 0.70mmol) were added H₂O (5.0 mL), HOAc (0.75 mL), formalin (2.25 mL) and NaCNBH₃ (0.132 g, 2.10 mmol) and the mixture was stirred at room temperature for 1 hour. The reaction was basified with 15% NaOH and the aqueous solution was extracted with CH₂Cl₂ (250 mL). The combined organic extracts were dried (MgSO₄) and concentrated in vacuo. The crude material was chromatographed (silica gel; MeOH/CH₂Cl₂, 9/1) to afford a colorless oil (0.089g, 53%). Treatment with hydrogen chloride in ether afforded the hydrochloride as a white solid. ¹H NMR (D₂O) δ 2.57-2.78 (m, 2H), 3.0 (s, 3H), 3.83-4.12 (m, 1H), 4.19-4.36 (m, 1H), 4.43-4.60 (m, 2H), 4.78-4.90 (m, 1H), 8.01 (d, 1H, J=6.0 Hz), 8.22 (d, 1H, J=3.0 Hz). MS (DCI/NH3): m/z 238.00 (M+H⁺). for C₁₁H₁₂N₃OCl•HCl•0.2 H2O: C, 47.53; H, 4.81; N, 15.18. Found: C, 47.57; H, 4.86; N, 15.13.

### Example 51

### 5-Carbamoyl-6-chloro-3-(1-methyl-2-(S)-azetidinylmethoxy)pyridine hydrochloride

### Step 51a. 5-Carbamoyl-6-chloro-3-(1-BOC-2-(S)-azetidinylmethoxy)pyridine

To the product of Example 49a (0.535 g, 1.70 mmol) were added 30% H₂O₂ (0.6347mL, 5.50mmol) and 6N NaOH (0.670mL, 4.0mmol) and the mixture was stirred at room temperature for 45 minutes. The mixture was then heated at 45°C for 75 minutes, cooled to room temperature, and poured into 15% NaOH. The aqueous solution was extracted with CH₂Cl₂ (250 mL), and the combined extracts were dried (MgSO₄) and concentrated in vacuo. The crude material was chromatographed (silica gel; EtOAc/hexane, 1:2) to afford a colorless oil (0.414 g, 72%). ¹H NMR (CDCl₃, 300 MHz): δ 1.42 (s, 9H), 2.28-2.36 (m, 2H), 3.86-3,91 (m, 2H), 4.37-4.42 (m, 1H), 4.50-4.59 (m, 1H), 7.84 (d, J=3.4 Hz, 1H), 8.22 (d, J=3.0 Hz, 1H). MS (DCI/NH₃) m/e: 342, (M+H)⁺, 359, (M+NH₄)⁺.

### Step 51b. 5-Carbamoyl-6-chloro-3-(1-methyl-2-(S)-azetidinylmethoxy)pyridine hydrochloride.

The product of Example 51a was treated as in Example 2b to afford the title compound as a hygroscopic oil. ¹H NMR (CD₃OD) δ 2.50-2.65 (m, 2H), 3.91-3.99 (m, 2H), 4.35-4.40 (m, 2H), 4.70-4.80 (m, 1H), 7.62 (d, 1H, J=5.0 Hz), 8.22 (d, 1H, J=5.0 Hz). MS (APCI): m/z 242.00 (M+H⁺). Anal. Calcd. for C₁₀H₁₂N₃O₂Cl•1.5 HCl•0.2 Et₂O: C, 41.68; H, 5.02; N, 13.50. Found: C, 41.71; H, 5.04; N, 13.54.

### Example 52

### 5-bromo-6-methyl-3-(2-(S)-azetidinylmethoxy)pyridine hydrochloride

### Step 52a. 3-bromo-2-methyl-5-nitropyridine

3-Bromo-2-chloro-5-nitropyridine (25 g, 105 mmol; prepared from 2-hydroxy-5-nitropyridine according to the procedure of V. Koch and S. Schnatterer, *Synthesis* **1990**, 499-501) was treated with the sodium salt of diethylmalonate (17.6 mL, 116 mmol) according to the procedure of Odashima *et al*., *Bull. Chem. Soc. Jpn.,* **1993,** 66: 797-803) to provide 17.1 g (78.8 mmol, 75%) of a dark red oil. TLC Rf 0.5 (4:1 hexanes/EtOAc). ¹H NMR (CDCl3, 300 MHz) δ 2.81 (s, 3H), 8.61 (d, 1H, J=2Hz), 9.26 (d, 1H, J=2Hz).

### Step 52b. 5-amino-3-bromo-2-methylpyridine

The compound of Example 52a (17.1g, 78.8 mmol) was dissolved in HOAc (50 mL) and water (150 mL) and treated with iron powder (13.3 g, 236 mmol) added in portions over 2 hours. The mixture was filtered and the filter cake was washed with EtOAc. The layers were separated and the aqueous phase was extracted with EtOAc. The combined organic fractions were washed with 1M sodium bicarbonate and water, then dried (MgSO₄) and concentrated to afford 12.65 g (86%) of the title compound. TLC R_{f} 0.25 (2:1 hexanes/EtOAc). MS (DCI/NH3)m/z 187 (M+H)⁺, 204 (M+NH₄)⁺.

### Step 52c. 5-Acetoxy-3-bromo-2-methylpyridine

The compound of Example 52b (12.6 g, 67 mmol) was treated with t-butyl nitrite and BF₃•OEt₂ followed by acetic anhydride according to the procedure of Example 43b. The crude product was chromatographed (silica gel; hexanes/EtOAc, 4:1) to afford the title compound (12.0 g, 58%). TLC Rf 0.5 (2:1 hexanes/EtOAc). MS (DCI/NH3) m/z 230 (M+H)⁺, 188.

### Step 52d. 3-bromo-5-hydroxy-2-methylpyridine

The product of Example 52c was stirred with 15% NaOH (75 mL) at 0 °C and then allowed to warm to ambient temperature. After 1 hour, the mixture was acidified with 6N HCl with cooling, and the resulting suspension was extracted with EtOAc. The EtOAc was washed with H₂O, dried (MgSO₄) and concentrated to provide 7.0 g (95%) of the title compound. TLC R_{f} 0.25 (2:1 hexanes/EtOAc). ¹H NMR (CDCl₃, 300 MHz) δ 2.59 (s, 3H), 7.46 (d, 1H, J=2Hz), 8.10 (d, 1H, J=2Hz). MS (DCI/NH3) m/z 188 (M+H)⁺, 207 (M+NH₄)⁺.

### Step 52e. 5-bromo-6-methyl-3-(1-BOC-2-(S)-azetidinylmethoxy)pyridine

Triphenylphosphine (6.3 g, 24 mmol) was dissolved in THF (100 mL), cooled to 0°C and treated with DEAD (3.8 mL, 24 mmol) for 15 minutes. Then the compound of Example 52d (3g, 16 mmol) and 1-BOC-2-(S)-azetidinemethanol(3.4 g, 18 mmol) were added, and the mixture was allowed to warm slowly to ambient temperature. After 3 days, the solvent was evaporated and the residue was chromatographed (silica gel; hexanes/EtOAc, 4:1) to provide the title compound as an oil, contaminated with byproduct derived from DEAD reagent. For the tide compound: TLC R_{f} 0.6 (1:1 hexanes/EtOAc). MS (DCI/NH₃) m/z 357 (M+H)⁺, 279.

### Step 52f. 5-bromo-6-methyl-3-(2-(S)-azetidinylmethoxy)pyridine hydrochloride

The product of example 52e (0.4 g, 1.12 mmol) was dissolved in methylene chloride (4 mL) and treated with TFA (2 mL) at 0°C for 1 hour. The solution was concentrated, and the residue was mixed with saturated bicarbonate and extracted with methylene chloride. The combined organic layers were washed with H₂O, and dried (MgSO₄). Evaporation of the solvent provided 0.25 g (76%) of neutral product., which was dissolved in ether and treated with 1N HCl in ether. The resulting solid was collected and washed with fresh ether to provide 151 mg (41%) of the title compound: mp 153-155 °C; [α]_{D} -7.4 (c 0.54, MeOH); ¹H NMR (CD₃OD) δ 2.63-2.76 (m, 2H), 2.78 (s, 3H), 4.04-4.18 (m, 2H), 4.50-4.63 (m, 2H), 4.88-4.96 (m, 1H), 8.50 (d, 1H, J=2 Hz), 8.10 (d, 1H, J=2 Hz); MS (CI/NH3): m/z 257 (M+H⁺), 274 (M+NH₄⁺); Anal. Calcd. for C₁₀H₁₃N₂OBr •2.0 HCl: C, 36.39; H, 4.58; N, 8.49. Found: C, 36.31; H, 4.66; N, 8.41.

### Example 55

### 6-Fluoro-5-methyl-3-(2-(S)-azetidinylmethoxy)pyridine hydrochloride

### Step 55a. 2-fluoro-3-methyl-5-nitropyridine

2-Chloro-3-methyl-5-nitropyridine ( 15 g, 86.9 mmol; from Maybridge Chemical Co.), KF (12 g, 258 mmol), and tetraphenylphosphonium bromide (20 g, 47.7 mmol) were combined in 200 mL of acetonitrile and heated at reflux for 4 days. The mixture was diluted with Et₂O (500 mL), filtered, and the solution was concentrated. The residue was triturated with hot hexane (4 x 200 mL), then the combined hexane solutions were concentrated to give 8.4 g (60%) of the title compound. ¹H NMR (DMSO-*d*_{*6*}, 300 MHz) δ 8.95 (dd, J= 1.6 Hz, 1H), 8.43 (m, 1H), 2.42 (s, 1H). MS (DCI/NH₃) m/z 157 (M+H)⁺.

### Step 55b. 3-Amino-6-fluoro-5-methylpyridine

2-fluoro-3-methyl-5-nitropyridine was combined with 100 mg of 5% Pd/C in EtOH (100 mL) and the mixture was stirred under a H₂ atmosphere for 16 hours. The mixture was filtered and concentrated. The crude product was chromatographed (silica gel; CHCl₃/MeOH, 99:1 to 94:6) to yield 5.2 g (78% ) of the title compound. ¹H NMR (DMSO-*d*_{*6*}, 300 MHz) δ 7.26 (t, J= 2.7 Hz, 1H), 6.95 (dd, J= 8.1 Hz, 1H), 5.11 (br, s, 2H), 2.10 (s, 3H). MS (DCI/NH₃) m/z 127 (M+H)⁺, 144 (M+NH₄)⁺.

### Step 55c. 3-acetoxy-6-fluoro-5-methylpyridine

To boron trifluoride etherate (10 mL, 81 mmol) at -15°C under N₂ was added the product of step 55b (5.1g, 40 mmol) in DME (30 mL). *t*-Butyl nitrite (5.5 mL, 46 mmol, Aldrich) was added at a such a rate that the temperature remained below 0 °C. Additional DME (25 mL) was then added. After 10 minutes at -10°C the reaction was allowed to warm to 5°C and stirred for 30 min. Pentane (400 mL) was then added to the reaction mixture, the solid was collected by suction filtration, washed with cold ether, air dried, and dissolved in 100 mL acetic anhydride. The resulting solution was heated to 77° C ± 5°C for 1 hour. The solvent was removed *in vacuo*, and the residue was suspended in saturated aqueous Na₂CO₃ (200 mL) and extracted with ethyl ether (2 x150). The ether solution was dried (MgSO4) and concentrated The crude product was chromatographed (silica gel; hexane/EtOAc 9:1 to 7:3) yield 3.62g (53% ) of the tide compound. MS m/e: 170 (M+H)⁺, 187 (M+NH₄)⁺. ¹H NMR (CDCl₃ 300 MHz) δ 7.8 (m, 1H) 7.34 (m, 1H), 2.32 (s, 3H), 2.29 (s, 3H).

### Step 55d. 2-Fluoro-5-hydroxy-3-methylpyridine

The product of step 55c (3.6 g, 21.3 mmol) was dissolved in 20% aqueous NaOH (25 mL). After complete consumption of the starting material the solution was neutralized by addition of HCL The aqueous mixture was extracted with ethyl acetate (2 X 150 mL). The organic extracts were dried (MgSO4), and the solvent was evaporated. The crude product was triturated with hexane to yield 2.35 g (86.9%) of the title compound. MS m/e: 128 (M+H)⁺, 145 (M+NH₄)⁺; ¹H NMR (CDCl₃, 300 MHz) δ 7.61(t, 1H, J=2.2 Hz), 7.17 (m, 1 Hz), 2.25 (s, 3H)

### Step 55e. 6-Fluoro-5-methyl-3-(1-Cbz-2-(S)-azetidinylmethoxy)pyridine

The procedure of example 2a was followed, substituting 2-fluoro-5-hydroxy-3-methylpyridine and 1-Cbz-2-(S)-azetidinemethanol for 5-bromo-9-chloropyridine-3-ol and 1-BOC-2-(S)-azetidinemethanol, respectively. Yield 60 %. MS (DCI/NH₃) m/z 331 (M+H)⁺, 348 (M+NH₄)^{+ 1}H NMR (CDCl₃ 300 MHz) : 7.63 (br s, 1H), 7.29 (m, 5H), 7.18 (br s, 1H), 5.05 (m, 2H), 4.59 (m, 1H), 4.3 (br s, 1H), 4.09 (m, 1H), 3.99 (m, 1H), 2.26-2.05 (m, 2H), 2.05 (s, 3H).

### Step 55f. 6-Fluoro-5-methyl-3-(2-(S)-azetidinylmethoxy)pyridine benzoate

The procedure of example 39c was followed, substituting 6-fluoro-5-methyl-3-(1-Cbz-2-(S)-azetidinylmethoxy)pyridine for 2-fluoro-3-(1-Cbz-2-(S)-azetidinylmethoxy)pyridine, and benzoic acid for HCl, resulting in a 53% yield of the title compound as an off-white solid: mp 104-108 °C; [α]_{D}-5.545 (c 0.55, MeOH); ¹H NMR (DMSO) δ 7.90(m, 2H), 7.70 (s, 1H), 7.51-7.48 (m, 2H), 7.42-7.39 (t, 2H, J=7.2 Hz), 4.27(m, 1H), 4.17(dd, 1H, J=7.3, 10.4 Hz), 4.08 (dd, 1H, J=4.9, 10.4 Hz), 3.79 (m, 1H), 3.47 (m, 1H), 2.35 (m, 1H), 2.19 (s, 3H), 2.16 (m, 1H). MS (DCI/NH3): m/z 197 (M+H⁺), 214 (M+NH₄⁺). Anal. Calcd. for C₁₀H₁₃N₂OF•C₇H₆O₂: C, 64.14; H, 6.02; N, 8.80. Found: C, 63.90; H, 6.10; N, 8.70.

### Example 56

### 6-Chloro-5-methyl-3-(2-(S)-azetidinylmethoxy)pyridine hydrochloride

### Step 56a. 3-Amino-6-chloro-5-methylpyridine

2-Chloro-3-methyl-5-nitropyridine (3.2 g, 18.5 mmol; from Maybridge Chemical Co.) was dissolved in a mechanically stirred solution of H₂O/HOAc (60 mL, 5:1). Iron powder was added over a 5 h period, maintaining the temperature below 40°C, and stirring was continued until TLC showed consumption of starting material. The reaction mixture was filtered through filter aid and the filter cake was washed with EtOAc. The aqueous filtrate was extracted with EtOAc and the combined organic fractions were washed with saturated NaHCO₃ solution, dried (MgSO₄) and concentrated. The residue was chromatographed (silica gel; CHCl₃/MeOH, 98:2) to afford an orange solid (2.34 g, 89%). MS (DCI/NH₃) m/e: 143 (M+H)⁺. NMR (DMSO-*d*_{*6*}, 300 MHz) δ 2.17 (s, 3H), 5.40 (br s, 2H), 6.90 (d, J=2.2 Hz, 1H), 7.54 (d, J=2.2 Hz, 1H).

### Step 56b. 6-Chloro-5-methyl-3-(2-(S)-azetidinylmethoxy)pyridine benzoate

Starting with the product of Example 56a in place of 3-amino-6-fluoro-5-methylpyridine, the procedures of Examples 55c-55f are followed to prepare the title compound.

### Example 57

### 5-Bromo-6-fluoro-3-(2-(S)-azetidinylmethoxy)pyridine hydrochloride

### Step 57a. 3-Bromo-2-(4-nitrophenylazo)-5-hydroxypyridine

5-Bromo-3-pyridinol from Example 44a (8.7 g, 0.05 mmol) and KOH (1.1 g, 19.6 mmol) were dissolved in water (200 mL). A suspension of p-nitrobenzenediazonium tetrafluoroborate (11.84 g, 0.5 mol, prepared as described in *J. Org. Chem.,* 44:1572-15783 (**1979**)) was added. The reaction was stirred for 1 hour, diluted with acetic acid (50 mL) and filtered. The crude product was allowed to air dry, then it was chromatographed (silica gel, eluting with chloroform/methanol 95:5-90:10) to provide the title compound (5.45 g, 33.7 % yield). MS (DCI/NH₃) m/z 323, 325 (M+H)⁺. NMR (DMSO-*d*_{*6*}, 300 MHz) δ 8.48-8.43 (m, 2H), 8.21 (d, J=2.4 Hz, 1H), 8.09-8.06 (m, 2H), 7.72 (d, J=2.4 Hz, 1H).

### Step 57b. 2-Amino-3-bromo-5-hydroxypyridine

The compound from step 57a above (5.0 g, 15.8 mmol) and tin chloride (25 g, 111 mmol) were suspended in conc. HCl and ethanol (150 mL), and the mixture was heated at reflux for 1 hour. The mixture was cooled to 0°C, then filtered. The filtrate was neutralized with sodium bicarbonate (180 g) and extracted with ethyl acetate (4 x 200 mL). The extracts were washed with brine, dried (MgSO₄) and concentrated. The residue was chromatographed (silica gel, eluting with chloroform/methanol/NH₄OH 95:5:0.5-90:10:1) to afford the title compound (3.3 g, 33.7 % yield). MS (DCI/NH₃) m/z 189, 191 (M+H)⁺. NMR (DMSO-*d*_{*6*}, 300 MHz) δ 7.57 (d, J=2.6 Hz, 1H), 7.43 (d, J=2.6 Hz, 1H).

### Step 57c. 3-Bromo-2-fluoro-5-hydroxypyridine

The compound from step 57b (3.0 g, 15.9 mmol) was dissolved in HF•pyridine (50 mL). The solution was cooled to 0°C and stirred under nitrogen, then sodium nitrite (1.09 g, 15.8 mmol) was added in portions over 20 minutes. The mixture was heated to 50°C for 1 hour, cooled to 0°C and basified with 20% NaOH. The aqueous phase was washed with methylene chloride (5 x 100 mL), neutralized with HCl, and extracted with ethyl acetate (5 x 100 mL). The extracts were dried (MgSO₄), filtered and concentrated *in vacuo,* yielding the title compound as a tan solid. MS (DCI/NH₃) m/z 192, 194 (M+H)⁺. NMR (DMSO-*d*_{*6*}, 300 MHz) δ 9.38 (d, J=2.6 Hz, 1H), 9.20 (d, J=2.6 Hz, 1H).

### Step 57d. 5-Bromo-6-fluoro-3-(2-(S)-azetidinylmethoxy)pyridine hydrochloride

Following the procedures of Example 2a-b, except substituting the product from step 57c for the 5-bromo-6-chloropyridinol-3-ol thereof, the title compound is prepared.

### Example 58

### 5-methoxy-6-chloro-3-(2-(S)-azetidinylmethoxy)pyridine hydrochloride

### Step 58a. 3-bromo-5-methoxypyridine

To a suspension of 12 g of 3,5-dibromopyridine and 40 g of 60% NaH in DMF was added 4.05 mL of methanol, and the reaction mixture was stirred for 4 hours at room temperature and 1 hour at 60°C. The DMF was removed under reduced pressure, and the product was isolated following extractive work-up. MS (DCI/NH₃) m/z 188/190 (M+H)⁺, 205/207 (M+NH₄)⁺. ¹H NMR (CDCl₃, 300 MHz) δ 8.32 (d, J=1.8 Hz, 1H), 8.27 (d, J=2.6 Hz, 1H), 7.42 (dd, J=1.8, 2.6 Hz, 1H), 3.88 (s, 3H)

### Step 58b. 3-amino-5-methoxypyridine

Treating the compound of step 58a according to the procedure of Example 41b, the title compound is prepared.

### Step 58c. 3-hydroxy-5-methoxypyridine

Treating the compound of step 58b according to the procedures of Examples 55b and 55c, the title compound is prepared.

### Step 58d. 2-amino-5-hydroxy-3-methyoxypyridine

Treating the compound of step 58c according to the procedures of Examples 57a and 57b, the title compound is prepared.

### Step 58e. 2-chloro-5-hydroxy-3-methyoxypyridine

Treating the compound of step 58d with NaNO₂ and HCl according to standard procedures, the title compound is prepared

### Step 58f. 5-methoxy-6-chloro-3-(2-(S)-azetidinylmethoxy)pyridine hydrochloride

Following the procedures of Examples 2a-b, except substituting the product from step 58e for the 5-bromo-6-chloropyridin-3-ol thereof, the title compound is prepared.

### Example 59

### 5-ethoxy-6-chloro-3-(2-(S)-azetidinylmethoxy)pyridine hydrochloride

Following the procedures of Example 58, except substituting ethanol for the methanol reagent in Example 58a, the title compound is prepared.

### Example 60

### 5-nitro-6-methyl-3-(2-(S)-azetidinylmethoxy)pyridine hydrochloride

### Step 60a. 3-benzyloxy-5-bromo-6-methylpyridine

The hydroxy compound from Ex 44a is treated with 1 equivalent of sodium hydride in THF, followed by treatment with benzyl bromide to afford the title compound.

### Step 60b. 3-benzyloxy-5-amino-6-methylpyridine

Treating the compound of step 60a according to the procedure of Example 41b, the title compound is prepared.

### Step 60c. 3-amino-5-hydroxy-6-methylpyridine

Treating the compound of step 60b according to the procedure of Example 41c, the title compound is prepared.

### Step 60d. 3-hydroxy-5-nitro-6-methylpyridine

Treating the compound of step 60a according to the procedure of Example 41d, the title compound is prepared.

### Step 60e. 5-nitro-6-methyl-3-(2-(S)-azetidinylmethoxy)pyridine hydrochloride

Following the procedures of Examples 2a-b, except substituting the product from step 60d for the 5-bromo-6-chloropyridin-3-ol thereof, the title compound is prepared.

### Example 61

### 5,6-dimethyl-3-(2-(S)-azetidinylmethoxy)pyridine hydrochloride

### Step 61a. 2,3-dimethyl-5-nitropyridine

2-Chloro-3-methyl-5-nitropyridine (from Maybridge Chemical Co.) is treated according to the procedure of Ex. 52a to afford the title compound.

### Step 61b. 5,6-dimethyl-3-(2-(S)-azetidinylmethoxy)pyridine hydrochloride

Following the procedures of Examples 2a-b, except substituting the product from step 61a for the 5-bromo-6-chloropytidin-3-ol thereof, the title compound is prepared.

### Examples 66-72

Following the procedures of Example 2a-b, except substituting the 3-hydroxy-5-nitro-6-methylpyridine prepared in Example 60d for the 5-bromo-6-chloropyridin-3-ol in step 2a, and substituting the starting material indicated in the Table 3 below for the 1-BOC-2-(S)-azetidinemethanol in step 2a the compounds of examples 66-68 are prepared. Following the procedures of Examples 2 and 3, except substituting the 3-hydroxy-5-nitro-6-methylpyridine prepared in Example 60d for the 5-bromo-6-chloropyridin-3-ol in step 2a, and substituting the starting material indicated in the table below for the 1-BOC-2-(S)-azetidinemethanol in step 2a, the HCl salts of the compounds of examples 69-72 are prepared.

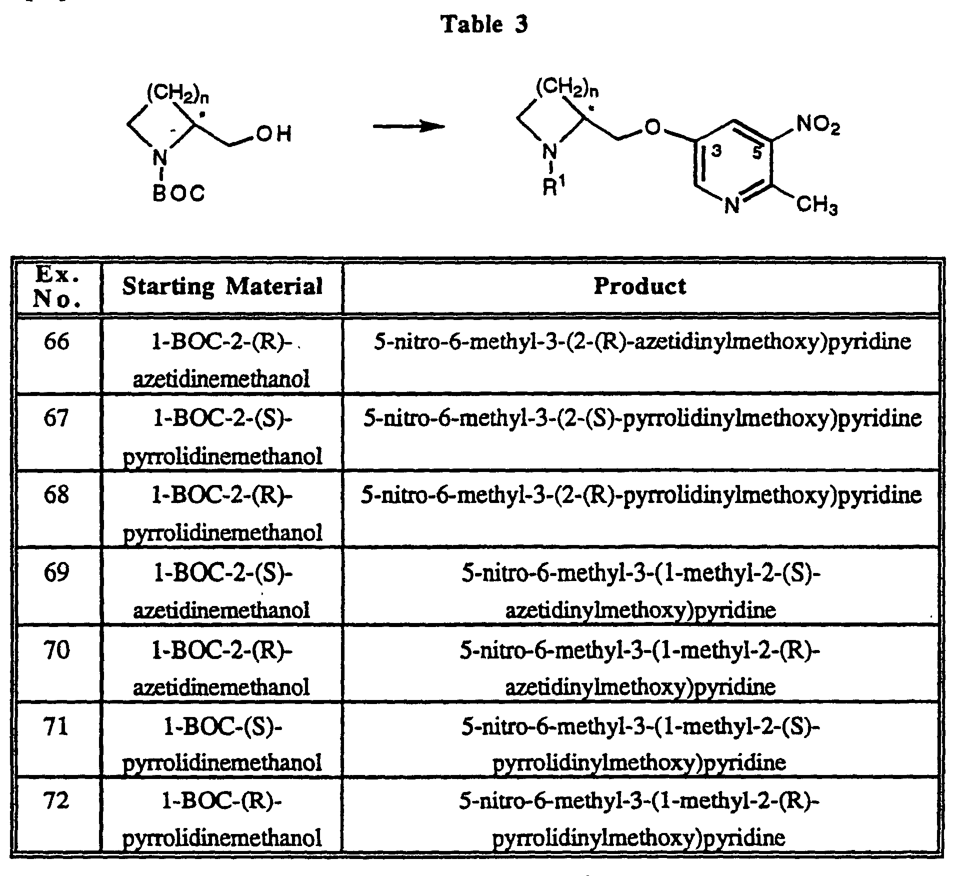

### Examples 80-87

In the following Examples, the starting materials (from Examples 60, and 66-72, as appropriate) are stirred under an atmosphere of hydrogen in the presence of 10% palladium on carbon and the progress of the reaction is monitored by TLC. When consumption of starting material is complete, the mixture is filtered, and the filtrate is concentrated. The residue is subjected to BOC deprotection under acidic conditions and isolation and salt formation as in Example 2b. Products with R¹ = methyl are obtained by treating the starting material under the conditions of Example 2b and 3 prior to conducting the above-described hydrogenation step.

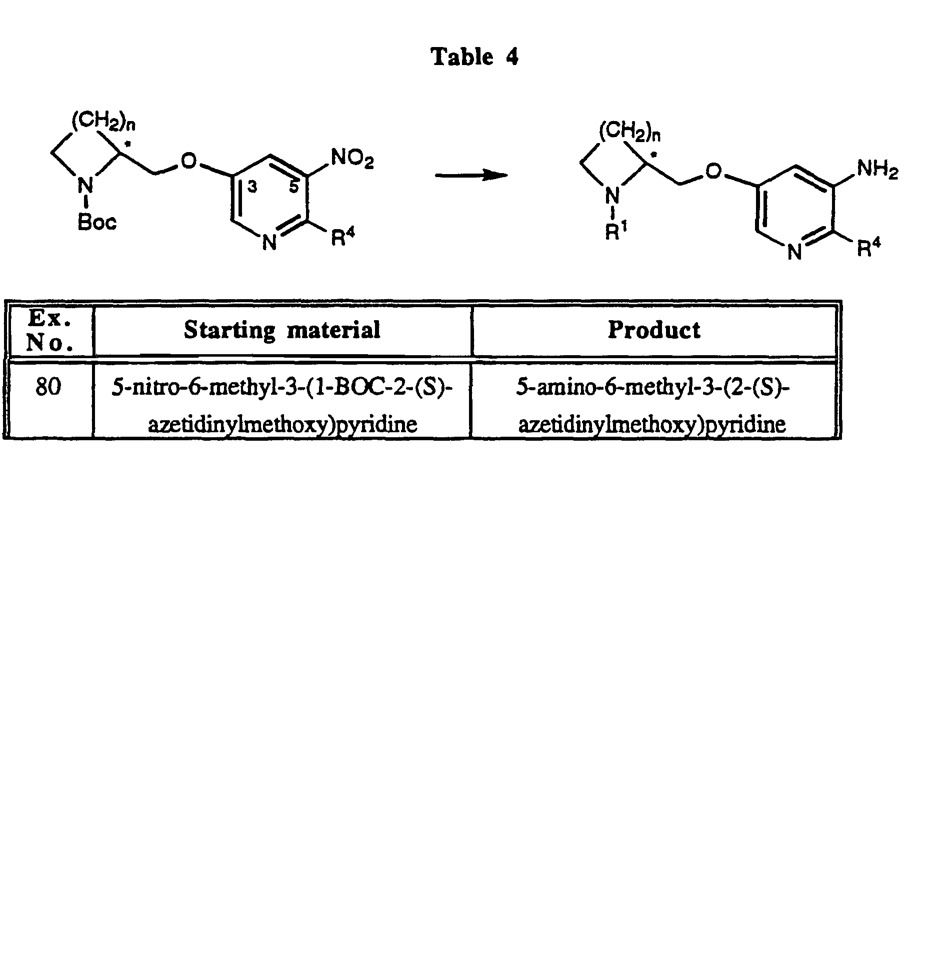

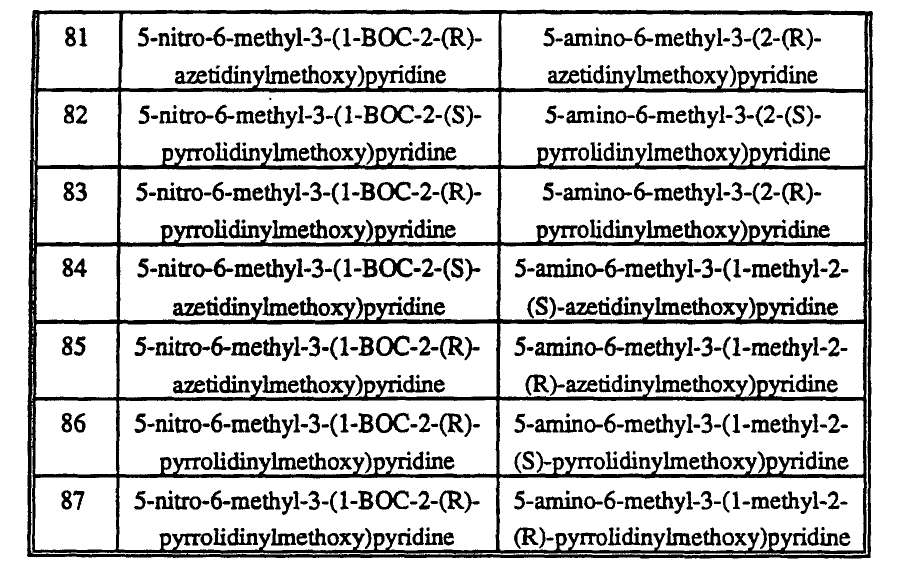

### Examples 88-94

Following the procedures of Example 52b-f, except substituting the starting material indicated below for the 1-BOC-2-(S)-azetidinemethanol in step 52b, and omitting the methylation step, the compounds of examples 88-91 are prepared. Following the procedures of Example 52b-f, except substituting the starting material indicated in the below for the 1-BOC-2-(S)-azetidinemethanol in step 52b the compounds of examples 92-94 are prepared.

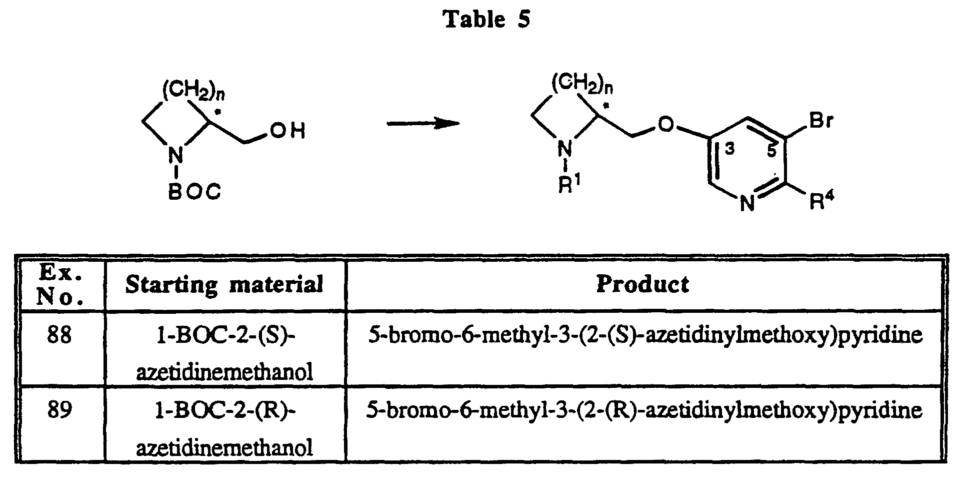

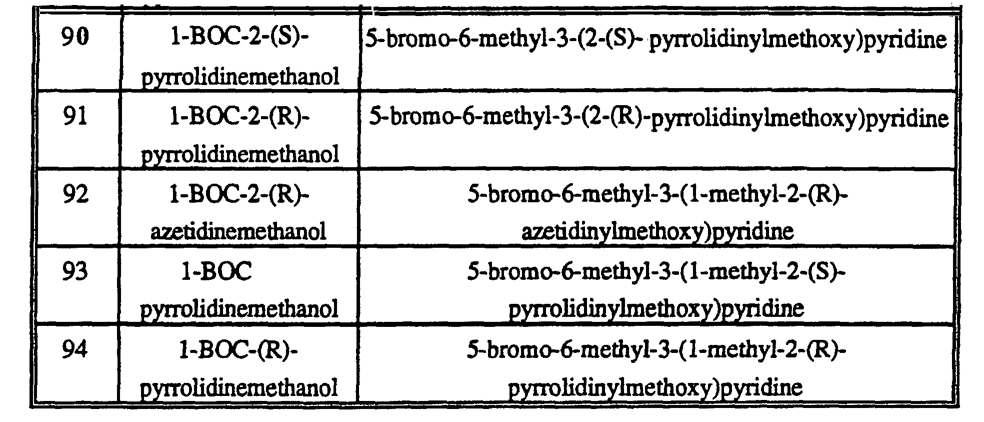

### Examples 95-101

Following the procedure of Example 57d, except substituting the starting material indicated in the table below for the 1-BOC-2-(S)-azetidinemethanol in step 57d, the compounds of examples 95-97 are prepared. Following the procedures of Example 57d, except substituting the starting material indicated in the table below for the 1-BOC-2-(S)-azetidinemethanol in step 57d, then reacting the product thereof with paraformaldehyde and sodium cyanoborohydride according to the procedure of Example 3, the HCl salts of the compounds of examples 98-101 are prepared.

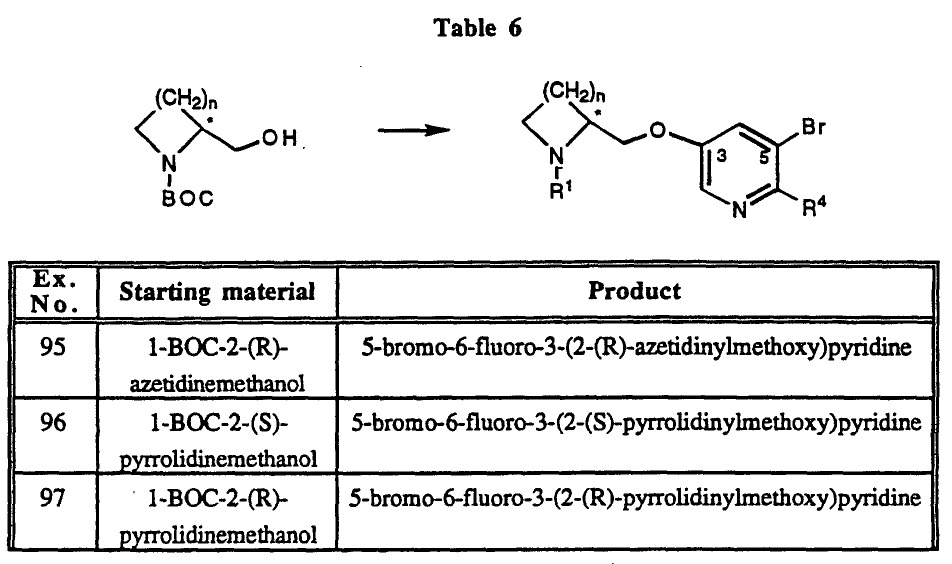

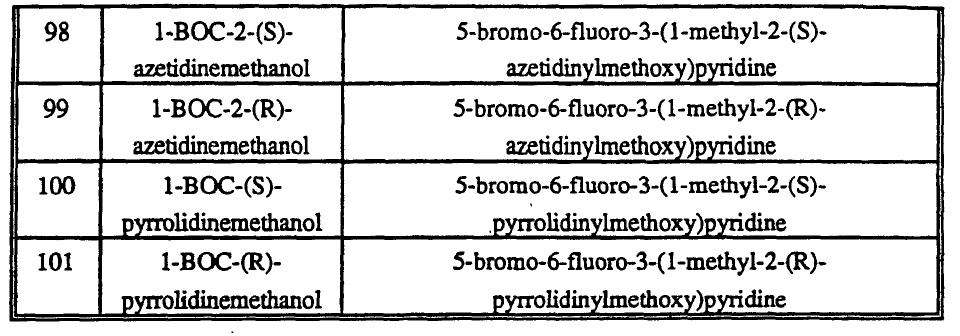

### Example 102

### 5-Bromo-6-chloro-3-(2-(R)-azetidinylmethoxy)pyridine hydrochloride

The title compound is prepared following the procedures of Example 2a-b, with the exception of substituting 1-BOC-2-(R)-azetidinemethanol for the 1-BOC-2-(S)-azetidinemethanol.

### Example 103

### 5-Bromo-6-chloro-3-(1-methyl-2-(R)-azetidinylmethoxy)pyridine hydrochloride

The title compound is prepared following the procedures of Example 3, with the exception of substituting 5-bromo-6-chloro-3-(1-BOC-2-(R)-azetidinylmethoxy)pyridine for the 5-bromo-6-chloro-3-(1-BOC-2-(S)-azetidinylmethoxy)pyridine.

### Examples 115-136

Following the procedures of Examples 49a-b and substituting the starting material of the table below for the 5-bromo-6-chloro-3-(1-BOC-2-(S)-azetidinylmethoxy)pyridine of step 49a, the compounds of examples 115-118, 123-126, and 131-133 are prepared. Following the procedures of Examples 49 and 50 and substituting the starting material of the table below for the 5-bromo-6-chloro-3-(1-BOC-2-(S)-azetidinylmethoxy)pyridine of step 49a, the HCl salts of the compounds of examples 119-122, 127-130, and 134-136 are prepared.

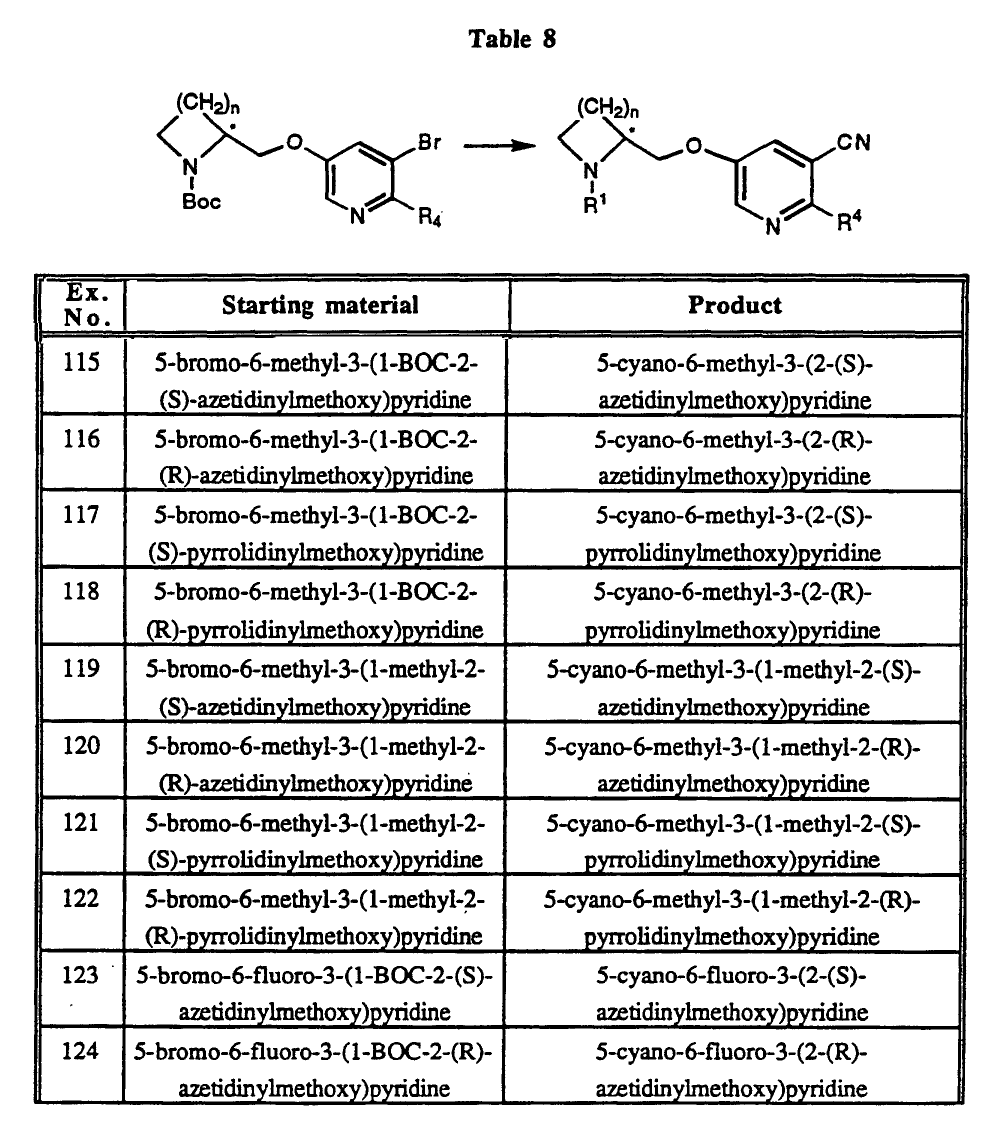

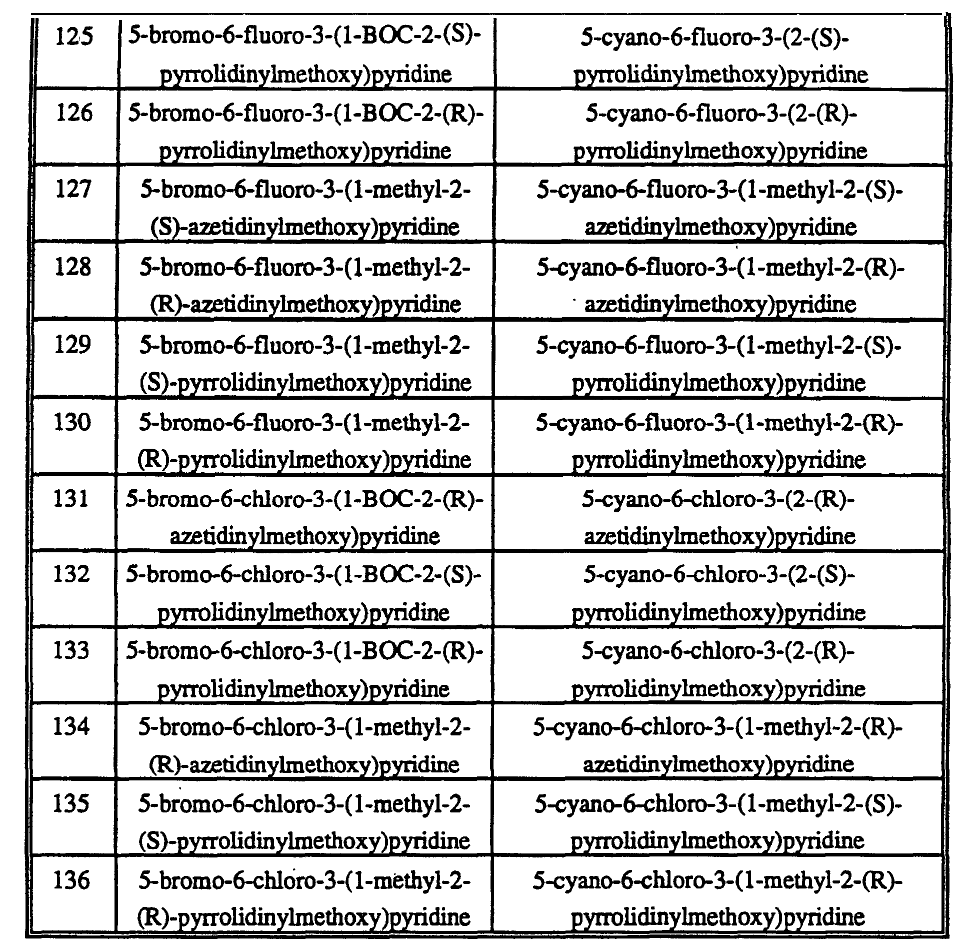

### Examples 145-167

Following the procedures of Examples 49a-b and substituting the starting material of the table below for the 5-cyano-6-chloro-3-(1-BOC-2-(S)-azetidinylmethoxy)pyridine of step 49a, the compounds of examples 145-148, 153-156, and 161-164 are prepared. Following the procedures of Examples 49 and 50 and substituting the starting material of the table below for the 5-cyano-6-chloro-3-(1-BOC-2-(S)-azetidinylmethoxy)pyridine of step 49a, the HCl salts of the compounds of examples 149-152, 157-160, and 165-167 are prepared.

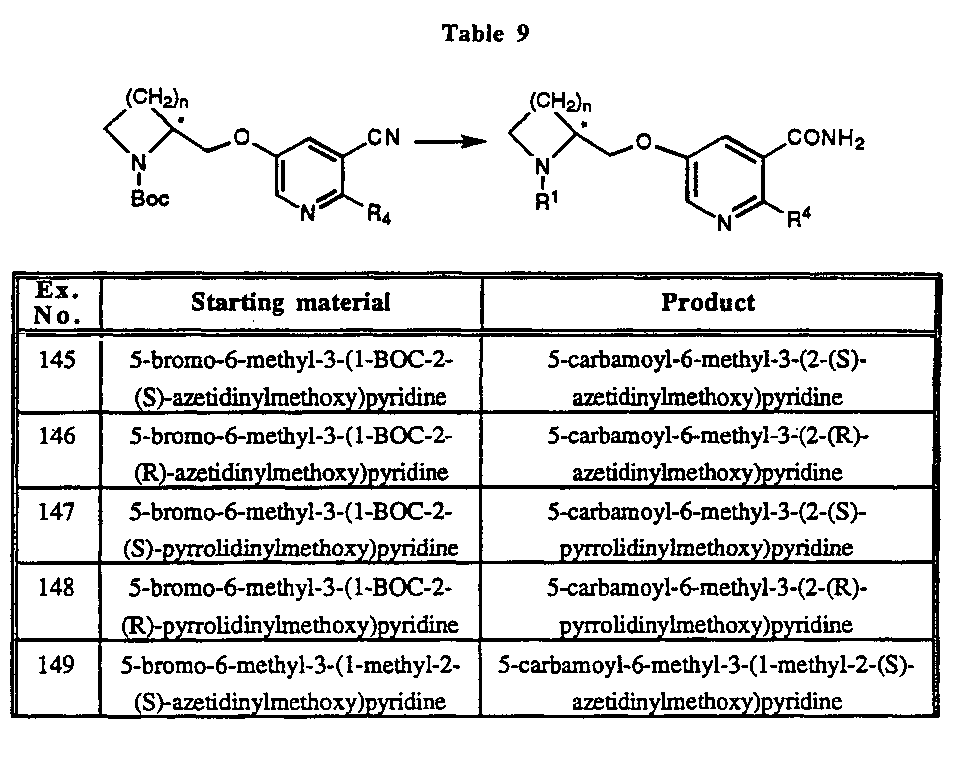

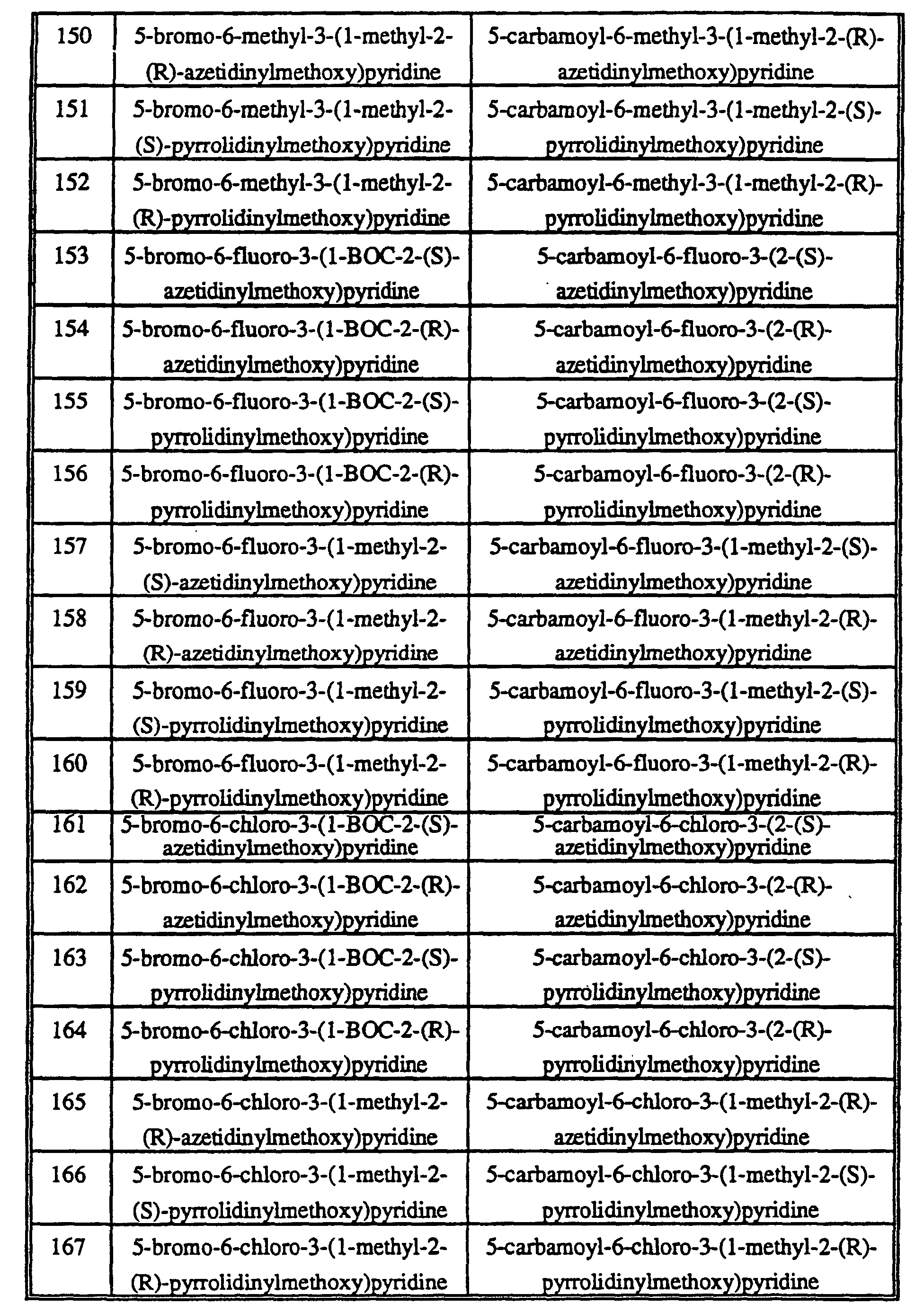

### Examples 168-183

The starting material from Example 168-183above are treated with bromine in aqueous alkali under conditions of the Hofmann rearrangement (*cf*., Allen and Wolf, *Org. Syn.,* 30: 3 (**1950**)) to replace the carbamoyl group with the amino group.

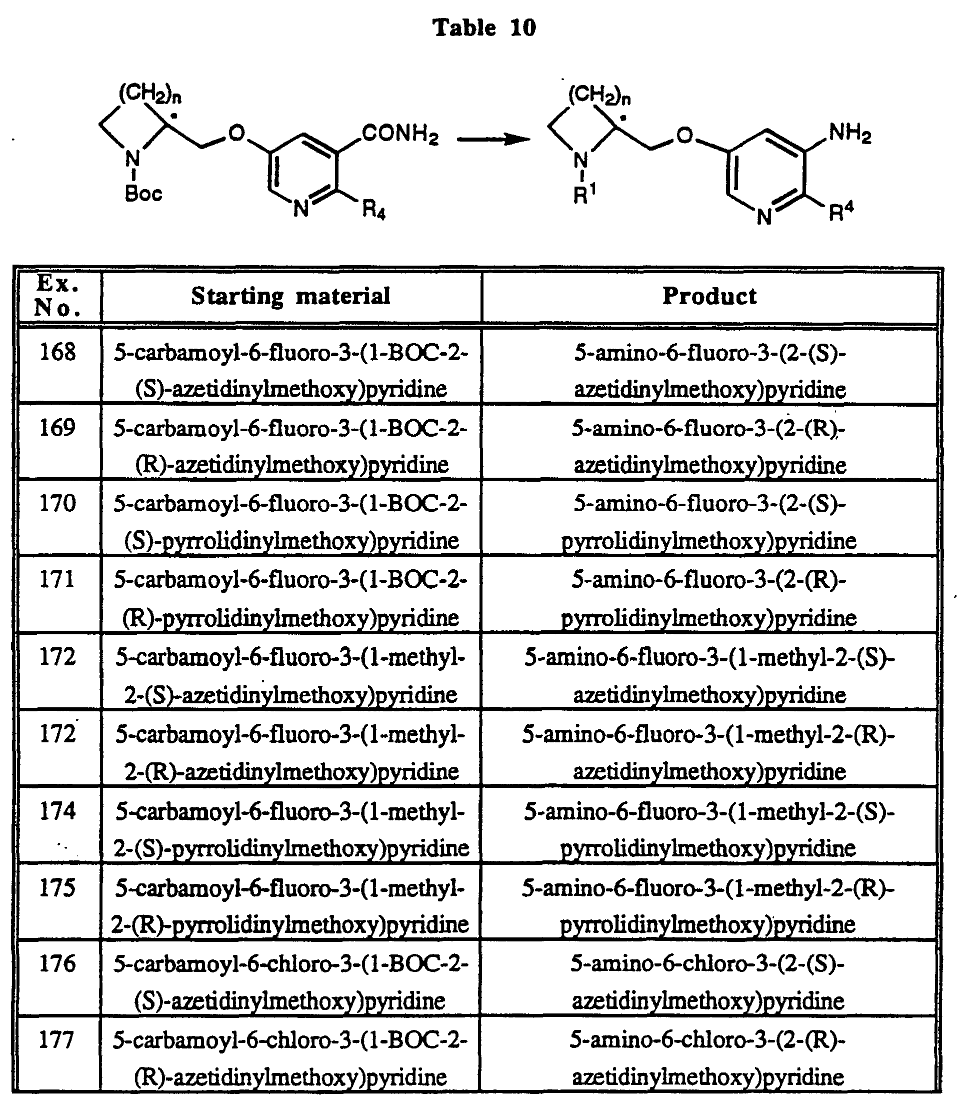

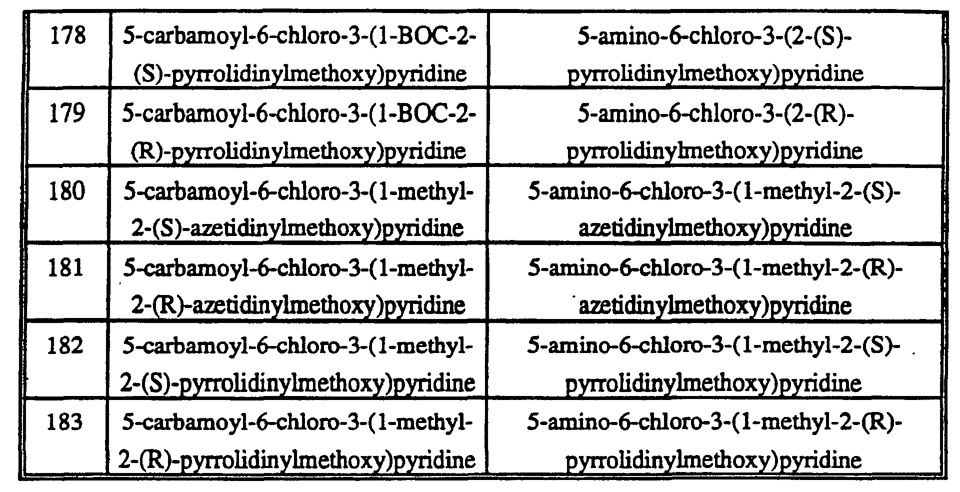

### Examples 192-215

The starting material compounds shown in the table below (some of which are BOC intermediates of Examples 168-183) are treated with 2,4-dinitrophenyl formate (for Examples 192-199) or acetic anhydride (for Examples 200-215). The product is subjected to BOC deprotection, isolation and salt formation as in Example 2 and 3.

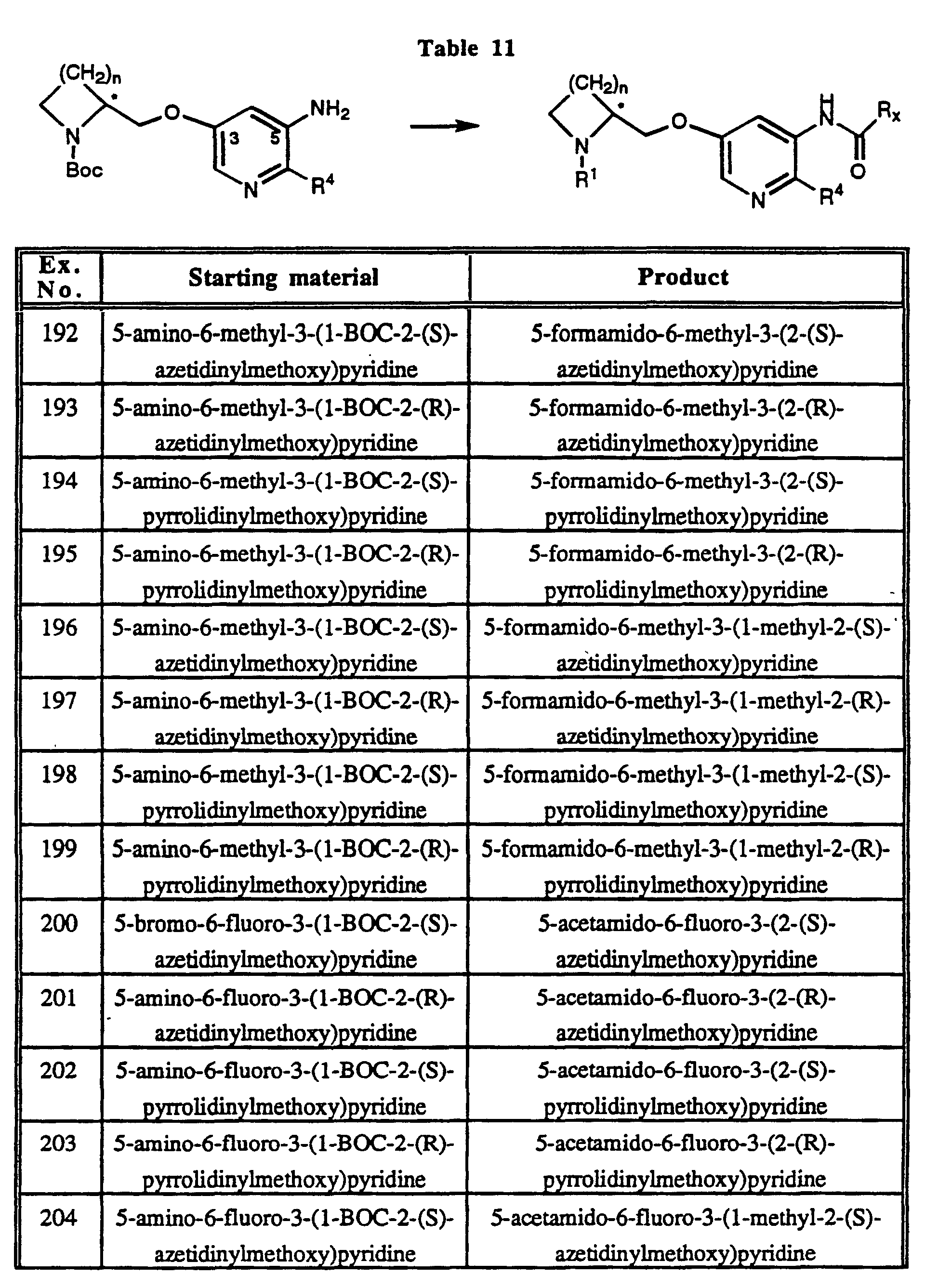

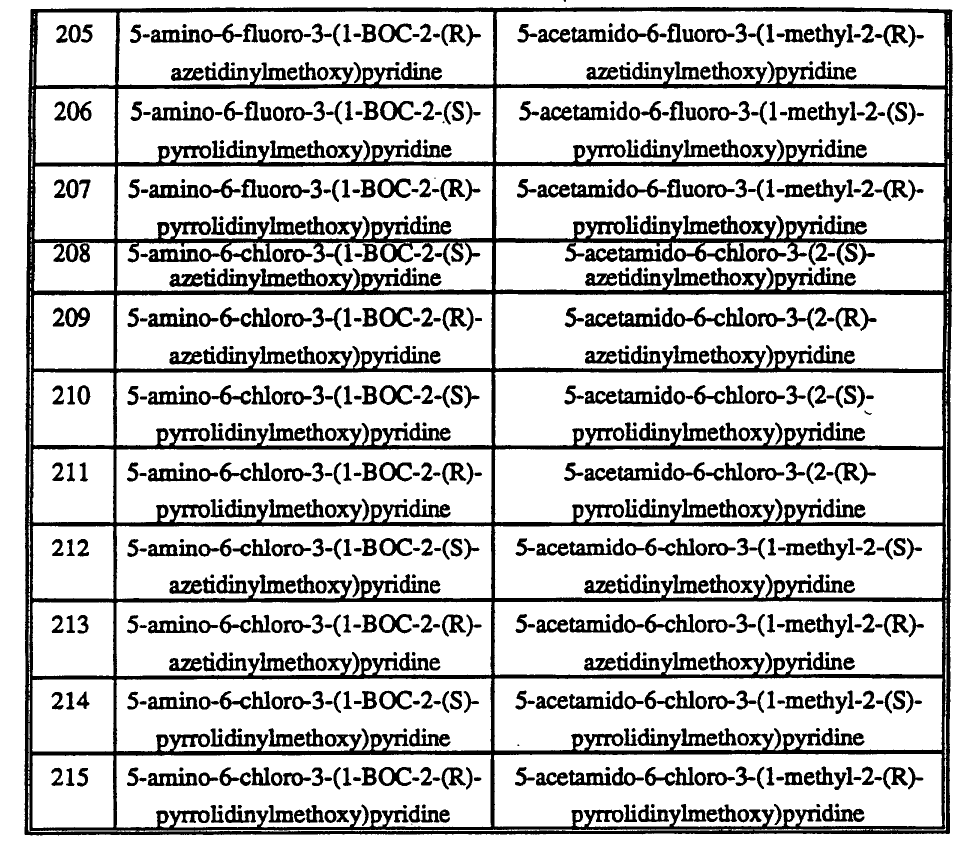

### Examples 224-247

The starting material compounds shown in the table below ( some of which are BOC-intermediates from Examples 192-215 above) are treated with borane as in Ex 1d followed by deprotection and salt formation as in Example 2b to give the product compounds shown. For products with R¹= methyl, BOC-deprotection as in Example 2b and N-methylation precede the treatment with borane.

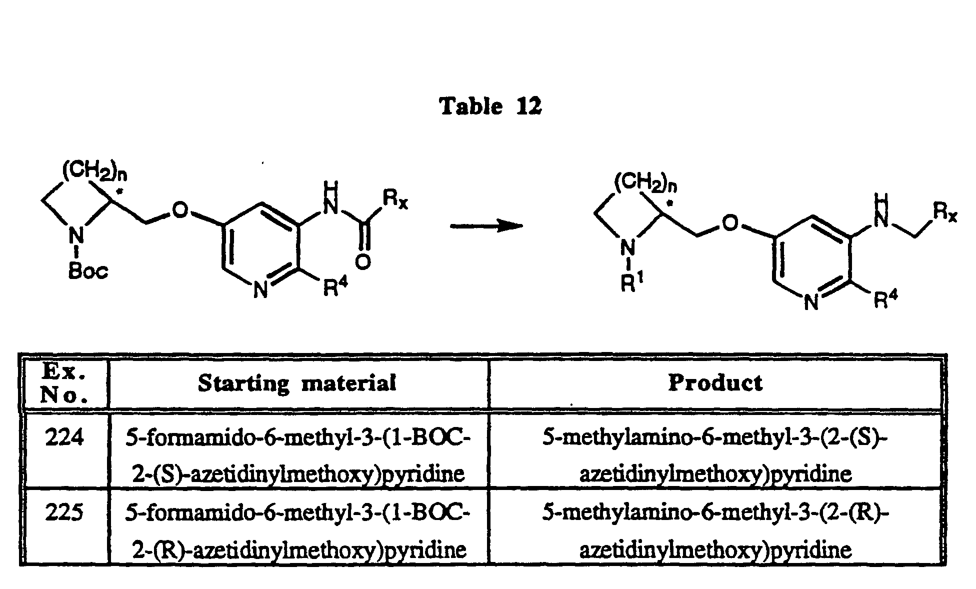

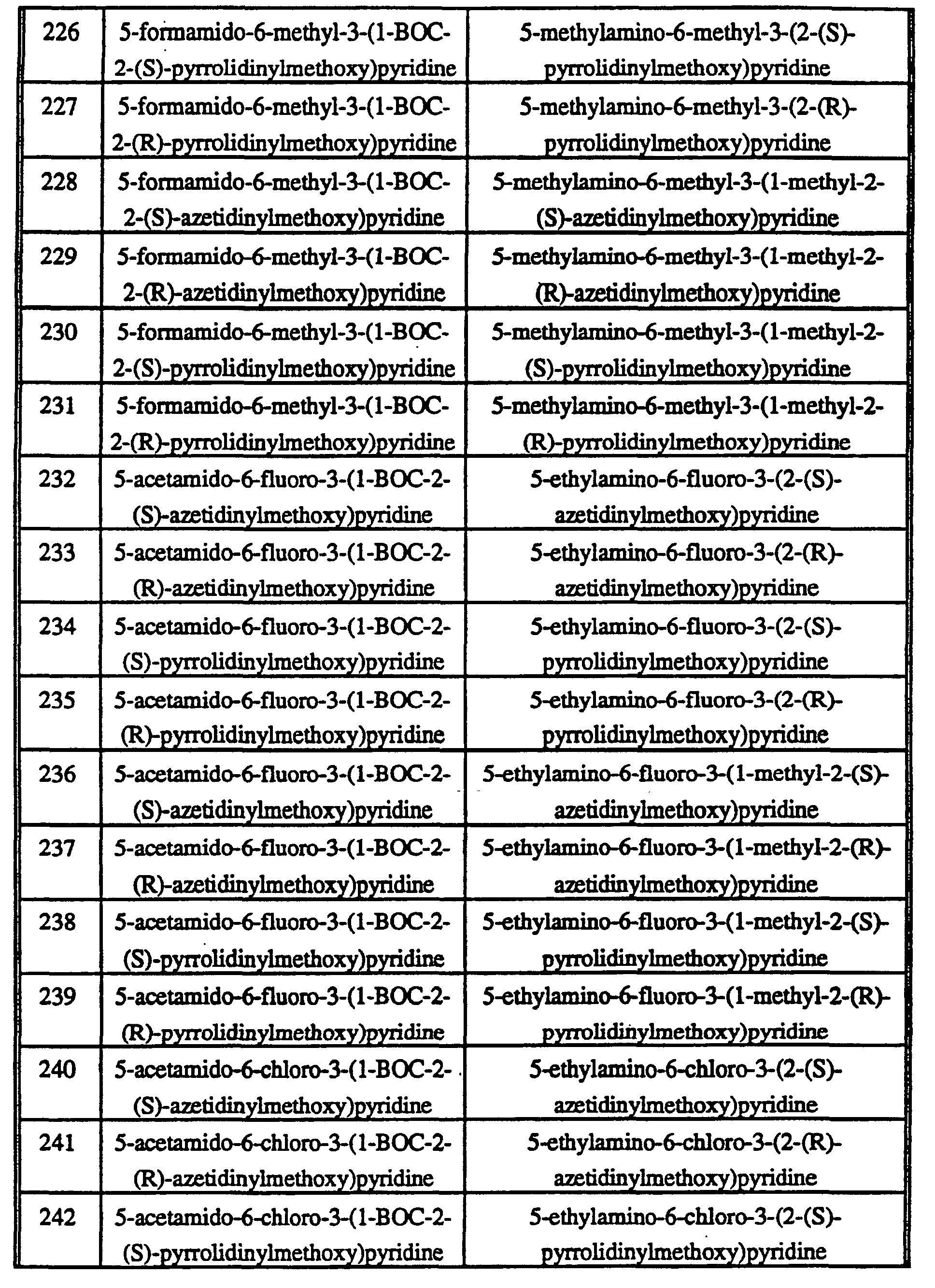

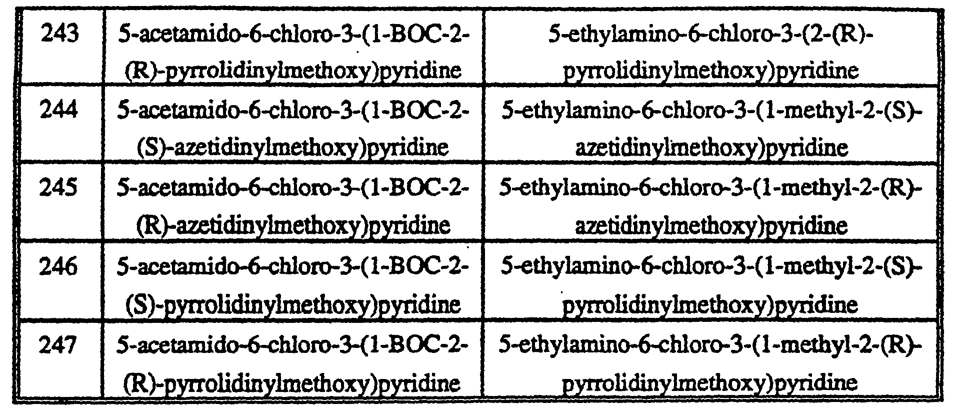

### Examples 248-271

The starting material compounds shown in the table below (some of which are BOC-protected intermediates of Examples 88-94 above) are treated as in Example 45 (for examples 248-263 wherein R⁴ = ethyl). In Examples 264-271, wherein R⁴ = propyl, the intermediates are treated as in Example 45 except substituting allyl tri-n-propyltin for the vinyl tri-n-butyltin therein.

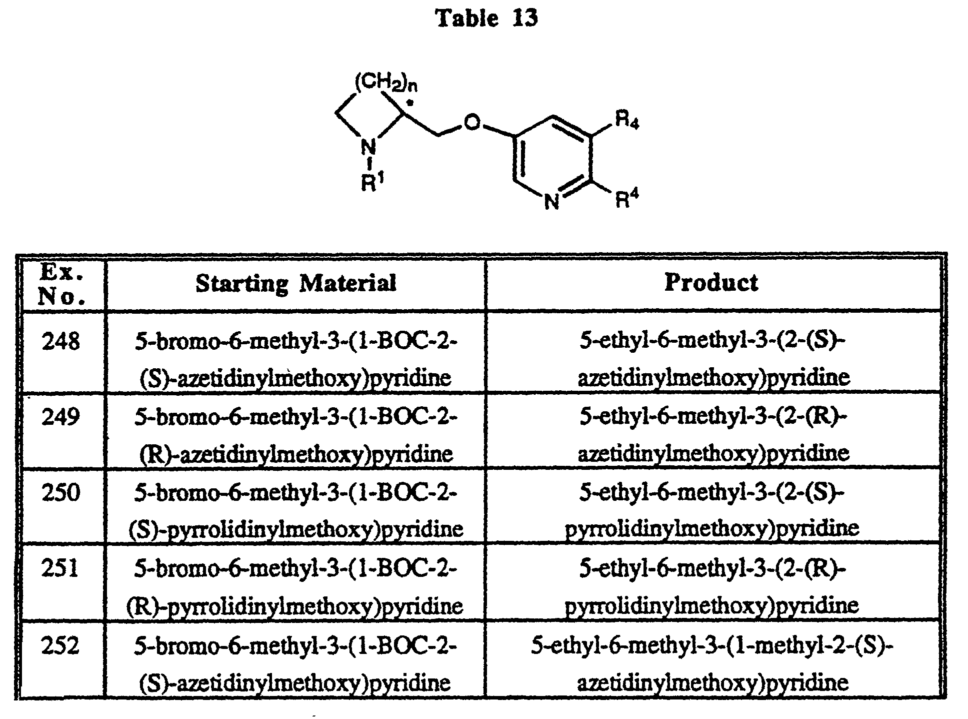

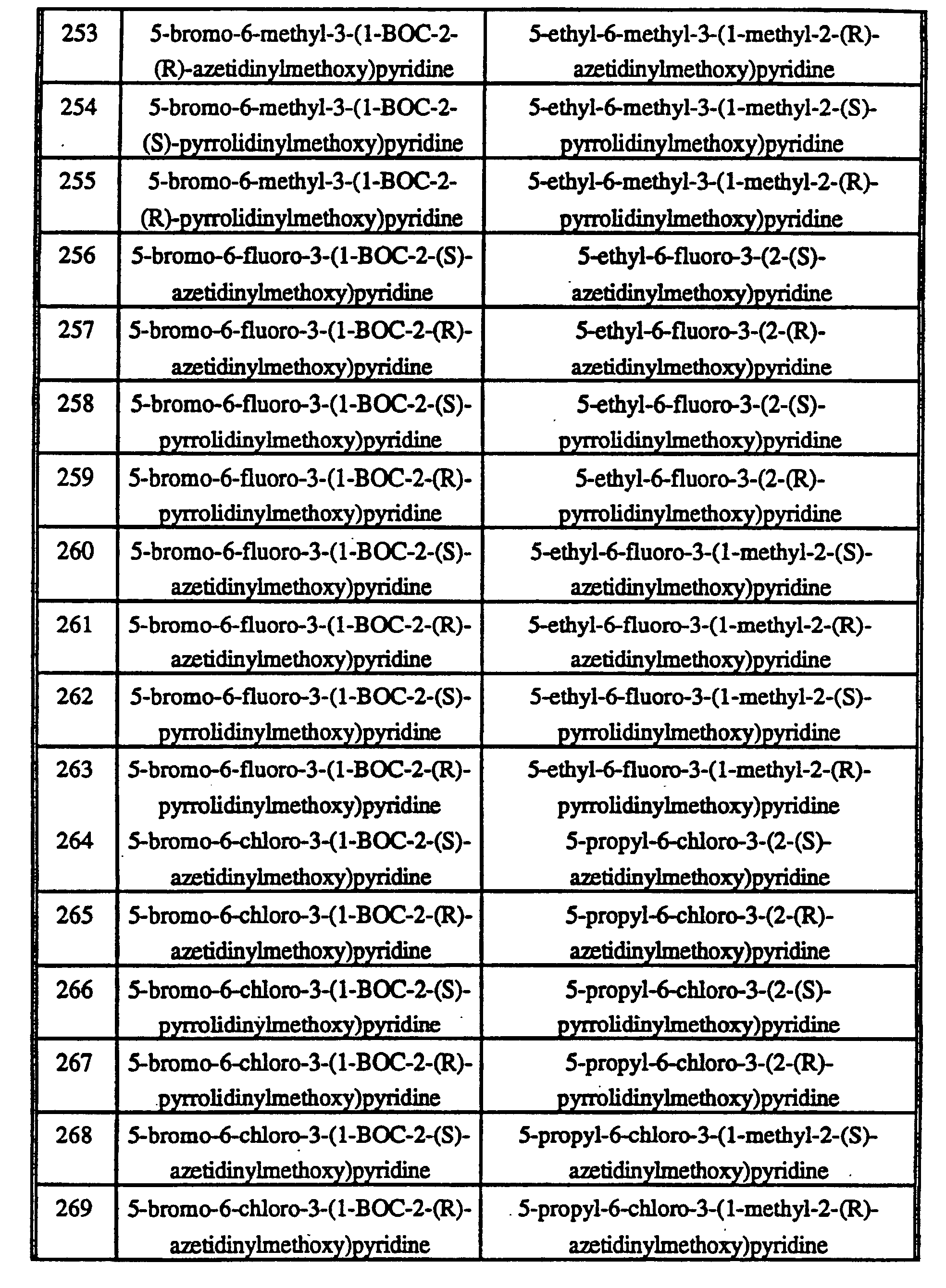

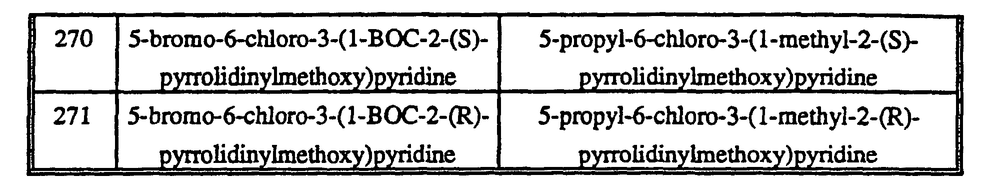

### Examples 272-278

Following the procedures of Example 55c-f, substituting the starting material shown in the table below for the 1-BOC-2-(S)-azetidinemethanol thereof, the product compounds of Examples 272-274 are prepared. Following the procedures of Example 55c-d, substituting the starting material shown in the table below for the 1-BOC-2-(S)-azetidinemethanol thereof, then reacting the product thereof with paraformaldehyde and sodium cyanoborohydride according to the procedure of Example 3, the product compounds of Examples 275-278 are prepared.

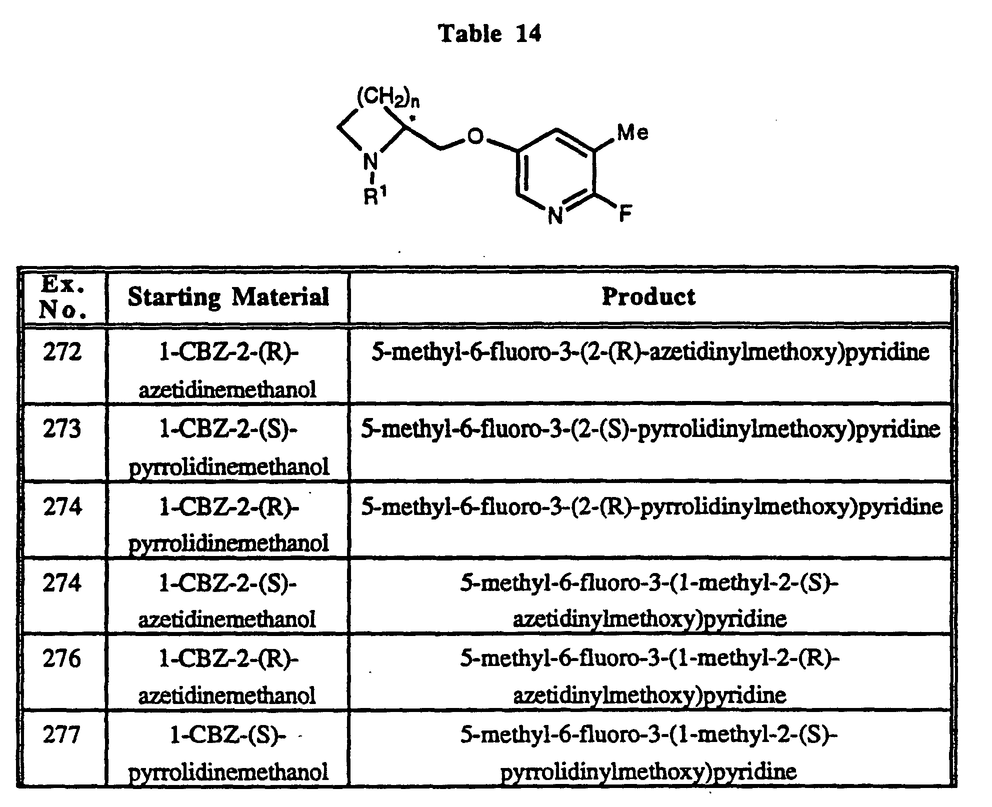

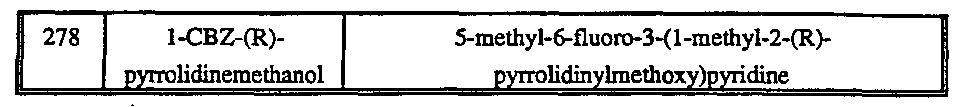

### Examples 279-285

Following the procedures of Example 56a-b, substituting the starting material shown in the table below for the 1-BOC-2-(S)-azetidinemethanol thereof, the product compounds of Examples 279-281 are prepared. Following the procedures of Example 56c-d, substituting the starting material shown in the table below for the 1-BOC-2-(S)-azetidinemethanol thereof, then reacting the product thereof with paraformaldehyde and sodium cyanoborohydride according to the procedure of Example 3, the product compounds of Examples 272-285 are prepared.

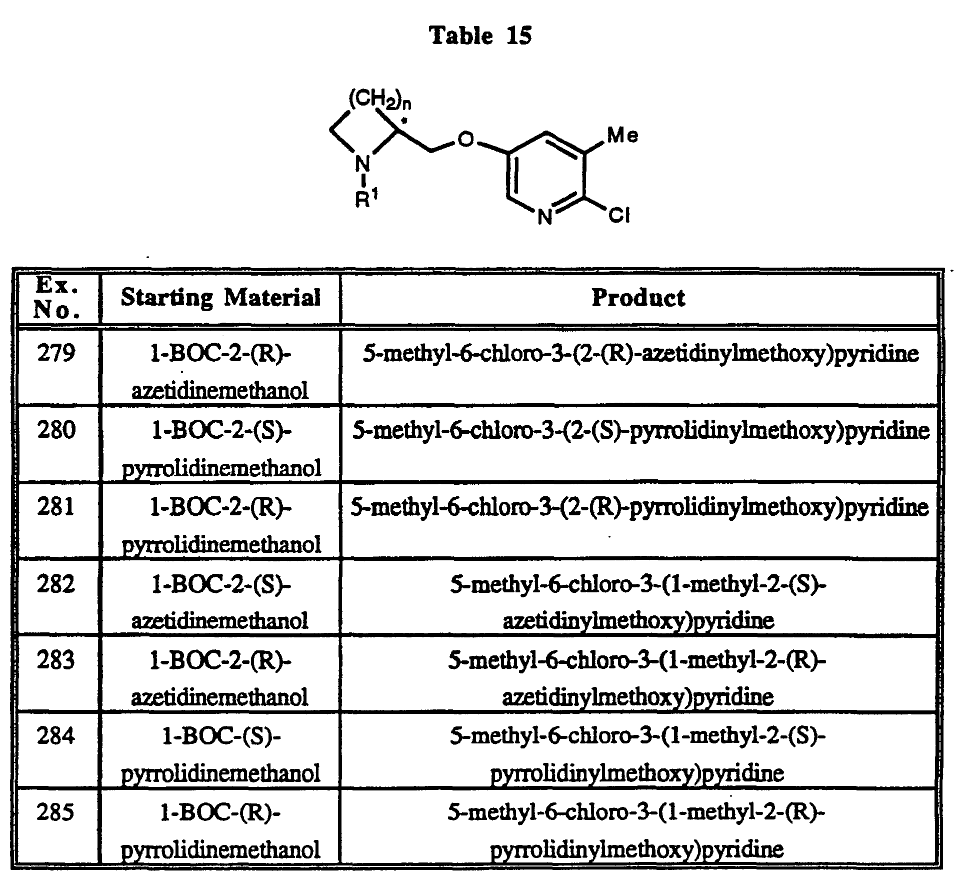

### Examples 286-292

Following the procedures of Example 61a-b, substituting the starting material shown in the table below for the 1-BOC-2-(S)-azetidinemethanol thereof, the product compounds of Examples 286-288 are prepared. Following the procedures of Example 61a-b, substituting the starting material shown in the table below for the 1-BOC-2-(S)-azetidinemethanol thereof, then reacting the product thereof with paraformaldehyde and sodium cyanoborohydride according to the procedure of Example 3, the product compounds of Examples 289-292 are prepared.

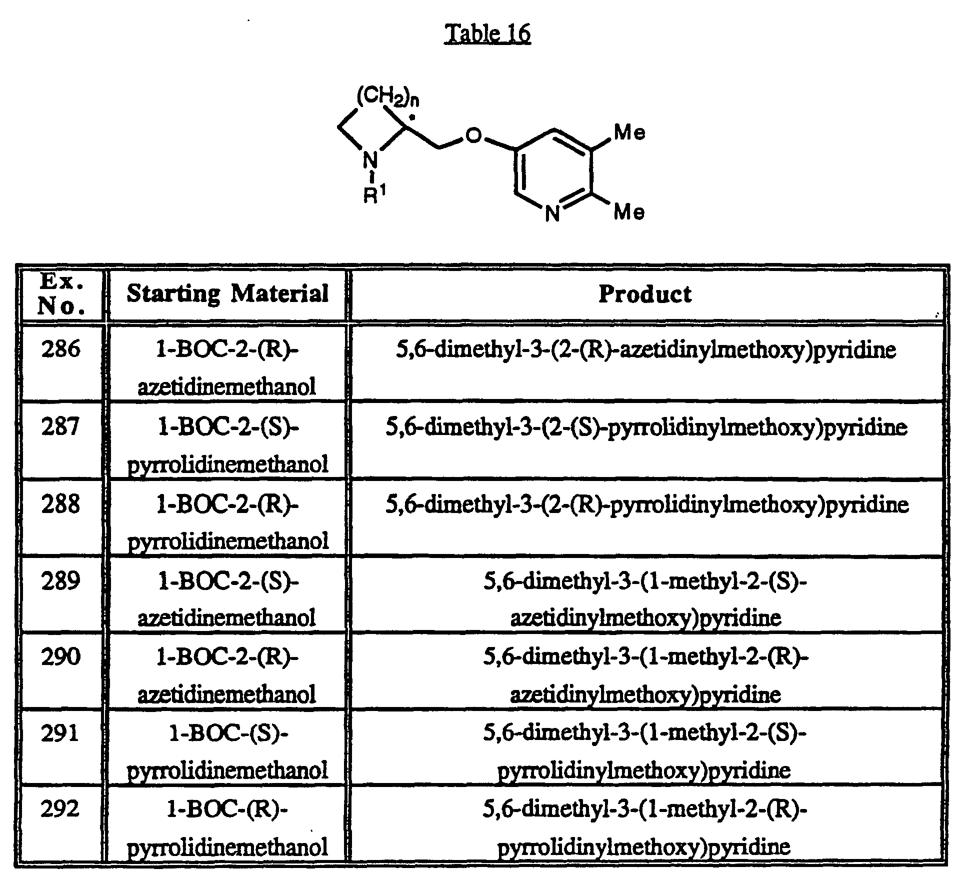

### Examples 293-300

Following the procedures of Example 58, substituting the starting material shown in the table below for the 1-BOC-2-(S)-azetidinemethanol thereof and replacing step 58e with the procedure and reagents of Example 57c, the product compounds of Examples 293-296 are prepared. Following the procedures of Example 58, substituting the starting material shown in the table below for the 1-BOC-2-(S)-azetidinemethanol thereof and replacing step 58e with the procedure and reagents of Example 57c, then reacting the product thereof with paraformaldehyde and sodium cyanoborohydride according to the procedure of Example 3, the product compounds of Examples 297-292 are prepared.

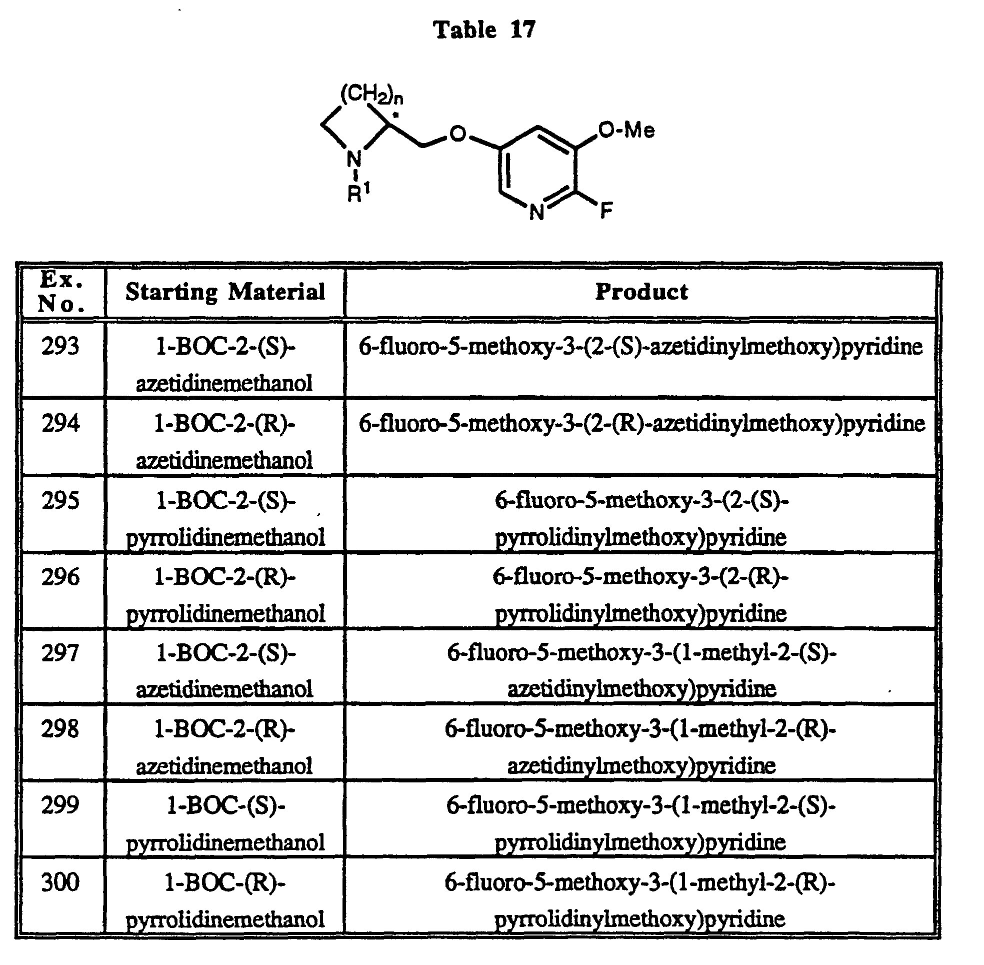

### Examples 301-308

Following the procedures of Example 58, substituting ethanol for the methanol reagent in Example 58a, substituting the starting material shown in the table below for the 1-BOC-2-(S)-azetidinemethanol in step 58a and replacing step 58e with the procedure and reagents of Example 57c, the product compounds of Examples 301-304 are prepared. Following the procedures of Example 58, substituting the starting material shown in the table below for the 1-BOC-2-(S)-azetidinemethanol thereof and replacing step 58e with the procedure and reagents of Example 57c, then reacting the product thereof with paraformaldehyde and sodium cyanoborohydride according to the procedure of Example 3, the product compounds of Examples 305-308 are prepared.

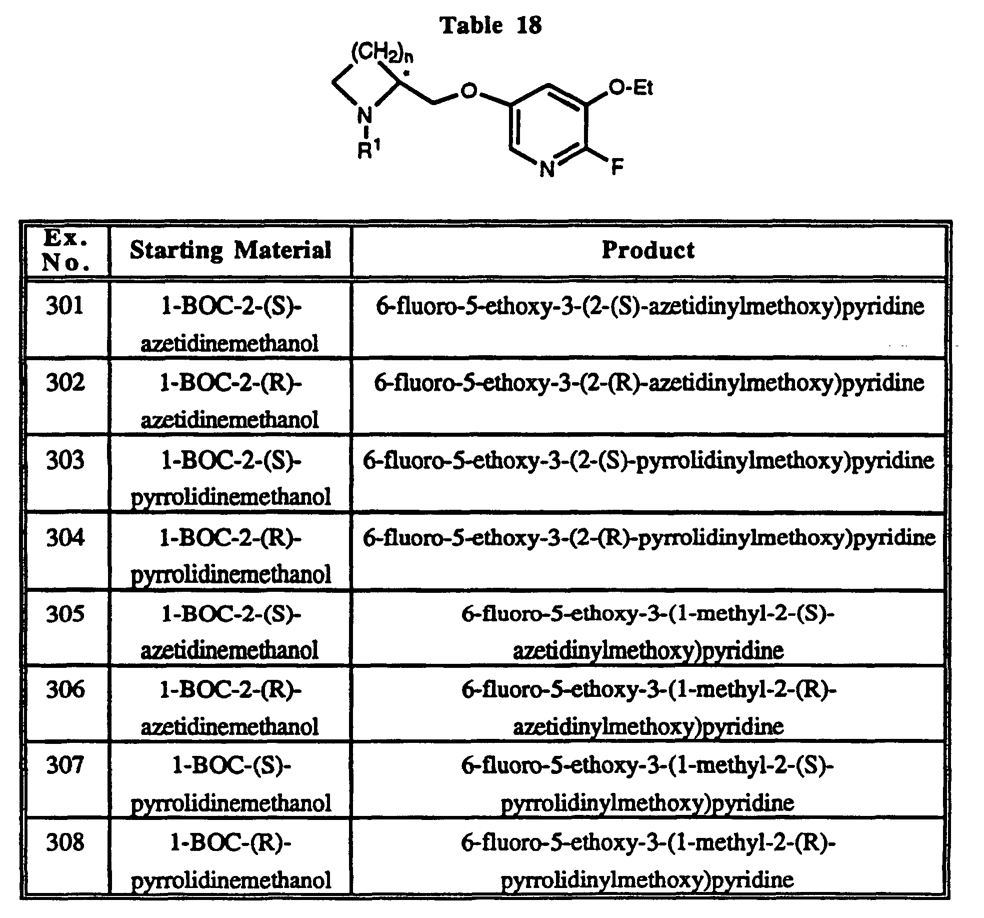

## Claims

1. A compound or pharmaceutically acceptable salt or ester thereof wherein said compound has the structure: 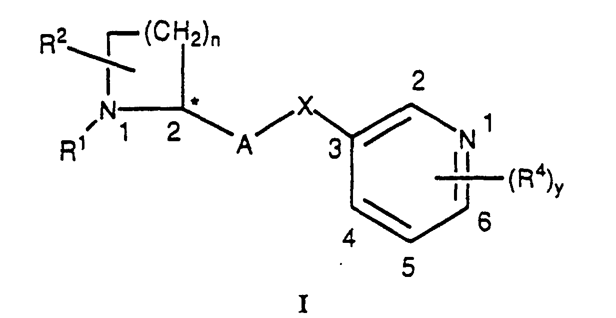 wherein the asterisk denotes a chiral center;
**n** is an integer selected from 1, 2, or 3;
**X** is oxygen or sulfur;
**R**^{**1**} is selected from the group consisting of hydrogen, allyl, and C₁-C₆-alkyl;
**R**^{**2**} is hydrogen or, when n=2, is also a single substituent selected from the group consisting of
CH₂OH,
CH₂F,
CH₂CN,
CH₂OCH₃,
Br,
Cl,
F,
OH,
CN,
C₁-C₃ alkoxyl,
OCOCH₃, and
O-methanesulfonyl,
with the proviso that when R² is substituted at the 3-position or the 5-position of the pyrrolidinyl ring, it is a C₁-C₃-alkyl group;
A is selected from the group consisting of 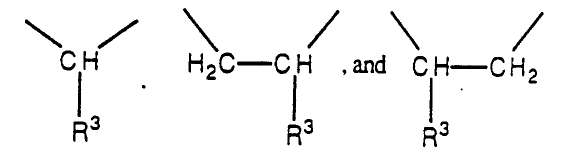 where R³ is H or C₁-C₆-alkyl;
y is 2; and
R⁴ is substituted at the 2,5-, 2,6- or 5,6- positions of the pyridine ring wherein the 2-position substituent is selected from the group consisting of
-Br,
-Cl,
-F,
-OH,
-(C₁-C₄ alkyl) and
-(C₁-C₃ alkoxy); and
the substituents at the 5- or 6-positions of the pyridine ring are selected from the group consisting of
-Br,
-Cl,
-F,
-OH,
-(C₁-C₄ alkyl),
-CN,
-CF₃,
-NO₂,
-CH₂OH,
-CH₂CN,
-(C₁-C₃ alkoxy),
-NH₂,
-NH-CHO,
-NHCO(C₁-C₃ alkyl),
-N(C₁-C₃ alkyl)CO(C₁-C3 alkyl),
-NH-(C₁-C₃ alkyl), N(C₁-C₃ alkyl)₂,
-COOH,
-COO(C₁-C₃ alkyl),
-CONH₂,
-CONH-(C₁-C₃ alkyl),
-CONHbenzyl, and
-OCO(C₁-C₃ alkyl).

2. A compound as defined by Claim 1 or a pharmaceutically acceptable salt or ester thereof wherein n=1.

3. A compound as defined by Claim 1 or a pharmaceutically acceptable salt or ester thereof wherein n=2.

4. A compound as defined by Claim 1 or a pharmaceutically acceptable salt or ester thereof wherein n=3.

5. A compound as defined by Claim 1 or a pharmaceutically acceptable salt or ester thereof wherein X is oxygen.

6. A compound as defined by Claim 1 or a pharmaceutically acceptable salt or ester thereof wherein X is sulfur.

7. A compound as defined by Claim 2 selected from the group consisting of
5-bromo-6-chloro-3-(2-(S)-azetidinylmethoxy)pyridine;
5-bromo-6-chloro-3-(1-methyl-2-(S)-azetidinylmethoxy)pyridine;
5,6-dichloro-3-(2-(S)-azetidinylmethoxy)pyridine;
5,6-dichloro-3-(1-methyl-2-(S)-azetidinylmethoxy)pyridine;
5,6-dichloro-3-(2-(R)-azetidinylmethoxy)pyridine;
5,6-dichloro-3-(1-methyl-2-(R)-azetidinylmethoxy)pyridine;
5-ethyl-6-chloro-3-(2-(S)-azetidinylmethoxy)pyridine;
5-cyano-6-chloro-3-(2-(S)-azetidinylmethoxy)pyridine;
5-cyano-6-chloro-3-(1-methyl-2-(S)-azetidinylmethoxy)pyridine;
5-carbamoyl-6-chloro-3-(1-methyl-2-(S)-azetidinylmethoxy)pyridine;
6-fluoro-5-methyl-3-(2-(S)-azetidinylmethoxy)pyridine;
6-chloro-5-methyl-3-(2-(S)-azetidinylmethoxy)pyridine;
5-bromo-6-fluoro-3-(2-(S)-azetidinylmethoxy)pyridine;
5-methoxy-6-chloro-3-(2-(S)-azetidinylmethoxy)pyridine;
5-ethoxy-6-chloro-3-(2-(S)-azetidinylmethoxy)pyridine;
5-nitro-6-methyl-3-(2-(S)-azetidinylmethoxy)pyridine;
5,6-dimethyl-3-(2-(S)-azetidinylmethoxy)pyridine;
5-nitro-6-methyl-3-(2-(R)-azetidinylmethoxy)pyridine;
5-nitro-6-methyl-3-(1-methyl-2-(S)-azetidinylmethoxy)pyridine;
5-nitro-6-methyl-3-(1-methyl-2-(R)-azetidinylmethoxy)pyridine;
5-amino-6-methyl-3-(2-(S)-azetidinylmethoxy)pyridine;
5-amino-6-methyl-3-(2-(R)-azetidinylmethoxy)pyridine;
5-amino-6-methyl-3-(1-methyl-2-(S)-azetidinylmethoxy)pyridine;
5-amino-6-methyl-3-(1-methyl-2-(R)-azetidinylmethoxy)pyridine;
5-bromo-6-methyl-3-(2-(S)-azetidinylmethoxy)pyridine;
5-bromo-6-methyl-3-(2-(R)-azetidinylmethoxy)pyridine;
5-bromo-6-methyl-3-(1-methyl-2-(R)-azetinylmethoxy)pyridine;
5-bromo-6-fluoro-3-(2-(R)-azetidinylmethoxy)pyridine;
5-bromo-6-fluoro-3-(1-methyl-2-(S)-azetidinylmethoxy)pyridine;
5-bromo-6-fluoro-3-(1-methyl-2-(R)-azetidinylmethoxy)pyridine;
5-Bromo-6-chloro-3-(2-(R)-azetidinylmethoxy)pyridine;
5-Bromo-6-chloro-3-(1-methyl-2-(R)-azetidinylmethoxy)pyridine;
5-cyano-6-methyl-3-(2-(S)-azetidinylmethoxy)pyridine;
5-cyano-6-methyl-3-(2-(R)-azetidinylmethoxy)pyridine;
5-cyano-6-methyl-3-(1-methyl-2-(S)-azetidinylmethoxy)pyridine;
5-cyano-6-methyl-3-(1-methyl-2-(R)-azetidinylmethoxy)pyridine;
5-cyano-6-fluoro-3-(2-(S)-azetidinylmethoxy)pyridine;
5-cyano-6-fluoro-3-(2-(R)-azetidinylmethoxy)pyridine;
5-cyano-6-fluoro-3-(1-methyl-2-(S)-azetidinylmethoxy)pyridine;
5-cyano-6-fluoro-3-(1-methyl-2-(R)-azetidinylmethoxy)pyridine;
5-cyano-6-chloro-3-(2-(R)-azetidinylmethoxy)pyridine;
5-cyano-6-chloro-3-(1-methyl-2-(R)-azetidinylmethoxy)pyridine;
5-carbamoyl-6-methyl-3-(2-(S)-azetidinylmethoxy)pyridine;
5-carbamoyl-6-methyl-3-(2-(R)-azetidinylmethoxy)pyridine;
5-carbamoyl-6-methyl-3-(1-methyl-2-(S)-azetidinylmethoxy)pyridine;
5-carbamoyl-6-methyl-3-(1-methyl-2-(R)-azetidinylmethoxy)pyridine;
5-carbamoyl-6-fluoro-3-(2-(S)-azetidinylmethoxy)pyridine;
5-carbamoyl-6-fluoro-3-(2-(R)-azetidinylmethoxy)pyridine;
5-carbamoyl-6-fluoro-3-(1-methyl-2-(S)-azetidinylmethoxy)pyridine;
5-carbamoyl-6-fluoro-3-(1-methyl-2-(R)-azetidinylmethoxy)pyridine;
5-carbamoyl-6-chloro-3-(2-(S)-azetidinylmethoxy)pyridine;
5-carbamoyl-6-chloro-3-(2-(R)-azetidinylmethoxy)pyridine;
5-carbamoyl-6-chloro-3-(1-methyl-2-(R)-azetidinylmethoxy)pyridine;
5-amino-6-fluoro-3-(2-(S)-azetidinylmethoxy)pyridine;
5-amino-6-fluoro-3-(2-(R)-azetidinylmethoxy)pyridine;
5-amino-6-fluoro-3-(1-methyl-2-(S)-azetidinylmethoxy)pyridine;
5-amino-6-fluoro-3-(1-methyl-2-(R)-azetidinylmethoxy)pyridine;
5-amino-6-chloro-3-(2-(S)-azetidinylmethoxy)pyridine;
5-amino-6-chloro-3-(2-(R)-azetidinylmethoxy)pyridine;
5-amino-6-chloro-3-(1-methyl-2-(S)-azetidinylmethoxy)pyridine;
5-amino-6-chloro-3-(1-methyl-2-(R)-azetidinylmethoxy)pyridine;
5-formamido-6-methyl-3-(2-(S)-azetidinylmethoxy)pyridine;
5-formamido-6-methyl-3-(2-(R)-azetidinylmethoxy)pyridine;
5-formamido-6-methyl-3-(1-methyl-2-(S)-azetidinylmethoxy)pyridine;
5-formamido-6-methyl-3-(1-methyl-2-(R)-azetidinylmethoxy)pyridine;
5-acetamido-6-fluoro-3-(2-(S)-azetidinylmethoxy)pyridine;
5-acetamido-6-fluoro-3-(2-(R)-azetidinylmethoxy)pyridine;
5-acetamido-6-fluoro-3-(1-methyl-2-(S)-azetidinylmethoxy)pyridine;
5-acetamido-6-fluoro-3-(1-methyl-2-(R)-azetidinylmethoxy)pyridine;
5-acetamido-6-chloro-3-(2-(S)-azetidinylmethoxy)pyridine;
5-acetamido-6-chloro-3-(2-(R)-azetidinylmethoxy)pyridine;
5-acetamido-6-chloro-3-(1-methyl-2-(S)-azetidinylmethoxy)pyridine;
5-acetamido-6-chloro-3-(1-methyl-2-(R)-azetidinylmethoxy)pyridine;
5-methylamino-6-methyl-3-(2-(S)-azetidinylmethoxy)pyridine;
5-methylamino-6-methyl-3-(2-(R)-azetidinylmethoxy)pyridine;
5-methylamino-6-methyl-3-(1-methyl-2-(S)-azetidinylmethoxy)pyridine;
5-methylamino-6-methyl-3-(1-methyl-2-(R)-azetidinylmethoxy)pyridine;
5-ethylamino-6-fluoro-3-(2-(S)-azetidinylmethoxy)pyridine;
5-ethylamino-6-fluoro-3-(2-(R)-azetidinylmethoxy)pyridine;
5-ethylamino-6-fluoro-3-(1-methyl-2-(S)-azetidinylmethoxy)pyridine;
5-ethylamino-6-fluoro-3-(1-methyl-2-(R)-azetidinylmethoxy)pyridine;
5-ethylamino-6-chloro-3-(2-(S)-azetidinylmethoxy)pyridine;
5-ethylamino-6-chloro-3-(2-(R)-azetidinylmethoxy)pyridine;
5-ethylamino-6-chloro-3-(1-methyl-2-(S)-azetidinylmethoxy)pyridine;
5-ethylamino-6-chloro-3-(1-methyl-2-(R)-azetidinylmethoxy)pyridine;
5-ethyl-6-methyl-3-(2-(S)-azetidinylmethoxy)pyridine;
5-ethyl-6-methyl-3-(2-(R)-azetidinylmethoxy)pyridine;
5-ethyl-6-methyl-3-(1-methyl-2-(S)-azetidinylmethoxy)pyridine;
5-ethyl-6-methyl-3-(1-methyl-2-(R)-azetidinylmethoxy)pyridine;
5-ethyl-6-fluoro-3-(2-(S)-azetidinylmethoxy)pyridine;
5-ethyl-6-fluoro-3-(2-(R)-azetidinylmethoxy)pyridine;
5-ethyl-6-fluoro-3-(1-methyl-2-(S)-azetidinylmethoxy)pyridine;
5-ethyl-6-fluoro-3-(1-methyl-2-(R)-azetidinylmethoxy)pyridine;
5-propyl-6-chloro-3-(2-(S)-azetidinylmethoxy)pyridine;
5-propyl-6-chloro-3-(2-(R)-azetidinylmethoxy)pyridine;
5-propyl-6-chloro-3-(1-methyl-2-(S)-azetidinylmethoxy)pyridine;
5-propyl-6-chloro-3-(1-methyl-2-(R)-azetidinylmethoxy)pyridine;
5-methyl-6-fluoro-3-(2-(R)-azetidinylmethoxy)pyridine;
5-methyl-6-fluoro-3-(1-methyl-2-(S)-azetidinylmethoxy)pyridine;
5-methyl-6-fluoro-3-(1-methyl-2-(R)-azetidinylmethoxy)pyridine;
5-methyl-6-chloro-3-(2-(R)-azetidinylmethoxy)pyridine;
5-methyl-6-chloro-3-(1-methyl-2-(S)-azetidinylmethoxy)pyridine;
5-methyl-6-chloro-3-(1-methyl-2-(R)-azetidinylmethoxy)pyridine;
5,6-dimethyl-3-(2-(R)-azetidinylmethoxy)pyridine;
5,6-dimethyl-3-(1-methyl-2-(S)-azetidinylmethoxy)pyridine;
5,6-dimethyl-3-(1-methyl-2-(R)-azetidinylmethoxy)pyridine;
6-fluoro-5-methoxy-3-(2-(S)-azetidinylmethoxy)pyridine;
6-fluoro-5-methoxy-3-(2-(R)-azetidinylmethoxy)pyridine;
6-fluoro-5-methoxy-3-(1-methyl-2-(S)-azetidinylmethoxy)pyridine;
6-fluoro-5-methoxy-3-(1-methyl-2-(R)-azetidinylmethoxy)pyridine;
6-fluoro-5-ethoxy-3-(2-(S)-azetidinylmethoxy)pyridine;
6-fluoro-5-ethoxy-3-(2-(R)-azetidinylmethoxy)pyridine;
6-fluoro-5-ethoxy-3-(1-methyl-2-(S)-azetidinylmethoxy)pyridine; and
6-fluoro-5-ethoxy-3-(1-methyl-2-(R)-azetidinylmethoxy)pyridine; or a
pharmaceutically acceptable salt or ester thereof.

8. A compound as defined by Claim 3 selected from the group consisting of
5-bromo-6-chloro-3-(2-(S)-pyrrolidinylmethoxy)pyridine;
5-bromo-6-chloro-3-(1-methyl-2-(S)-pyrrolidinylmethoxy)pyridine;
5-bromo-6-chloro-3-(2-(R)-pyrrolidinylmethoxy)pyridine;
5-bromo-6-chloro-3-(1-methyl-2-(R)-pyrrolidinylmethoxy)pyridine;
5,6-dichloro-3-(2-(R)-pyrrolidinylmethoxy)pyridine;
5,6-dichloro-3-(1-methyl-2-(R)-pyrrolidinylmethoxy)pyridine;
5,6-dichloro-3-(2-(S)-pyrrolidinylmethoxy)pyridine;
5,6-dichloro-3-(1-methyl-2-(S)-pyrrolidinylmethoxy)pyridine;
5-ethyl-6-chloro-3-(2-(S)-pyrrolidinylmethoxy)pyridine;
5-nitro-6-methyl-3-(2-(S)-pyrrolidinylmethoxy)pyridine;
5-nitro-6-methyl-3-(2-(R)-pyrrolidinylmethoxy)pyridine;
5-nitro-6-methyl-3-(1-methyl-2-(S)-pyrrolidinylmethoxy)pyridine;
5-nitro-6-methyl-3-(1-methyl-2-(R)-pyrrolidinylmethoxy)pyridine;
5-amino-6-methyl-3-(2-(S)-pyrrolidinylmethoxy)pyridine;
5-amino-6-methyl-3-(2-(R)-pyrrolidinylmethoxy)pyridine;
5-amino-6-methyl-3-(1-methyl-2-(S)-pyrrolidinylmethoxy)pyridine;
5-amino-6-methyl-3-(1-methyl-2-(R)-pyrrolidinylmethoxy)pyridine;
5-bromo-6-methyl-3-(2-(S)-pyrrolidinylmethoxy)pyridine;
5-bromo-6-methyl-3-(2-(R)-pyrrolidinylmethoxy)pyridine;
5-bromo-6-methyl-3-(1-methyl-2-(S)-pyrrolidinylmethoxy)pyridine;
5-bromo-6-methyl-3-(1-methyl-2-(R)-pyrrolidinylmethoxy)pyridine;
5-bromo-6-fluoro-3-(2-(S)-pyrrolidinylmethoxy)pyridine;
5-bromo-6-fluero-3-(2-(R)-pyrrolidinylmethoxy)pyridine;
5-bromo-6-fluoro-3-(1-methyl-2-(S)-pyrrolidinylmethoxy)pyridine;
5-bromo-6-fluoro-3-(1-methyl-2-(R)-pyrrolidinylmethoxy)pyridine;
5-cyano-6-methyl-3-(2-(S)-pyrrolidinylmethoxy)pyridine;
5-cyano-6-methyl-3-(2-(R)-pyrrolidinylmethoxy)pyridine;
5-cyano-6-methyl-3-(1-methyl-2-(S)-pyrrolidinylmethoxy)pyridine;
5-cyano-6-methyl-3-(1-methyl-2-(R)-pyrrolidinylmethoxy)pyridine;
5-cyano-6-fluoro-3-(2-(S)-pyrrolidinylmethoxy)pyridine;
5-cyano-6-fluoro-3-(2-(R)-pyrrolidinylmethoxy)pyridine;
5-cyano-6-fluoro-3-(1-methyl-2-(S)-pyrrolidinylmethoxy)pyridine;
5-cyano-6-fluoro-3-(1-methyl-2-(R)-pyrrolidinylmethoxy)pyridine;
5-cyano-6-chloro-3-(2-(S)-pyrrolidinylmethoxy)pyridine;
5-cyano-6-chloro-3-(2-(R)-pyrrolidinylmethoxy)pyridine;
5-cyano-6-chloro-3-(1-methyl-2-(S)-pyrrolidinylmethoxy)pyridine;
5-cyano-6-chloro-3-(1-methyl-2-(R)-pyrrolidinylmethoxy)pyridine;
5-carbamoyl-6-methyl-3-(2-(S)-pyrrolidinylmethoxy)pyridine;
5-carbamoyl-6-methyl-3-(2-(R)-pyrrolidinylmethoxy)pyridine;
5-carbamoyl-6-methyl-3-(1-methyl-2-(S)-pyrrolidinylmethoxy)pyridine;
5-carbamoyl-6-methyl-3-(1-methyl-2-(R)-pyrrolidinylmethoxy)pyridine;
5-carbamoyl-6-fluoro-3-(2-(S)-pyrrolidinylmethoxy)pyridine;
5-carbamoyl-6-fluoro-3-(2-(R)-pyrrolidinylmethoxy)pyridine;
5-carbamoyl-6-fluoro-3-(1-methyl-2-(S)-pyrrolidinylmethoxy)pyridine;
5-carbamoyl-6-fluoro-3-(1-methyl-2-(R)-pyrrolidinylmethoxy)pyridine;
5-carbamoyl-6-chloro-3-(2-(S)-pyrrolidinylmethoxy)pyridine;
5-carbamoyl-6-chloro-3-(2-(R)-pyrrolidinylmethoxy)pyridine;
5-carbamoyl-6-chloro-3-(1-methyl-2-(S)-pyrrolidinylmethoxy)pyridine;
5-carbamoyl-6-chloro-3-(1-methyl-2-(R)-pyrrolidinylmethoxy)pyridine;
5-amino-6-fluoro-3-(2-(S)-pyrrolidinylmethoxy)pyridine;
5-amino-6-fluoro-3-(2-(R)-pyrrolidinylmethoxy)pyridine;
5-amino-6-fluoro-3-(1-methyl-2-(S)-pyrrolidinylmethoxy)pyridine;
5-amino-6-fluoro-3-(1-methyl-2-(R)-pyrrolidinylmethoxy)pyridine;
5-amino-6-chloro-3-(2-(S)-pyrrolidinylmethoxy)pyridine;
5-amino-6-chloro-3-(2-(R)-pyrrolidinylmethoxy)pyridine;
5-amino-6-chloro-3-(1-methyl-2-(S)-pyrrolidinylmethoxy)pyridine;
5-amino-6-chloro-3-(1-methyl-2-(R)-pyrrolidinylmethoxy)pyridine;
5-formamido-6-methyl-3-(2-(S)-pyrrolidinylmethoxy)pyridine;
5-formamido-6-methyl-3-(2-(R)-pyrrolidinylmethoxy)pyridine;
5-formamido-6-methyl-3-(1-methyl-2-(S)-pyrrolidinylmethoxy)pyridine;
5-formamido-6-methyl-3-(1-methyl-2-(R)-pyrrolidinylmethoxy)pyridine;
5-acetamido-6-fluoro-3-(2-(S)-pyrrolidinylmethoxy)pyridine;
5-acetamido-6-fluoro-3-(2-(R)-pyrrolidinylmethoxy)pyridine;
5-acetamido-6-fluoro-3-(1-methyl-2-(S)-pyrrolidinylmethoxy)pyridine;
5-acetamido-6-fluoro-3-(1-methyl-2-(R)-pyrrolidinylmethoxy)pyridine;
5-acetamido-6-chloro-3-(2-(S)-pyrrolidinylmethoxy)pyridine;
5-acetamido-6-chloro-3-(2-(R)-pyrrolidinylmethoxy)pyridine;
5-acetamido-6-chloro-3-(1-methyl-2-(S)-pyrrolidinylmethoxy)pyridine;
5-acetamido-6-chloro-3-(1-methyl-2-(R)-pyrrolidinylmethoxy)pyridine;
5-methylamino-6-methyl-3-(2-(S)-pyrrolidinylmethoxy)pyridine;
5-methylamino-6-methyl-3-(2-(R)-pyrrolidinylmethoxy)pyridine;
5-methylamino-6-methyl-3-(1-methyl-2-(S)-pyrrolidinylmethoxy)pyridine;
5-methylamino-6-methyl-3-(1-methyl-2-(R)-pyrrolidinylmethoxy)pyridine;
5-ethylamino-6-fluoro-3-(2-(S)-pyrrolidinylmethoxy)pyridine;
5-ethylamino-6-fluoro-3-(2-(R)-pyrrolidinylmethoxy)pyridine;
5-ethylamino-6-fluoro-3-(1-methyl-2-(S)-pyrrolidinylmethoxy)pyridine;
5-ethylamino-6-fluoro-3-(1-methyl-2-(R)-pyrrolidinylmethoxy)pyridine;
5-ethylamino-6-chloro-3-(2-(S)-pyrrolidinylmethoxy)pyridine;
5-ethylamio-6-chloro-3-(2-(R)-pyrrolidinylmethoxy)pyridine;
5-ethylamino-6-chloro-3-(1-methyl-2-(S)-pyrrolidinylmethoxy)pyridine;
5-ethylamino-6-chloro-3-(1-methyl-2-(R)-pyrrolidinylmethoxy)pyridine;
5-ethyl-6-methyl-3-(2-(S)-pyrrolidinylmethoxy)pyridine;
5-ethyl-6-methyl-3-(2-(R)-pyrrolidinylmethoxy)pyridine;
5-ethyl-6-methyl-3-(1-methyl-2-(S)pyrrolidinylmethoxy)pyridine;
5-ethyl-6-methyl-3-(1-methyl-2-(R)-pyrrolidinylmethoxy)pyridine;
5-ethyl-6-fluoro-3-(2-(S)-pyrrolidinylmethoxy)pyridine;
5-ethyl-6-fluoro-3-(2-(R)-pyrrolidinylmethoxy)pyridine;
5-ethyl-6-fluoro-3-(1-methyl-2-(S)-pyrrolidinylmethoxy)pyridine;
5-ethyl-6-fluoro-3-(1-methyl-2-(R)-pyrrolidinylmethoxy)pyridine;
5-propyl-6-chloro-3-(2-(S)-pyrrolidinylmethoxy)pyridine;
5-propyl-6-chloro-3-(2-(R)-pyrrolidinylmethoxy)pyridine;
5-propyl-6-chloro-3-(1-methyl-2-(S)-pyrrolidinylmethoxy)pyridine;
5-propyl-6-chloro-3-(1-methyl-2-(R)-pyrrolidinylmethoxy)pyridine;
5-methyl-6-fluoro-3-(2-(S)-pyrrolidinylmethoxy)pyridine;
5-methyl-6-fluoro-3-(2-(R)-pyrrolidinylmethoxy)pyridine;
5-methyl-6-fluoro-3-(1-methyl-2-(S)-pyrrolidinylmethoxy)pyridine;
5-methyl-6-fluoro-3-(1-methyl-2-(R)-pyrrolidinylmethoxy)pyridine;
5-methyl-6-chloro-3-(2-(S)-pyrrolidinylmethoxy)pyridine;
5-methyl-6-chloro-3-(2-(R)-pyrrolidinylmethoxy)pyridine;
5-methyl-6-chloro-3-(1-methyl-2-(S)-pyrrolidinylmethoxy)pyridine;
5-methyl-6-chloro-3-(1-methyl-2-(R)-pyrrolidinylmethoxy)pyridine;
5,6-dimethyl-3-(2-(S)-pyrrolidinylmethoxy)pyridine;
5,6-dimethyl-3-(2-(R)-pyrrolidinylmethoxy)pyridine;
5,6-dimethyl-3-(1-methyl-2-(S)-pyrrolidinylmethoxy)pyridine;
5,6-dimethyl-3-(1-methyl-2-(R)-pyrrolidinylmethoxy)pyridine;
6-fluoro-5-methoxy-3-(2-(S)-pyrrolidinylmethoxy)pyridine;
6-fluoro-5-methoxy-3-(2-(R)-pyrrolidinylmethoxy)pyridine;
6-fluoro-5-methoxy-3-(1-methyl-2-(S)-pyrrolidinylmethoxy)pyridine;
6-fluoro-5-methoxy-3-(1-methyl-2-(R)-pyrrolidinylmethoxy)pyridine;
6-fluoro-5-ethoxy-3-(2-(S)-pyrrolidinylmethoxy)pyridine;
6-fluoro-5-ethoxy-3-(2-(R)-pyrrolidinylmethoxy)pyridine;
6-fluoro-5-ethoxy-3-(1-methyl-2-(S)-pyrrolidinylmethoxy)pyridine; and
6-fluoro-5-ethoxy-3-(1-methyl-2-(R)-pyrrolidinylmethoxy)pyridine; or a
pharmaceutically acceptable salt or ester thereof.

9. A pharmaceutical composition comprising an amount of a compound as defined by Claim 1 effective to control chemical synaptic transmission in a mammal in combination with a pharmaceutically acceptable carrier.

10. A compound of Claim 1 for use as a therapeutic agent.

11. Use of a compound as defined by Claim 1 for manufacturing a medicament for controlling chemical synaptic transmission in a mammal.

## Patentansprüche

1. Eine Verbindung oder pharmazeutisch verträgliches Salz oder Ester davon, worin die Verbindung folgende Struktur hat: 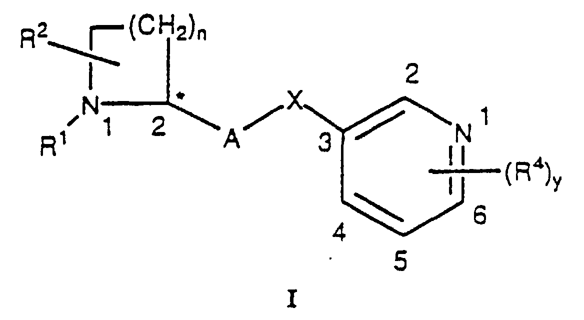 worin das Sternchen ein chirales Zentrum kennzeichnet;
**n** ist eine ganze Zahl gewählt aus 1, 2 oder 3;
**X** ist Sauerstoff oder Schwefel;
**R**^{**1**} ist gewählt aus der Gruppe bestehend aus Wasserstoff, Allyl und C₁-C₆-Alkyl;
**R**^{**2**} ist Wasserstoff oder, wenn n=2, auch ein einzelner Substituent gewählt aus der Gruppe bestehend aus
CH₂OH,
CH₂F,
CH₂CN,
CH₂OCH₃,
Br,
Cl,
F,
OH,
CN, C₁-C₃-Alkoxyl, OCOCH₃ und O-Methansulfonyl,
unter der Voraussetzung, daß, wenn R¹ an der 3-Position oder der 5-Position des Pyrrolidinylringes substituiert ist, es eine C₁-C₃-Alkylgruppe ist;
A ist gewählt aus der Gruppe bestehend aus 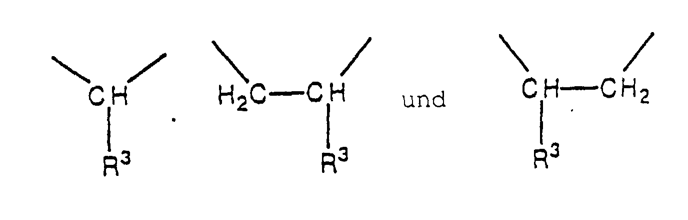 worin R³ H oder C₁-C₆-Alkyl ist;
y ist 2; und
R⁴ ist an den 2,5-, 2,6- oder 5,6-Positionen des Pyridinringes substituiert, wobei der Substituent an der 2-Position gewählt ist aus der Gruppe bestehend aus
-Br,
-Cl,
-F,
-OH,
-(C₁-C₄ Alkyl) und
-(C₁-C₃ Alkoxy); und
die Substituenten an den 5- oder 6-Positionen des Pyridinringes sind gewählt aus der Gruppe bestehend aus
-Br,
-Cl,
-F,
-OH,
-(C₁-C₄ Alkyl),
-CN,
-CF₃,
-NO₂,
-CH₂OH,
-CH₂CN,
-(C₁-C₃ Alkoxy),
-NH₂,
-NH-CHO,
-NHCO(C₁-C₃ Alkyl),
-N(C₁-C₃ Alkyl)CO(C₁-C₃ Alkyl),
-NH-(C₁-C₃ Alkyl, N(C₁-C₃ Alkyl)₂,
-COOH,
-COO(C₁-C₃ Alkyl),
-CONH₂,
-CONH-(C₁-C₃ Alkyl),
-CONHBenzyl, und
-OCO(C₁-C₃ Alkyl).

2. Eine Verbindung wie in Anspruch 1 definiert, oder ein pharmazeutisch verträgliches Salz oder Ester davon, wobei n=1 ist.

3. Eine Verbindung wie in Anspruch 1 definiert, oder ein pharmazeutisch verträgliches Salz oder Ester davon, wobei n=2 ist.

4. Eine Verbindung wie in Anspruch 1 definiert, oder ein pharmazeutisch verträgliches Salz oder Ester davon, wobei n=3 ist.

5. Eine Verbindung wie in Anspruch 1 definiert, oder ein pharmazeutisch verträgliches Salz oder Ester davon, wobei X Sauerstoff ist.

6. Eine Verbindung wie in Anspruch 1 definiert, oder ein pharmazeutisch verträgliches Salz oder Ester davon, wobei X Schwefel ist.

7. Eine Verbindung wie in Anspruch 2 definiert, gewählt aus der Gruppe bestehend aus
5-Brom-6-chlor-3-(2-(S)-azetidinylmethoxy)pyridin;
5-Brom-6-chlor-3-(1-methyl-2-(S)-azetidinylmethoxy)pyridin;
5,6-Dichlor-3-(2-(S)-azetidinylmethoxy)pyridin;
5,6-Dichlor-3-(1-methyl-2-(S)-azetidinylmethoxy)pyridin;
5,6-Dichlor-3-(2-(R)-azetidinylmethoxy)pyridin;
5,6-Dichlor-3-(1-methyl-2-(R)-azetidinylmethoxy)pyridin;
5-Ethyl-6-chlor-3-(2-(S)-azetidinylmethoxy)pyridin;
5-Cyano-6-chlor-3-(2-S)-azetidinylmethoxy)pyridin;
5-Cyano-6-chlor-3-(1-methyl-2-(S)-azetidinylmethoxy)pyridin;
5-Carbamoyl-6-chlor-3-(1-methyl-2-(S)-azetidinylmethoxy)pyridin;
6-Fluor-5-methyl-3-(2-(S)-azetidinylmethoxy)pyridin;
6-Chlor-5-methyl-3-(2-(S)-azetidinylmethoxy)pyridin;
5-Brom-6-fluor-3-(2-(S)-azetidinylmethoxy)pyridin;
5-Methoxy-6-chlor-3-(2-(S)-azetidinylmethoxy)pyridin;
5-Ethoxy-6-chlor-3-(2-(S)-azetidinylmethoxy)pyridin;
5-Nitro-6-methyl-3-(2-(S)-azetidinylmethoxy)pyridin;
5,6-Dimethyl-3-(2-(S)-azetidinylmethoxy)pyridin;
5-Nitro-6-methyl-3-(2-(R)-azetidinylmethoxy)pyridin;
5-Nitro-6-methyl-3-(1-methyl-2-(S)-azetidinylmethoxy)pyridin;
5-Nitro-6-methyl-3-(1-methyl-2-(R)-azetidinylmethoxy)pyridin;
5-Amino-6-methyl-3-(2-(S)-azetidinylmethoxy)pyridin;
5-Amino-6-methyl-3-(2-(R)-azetidinylmethoxy)pyridin;
5-Amino-6-methyl-3-(1-methyl-2-(S)-azetidinylmethoxy)pyridin;
5-Amino-6-methyl-3-(1-methyl-2-(R)-azetidinylmethoxy)pyridin;
5-Brom-6-methyl-3-(2-(S)-azetidinylmethoxy)pyridin;
5-Brom-6-methyl-3-(2-(R)-azetidinylmethoxy)pyridin;
5-Brom-6-methyl-3-(1-methyl-2-(R)-azetidinylmethoxy)pyridin;
5-Brom-6-fluor-3-(2-(R)-azetidinylmethoxy)pyridin;
5-Brom-6-fluor-3-(1-methyl-2-(S)-azetidinylmethoxy)pyridin;
5-Brom-6-fluor-3-(1-methyl-2-(R)-azetidinylmethoxy)pyridin;
5-Brom-6-chlor-3-(2-(R)-azetidinylmethoxy)pyridin;
5-Brom-6-chlor-3-(1-methyl-2-(R)-azetidinylmethoxy)pyridin;
5-Cyano-6-methyl-3-(2-(S)-azetidinylmethoxy)pyridin;
5-Cyano-6-methyl-3-(2-(R)-azetidinylmethoxy)pyridin;
5-Cyano-6-methyl-3-(1-methyl-2-(S)-azetidinylmethoxy)pyridin;
5-Cyano-6-methyl-3-(1-methyl-2-(R)-azetidinylmethoxy)pyridin;
5-Cyano-6-fluor-3-(2-(S)-azetidinylmethoxy)pyridin;
5-Cyano-6-fluor-3-(2-(R)-azetidinylmethoxy)pyridin;
5-Cyano-6-fluor-3-(1-methyl-2-(S)-azetidinylmethoxy)pyridin;
5-Cyano-6-fluor-3-(1-methyl-2-(R)-azetidinylmethoxy)pyridin;
5-Cyano-6-chlor-3-(2-(R)-azetidinylmethoxy)pyridin;
5-Cyano-6-chlor-3-(1-methyl-2-(R)-azetidinylmethoxy)pyridin;
5-Carbamoyl-6-methyl-3-(2-(S)-azetidinylmethoxy)pyridin;
5-Carbamoyl-6-methyl-3-(2-(R)-azetidinylmethoxy)pyridin;
5-Carbamoyl-6-methyl-3-(1-methyl-2-(S)-azetidinylmethoxy)pyridin;
5-Carbamoyl-6-methyl-3-(1-methyl-2-(R)-azetidinylmethoxy) pyridin;
5-Carbamoyl-6-fluor-3-(2-(S)-azetidinylmethoxy)pyridin;
5-Carbamoyl-6-fluor-3-(2-(R)-azetidinylmethoxy)pyridin;
5-Carbamoyl-6-fluor-3-(1-methyl-2-(S)-azetidinylmethoxy)pyridin;
5-Carbamoyl-6-fluor-3-(1-methyl-2-(R)-azetidinylmethoxy)pyridin;
5-Carbamoyl-6-chlor-3-(2-(S)-azetidinylmethoxy)pyridin;
5-Carbamoyl-6-chlor-3-(2-(R)-azetidinylmethoxy)pyridin;
5-Carbamoyl-6-chlor-3-(1-methyl-2-(R)-azetidinylmethoxy)pyridin;
5-Amino-6-fluor-3-(2-(S)-azetidinylmethoxy)pyridin;
5-Amino-6-fluor-3-(2-(R)-azetidinylmethoxy)pyridin;
5-Amino-6-fluor-3-(1-methyl-2-(S)-azetidinylmethoxy)pyridin;
5-Amino-6-fluor-3-(1-methyl-2-(R)-azetidinylmethoxy)pyridin;
5-Amino-6-chlor-3-(2-(S)-azetidinylmethoxy)pyridin;
5-Amino-6-chlor-3-(2-(R)-azetidinylmethoxy)pyridin;
5-Amino-6-chlor-3-(1-methyl-2-(S)-azetidinylmethoxy)pyridin;
5-Amino-6-chlor-3-(1-methyl-2-(R)-azetidinylmethoxy)pyridin;
5-Formamido-6-methyl-3-(2-(S)-azetidinylmethoxy)pyridin;
5-Formamido-6-methyl-3-(2-(R)-azetidinylmethoxy)pyridin;
5-Formamido-6-methyl-3-(1-methyl-2-(S)-azetidinylmethoxy) pyridin;
5-Formamido-6-methyl-3-(1-methyl-2-(R)-azetidinylmethoxy)pyridin;
5-Acetamido-6-fluor-3-(2-(S)-azetidinylmethoxy)pyridin;
5-Acetamido-6-fluor-3-(2-(R)-azetidinylmethoxy)pyridin;
5-Acetamido-6-fluor-3-(1-methyl-2-(S)-azetidinylmethoxy)pyridin;
5-Acetamido-6-fluor-3-(1-methyl-2-(R)-azetidinylmethoxy)pyridin;
5-Acetamido-6-chlor-3-(2-(S)-azetidinylmethoxy)pyridin;
5-Acetamido-6-chlor-3-(2-(R)-azetidinylmethoxy)pyridin;
5-Acetamido-6-chlor-3-(1-methyl-2-(S)-azetidinylmethoxy)pyridin;
5-Acetamido-6-chlor-3-(1-methyl-2-(R)-azetidinylmethoxy)pyridin;
5-Methylamino-6-methyl-3-(2-(S)-azetidinylmethoxy)pyiridin;
5-Methylamino-6-methyl-3-(2-(R)-azetidinylmethoxy)pyiridin;
5-Methylamino-6-methyl-3-(1-methyl-2-(S)-azetidinylmethoxy)pyiridin;
5-Methylamino-6-methyl-3-(1-methyl-2-(R)-azetidinylmethoxy)pyiridin;
5-Ethylamino-6-fluor-3-(2-(S)-azetidinylmethoxy)pyiridin;
5-Ethylamino-6-fluor-3-(2-(R)-azetidinylmethoxy)pyiridin;
5-Ethylamino-6-fluor-3-(1-methyl-2-(S)-azetidinylmethoxy)pyiridin;
5-Ethylamino-6-fluor-3-(1-methyl-2-(R)-azetidinylmethoxy)pyiridin;
5-Ethylamino-6-chlor-3-(2-(S)-azetidinylmethoxy)pyiridin;
5-Ethylamino-6-chlor-3-(2-(R)-azetidinylmethoxy)pyiridin;
5-Ethylamino-6-chlor-3-(1-methyl-2-(S)-azetidinylmethoxy)pyiridin;
5-Ethylamino-6-chlor-3-(1-methyl-2-(R)-azetidinylmethoxy)pyiridin;
5-Ethyl-6-methyl-3-(2-(S)-azetidinylmethoxy)pyiridin;
5-Ethyl-6-methyl-3-(2-(R)-azetidinylmethoxy)pyiridin;
5-Ethyl-6-methyl-3-(1-methyl-2-(S)-azetidinylmethoxy)pyiridin;
5-Ethyl-6-methyl-3-(1-methyl-2-(R)-azetidinylmethoxy)pyiridin;
5-Ethyl-6-fluor-3-(2-(S)-azetidinylmethoxy)pyiridin;
5-Ethyl-6-fluor-3-(2-(R)-azetidinylmethoxy)pyiridin;
5-Ethyl-6-fluor-3-(1-methyl-2-(S)-azetidinylmethoxy)pyiridin;
5-Ethyl-6-fluor-3-(1-methyl-2-(R)-azetidinylmethoxy)pyiridin;
5-Propyl-6-chlor-3-(2-(S)-azetidinylmethoxy)pyiridin;
5-Propyl-6-chlor-3-(2-(R)-azetidinylmethoxy)pyiridin;
5-Propyl-6-chlor-3-(1-methyl-2-(S)-azetidinylmethoxy)pyiridin;
5-Propyl-6-chlor-3-(1-methyl-2-(R)-azetidinylmethoxy)pyiridin;
5-Methyl-6-fluor-3-(2-(R)-azetidinylmethoxy)pyiridin;
5-Methyl-6-fluor-3-(1-methyl-2-(S)-azetidinylmethoxy)pyiridin;
5-Methyl-6-fluor-3-(1-methyl-2-(R)-azetidinylmethoxy)pyiridin;
5-Methyl-6-chlor-3-(2-(R)-azetidinylmethoxy)pyiridin;
5-Methyl-6-chlor-3-(1-methyl-2-(S)-azetidinylmethoxy)pyiridin;
5-Methyl-6-chlor-3-(1-inethyl-2-(R)-azetidinylmethoxy)pyiridin;
5,6-Dimethyl-3-(2-(R)-azetidinylmethoxy)pyiridin;
5,6-Dimethyl-3-(1-methyl-2-(S)-azetidinylmethoxy)pyiridin;
5,6-Dimethyl-3-(1-methyl-2-(R)-azetidinylmethoxy)pyiridin;
6-Fluor-5-methoxy-3-(2-(S)-azetidinylmethoxy)pyiridin;
6-Fluor-5-methoxy-3-(2-(R)-azetidinylmethoxy)pyiridin;
6-Fluor-5-methoxy-3-(1-methyl-2-(S)-azetidinylmethoxy)pyiridin;
6-Fluor-5-methoxy-3-(1-methyl-2-(R)-azetidinylmethoxy)pyiridin;
6-Fluor-5-ethoxy-3-(2-(S)-azetidinylmethoxy)pyiridin;
6-Fluor-5-ethoxy-3-(2-(R)-azetidinylmethoxy)pyiridin;
6-Fluor-5-ethoxy-3-(1-methyl-2-(S)-azetidinylmethoxy)pyiridin; und
6-Fluor-5-ethoxy-3-(1-methyl-2-(R)-azetidinylmethoxy)pyiridin;
oder ein pharmazeutisch verträgliches Salz oder Ester davon

8. Eine Verbindung wie in Anspruch 3 definiert, gewählt aus der Gruppe bestehend aus
5-Brom-6-chlor-3-(2-(S)-pyrrolidinylmethoxy)pyridin;
5-Brom-6-chlor-3-(1-methyl-2-(S)-pyrrolidinylmethoxy)pyridin;
5-Brom-6-chlor-3-(2-(R)-pyrrolidinylmethoxy)pyridin;
5-Brom-6-chlor-3-(1-methyl-2-(R)-pyrrolidinylmethoxy)pyridin;
5,6-Dichlor-3-(2-(R)-pyrrolidinylmethoxy)pyridin;
5,6-Dichlor-3-(1-methyl-2-(R)-pyrrolidinylmethoxy)pyridin;
5,6-Dichlor-3-(2-(S)-pyrrolidinylmethoxy)pyridin;
5,6-Dichlor-3-(1-methyl-2-(S)-pyrrolidinylmethoxy)pyridin;
5-Ethyl-6-chlor-3-(2-(S)-pyrrolidinylmethoxy)pyridin;
5-Nitro-6-methyl-3-(2-(S)-pyrrolidinylmethoxy)pyridin;
5-Nitro-6-methyl-3-(2-(R)-pyrrolidinylmethoxy)pyridin;
5-Nitro-6-methyl-3-(1-methyl-2-(S)-pyrrolidinylmethoxy)pyridin;
5-Nitro-6-methyl-3-(1-methyl-2-(R)-pyrrolidinylmethoxy)pyridin;
5-Amino-6-methyl-3-(2-(S)-pyrrolidinylmethoxy)pyridin;
5-Amino-6-methyl-3-(2-(R)-pyrrolidinylmethoxy)pyridin;
5-Amino-6-methyl-3-(1-methyl-2-(S)-pyrrolidinylmethoxy)pyridin;
5-Amino-6-methyl-3-(1-methyl-2-(R)-pyrrolidinylmethoxy)pyridin;
5-Brom-6-methyl-3-(2-(S)-pyrrolidinylmethoxy)pyridin;
5-Brom-6-methyl-3-(2-(R)-pyrrolidinylmethoxy)pyridin;
5-Brom-6-methyl-3-(1-methyl-2-(S)-pyrrolidinylmethoxy)pyridin;
5-Brom-6-methyl-3-(1-methyl-2-(R)-pyrrolidinylmethoxy)pyridin;
5-Brom-6-fluor-3-(2-(S)-pyrrolidinylmethoxy)pyridin;
5-Brom-6-fluor-3-(2-(R)-pyrrolidinylmethoxy)pyridin;
5-Brom-6-fluor-3-(1-methyl-2-(S)-pyrrolidinylmethoxy)pyridin;
5-Brom-6-fluor-3-(1-methyl-2-(R)-pyrrolidinylmethoxy)pyridin;
5-Cyano-6-methyl-3-(2-(S)-pyrrolidinylmethoxy)pyridin;
5-Cyano-6-methyl-3-(2-(R)-pyrrolidinylmethoxy)pyridin;
5-Cyano-6-methyl-3-(1-methyl-2-(S)-pyrrolidinylmethoxy)pyridin;
5-Cyano-6-methyl-3-(1-methyl-2-(R)-pyrrolidinylmethoxy)pyridin;
5-Cyano-6-fluor-3-(2-(S)-pyrrolidinylmethoxy)pyridin;
5-Cyano-6-fluor-3-(2-(R)-pyrrolidinylmethoxy)pyridin;
5-Cyano-6-fluor-3-(1-methyl-2-(S)-pyrrolidinylmethoxy)pyridin;
5-Cyano-6-fluor-3-(1-methyl-2-(R)-pyrrolidinylmethoxy)pyridin;
5-Cyano-6-chlor-3-(2-(S)-pyrrolidinylmethoxy)pyridin;
5-Cyano-6-chlor-3-(2-(R)-pyrrolidinylmethoxy)pyridin;
5-Cyano-6-chlor-3-(1-methyl-2-(S)-pyrrolidinylmethoxy)pyridin;
5-Cyano-6-chlor-3-(1-methyl-2-(R)-pyrrolidinylmethoxy)pyridin;
5-Carbamoyl-6-methyl-3-(2-(S)-pyrrolidinylmethoxy)pyridin;
5-Carbamoyl-6-methyl-3-(2-(R)-pyrrolidinylmethoxy)pyridin;
5-Carbamoyl-6-methyl-3-(1-methyl-2-(S)-pyrrolidinylmethoxy)pyridin;
5-Carbamoyl-6-methyl-3-(1-methyl-2-(R)-pyrrolidinylmethoxy)pyridin;
5-Carbamoyl-6-fluor-3-(2-(S)-pyrrolidinylmethoxy)pyridin;
5-Carbamoyl-6-fluor-3-(2-(R)-pyrrolidinylmethoxy)pyridin;
5-Carbamoyl-6-fluor-3-(1-methyl-2-(S)-pyrrolidinylmethoxy)pyridin;
5-Carbamoyl-6-fluor-3-(1-methyl-2-(R)-pyrrolidinylmethoxy)pyridin;
5-Carbamoyl-6-chlor-3-(2-(S)-pyrrolidinylmethoxy)pyridin;
5-Carbamoyl-6-chlor-3-(2-(R)-pyrrolidinylmethoxy)pyridin;
5-Carbamoyl-6-chlor-3-(1-methyl-2-(S)-pyrrolidinylmethoxy)pyridin;
5-Carbamoyl-6-chlor-3-(1-methyl-2-(R)-pyrrolidinylmethoxy)pyridin;
5-Amino-6-fluor-3-(2-(S)-pyrrolidinylmethoxy)pyridin;
5-Amino-6-fluor-3-(2-(R)-pyrrolidinylmethoxy)pyridin;
5-Amino-6-fluor-3-(1-methyl-2-(S)-pyrrolidinylmethoxy)pyridin;
5-Amino-6-fluor-3-(1-methyl-2-(R)-pyrrolidinylmethoxy)pyridin;
5-Amino-6-chlor-3-(2-(S)-pyrrolidinylmethoxy)pyridin;
5-Amino-6-chlor-3-(2-(R)-pyrrolidinylmethoxy)pyridin;
5-Amino-6-chlor-3-(1-methyl-2-(S)-pyrrolidinylmethoxy)pyridin;
5-Amino-6-chlor-3-(1-methyl-2-(R)-pyrrolidinylmethoxy)pyridin;
5-Formamido-6-methyl-3-(2-(S)-pyrrolidinylmethoxy)pyridin;
5-Formamido-6-methyl-3-(2-(R)-pyrrolidinylmethoxy)pyridin;
5-Formamido-6-methyl-3-(1-methyl-2-(S)-pyrrolidinylmethoxy)pyridin;
5-Formamido-6-methyl-3-(1-methyl-2-(R)-pyrrolidinylmethoxy)pyridin;
5-Acetamido-6-fluor-3-(2-(S)-pyrrolidinyl-methoxy)pyridin;
5-Acetamido-6-fluor-3-(2-(R)-pyrrolidinyl-methoxy)pyridin;
5-Acetamido-6-fluor-3-(1-methyl-2-(S)-pyrrolidinylmethoxy)pyridin;
5-Acetamido-6-fluor-3-(1-methyl-2-(R)-pyrrolidinylmethoxy)pyridin;
5-Acetamido-6-chlor-3-(2-(S)-pyrrolidinylmethoxy)pyridin;
5-Acetamido-6-chlor-3-(2-(R)-pyrrolidinylmethoxy)pyridin;
5-Acetamido-6-chlor-3-(1-methyl-2-(S)-pyrrolidinylmethoxy) pyridin;
5-Acetamido-6-chlor-3-(1-methyl-2-(R)-pyrrolidinylmethoxy)pyridin;
5-Methylamino-6-methyl-3-(2-(S)-pyrrolidinylmethoxy)pyridin;
5-Methylamino-6-methyl-3-(2-(R)-pyrrolidinylmethoxy)pyridin;
5-Methylamino-6-methyl-3-(1-methyl-2-(S)-pyrrolidinylmethoxy)pyridin;
5-Methylamino-6-methyl-3-(1-methyl-2-(R)-pyrrolidinylmethoxy)pyridin;
5-Ethylamino-6-fluor-3-(2-(S)-pyrrolidinylmethoxy)pyridin;
5-Ethylamino-6-fluor-3-(2-(R)-pyrrolidinylmethoxy)pyridin;
5-Ethylamino-6-fluor-3-(1-methyl-2-(S)-pyrrolidinylmethoxy)pyridin;
5-Ethylamino-6-fluor-3-(1-methyl-2-(R)-pyrrolidinylmethoxy)pyridin;
5-Ethylamino-6-chlor-3-(2-(S)-pyrrolidinylmethoxy)pyridin;
5-Ethylamino-6-chlor-3-(2-(R)-pyrrolidinylmethoxy)pyridin;
5-Ethylamino-6-chlor-3-(1-methyl-2-(S)-pyrrolidinylmethoxy)pyridin;
5-Ethylamino-6-chlor-3-(1-methyl-2-(R)-pyrrolidinylmethoxy)pyridin;
5-Ethyl-6-methyl-3-(2-(S)-pyrrolidinylmethoxy)pyridin;
5-Ethyl-6-methyl-3-(2-(R)-pyrrolidinylmethoxy)pyridin;
5-Ethyl-6-methyl-3-(1-methyl-2-(S)-pyrrolidinylmethoxy)pyridin;
5-Ethyl-6-methyl-3-(1-methyl-2-(R)-pyrrolidinylmethoxy) pyridin;
5-Ethyl-6-fluor-3-(2-(S)-pyrrolidinylmethoxy)pyridin;
5-Ethyl-6-fluor-3-(2-(R)-pyrrolidinylmethoxy)pyridin;
5-Ethyl-6-fluor-3-(1-methyl-2-(S)-pyrrolidinylmethoxy)pyridin;
5-Ethyl-6-fluor-3-(1-methyl-2-(R)-pyrrolidinylmethoxy)pyridin;
5-Propyl-6-chlor-3-(2-(S)-pyrrolidinylmethoxy)pyridin;
5-Propyl-6-chlor-3-(2-(R)-pyrrolidinylmethoxy)pyridin;
5-Propyl-6-chlor-3-(1-methyl-2-(S)-pyrrolidinylmethoxy)pyridin;
5-Propyl-6-chlor-3-(1-methyl-2-(R)-pyrrolidinylmethoxy)pyridin;
5-Methyl-6-fluor-3-(2-(S)-pyrrolidinylmethoxy)pyridin;
5-Methyl-6-fluor-3-(2-(R)-pyrrolidinylmethoxy)pyridin;
5-Methyl-6-fluor-3-(1-methyl-2-(S)-pyrrolidinylmethoxy)pyridin;
5-Methyl-6-fluor-3-(1-methyl-2-(R)-pyrrolidinylmethoxy)pyridin;
5-Methyl-6-chlor-3-(2-(S)-pyrrolidinylmethoxy)pyridin;
5-Methyl-6-chlor-3-(2-(R)-pyrrolidinylmethoxy)pyridin;
5-Methyl-6-chlor-3-(1-methyl-2-(S)-pyrrolidinylmethoxy)pyridin;
5-Methyl-6-chlor-3-(1-methyl-2-(R)-pyrrolidinylmethoxy)pyridin;
5,6-Dimethyl-3-(2-(S)-pyrrolidinylmethoxy)pyridin;
5,6-Dimethyl-3-(2-(R)-pyrrolidinylmethoxy)pyridin;
5,6-Dimethyl-3-(1-methyl-2-(S)-pyrrolidinylmethoxy)pyridin;
5,6-Dimethyl-3-(1-methyl-2-(R)-pyrrolidinylmethoxy)pyridin;
6-Fluor-5-methoxy-3-(2-(S)-pyrrolidinylmethoxy)pyridin;
6-Fluor-5-methoxy-3-(2-(R)-pyrrolidinylmethoxy)pyridin;
6-Fluor-5-methoxy-3-(1-methyl-2-(S)-pyrrolidinylmethoxy)pyridin;
6-Fluor-5-methoxy-3-(1-methyl-2-(R)-pyrrolidinylmethoxy)pyridin;
6-Fluor-5-ethoxy-3-(2-(S)-pyrrolidinylmethoxy) pyridin;
6-Fluor-5-ethoxy-3-(2-(R)-pyrrolidinylmethoxy)pyridin;
6-Fluor-5-ethoxy-3-(1-methyl-2-(S)-pyrrolidinylmethoxy)pyridin; und
6-Fluor-5-ethoxy-3-(1-methyl-2-(R)-pyrrolidinylmethoxy)pyridin; oder ein pharmazeutisch verträgliches Salz oder Ester davon.

9. Eine pharmazeutische Zusammensetzung, die eine Menge einer Verbindung wie in Anspruch 1 definiert umfaßt, wirksam zur Kontrolle chemischer synaptischer Übertragung in einem Säugetier, in Kombination mit einem pharmazeutisch verträglichen Träger.

10. Eine Verbindung gemäß Anspruch 1 zur Verwendung als ein therapeutischer Wirkstoff.

11. Verwendung einer Verbindung wie in Anspruch 1 definiert, zur Herstellung eines Medikamentes zur Kontrolle chemischer synaptischer Übertragung in einem Säugetier.

## Revendications

1. Composé ou son sel ou son ester acceptable du point de vue pharmaceutique, **caractérisé en ce que** ledit composant a la structure : 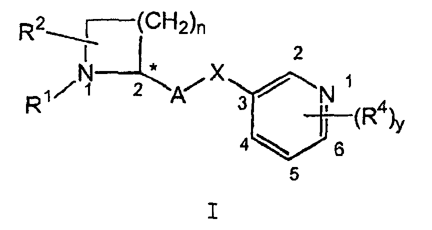 dans laquelle l'astérisque mentionne un centre chiral ;
n est un nombre entier choisi parmi 1, 2, ou 3 ;
X est l'oxygène ou le soufre ;
R¹ est choisi dans le groupe constitué par l'hydrogène, un groupe allyle, et alkyle en C₁-C₆ ;
R² est l'hydrogène ou, quand n = 2, est également un substituant unique choisi dans le groupe constitué par
CH₂OH,
CH₂F,
CH₂CN,
CH₂OCH₃,
Br,
Cl,
F,
OH,
CN,
alcoxyle en C₁-C₃,
OCOCH₃, et
O-méthanesulfonyle,
sous réserve que quand R² est substitué en position 3 ou en position 5 du cycle pyrrolininyle, c'est un groupe alkyle en C₁-C₃ ;
A est choisi dans le groupe constitué par 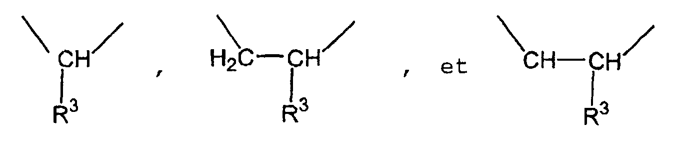
où R³ est H ou un groupe alkyle en C₁-C₆ ;
Y vaut 2 ; et
R⁴ est substitué en positions 2,5, 2,6 ou 5,6 du cycle pyridine où le substituant en position 2 est choisi dans le groupe constitué par
-Br,
-Cl,
-F,
-OH,
-(alkyle en C₁-C₄) et
-(alcoxy en C₁-C₃) ; et
les substituants en position 5 ou 6 du cycle pyridine sont choisis dans le groupe constitué par
-Br,
-Cl,
-F,
-OH,
-(alkyle en C₁-C₄),
-CN,
-CF₃,
-NO₂,
-CH₂OH,
-CH₂CN,
-(alcoxy en C₁-C₃),
-NH₂,
-NH-CHO,
-NHCO(alkyle en C₁-C₃),
-N(alkyl en C₁-C₃)CO(alkyle en C₁-C₃),
-NH-(alkyle en C₁-C₃), N(alkyle en C₁-C₃)₂,
-COOH,
-COO(alkyle en C₁-C₃),
-CONH₂,
-CONH-(alkyle en C₁-C₃),
-CONHbenzyle, et
-OCO(alkyle en C₁-C₃).

2. Composé tel que défini dans la revendication 1 ou son sel ou son ester acceptable du point de vue pharmaceutique dans lequel n=1.

3. Composé tel que défini dans la revendication 1 ou son sel ou son ester acceptable du point de vue pharmaceutique dans lequel n=2.

4. Composé tel que défini dans la revendication 1 ou son sel ou son ester acceptable du point de vue pharmaceutique dans lequel n=3.

5. Composé tel que défini dans la revendication 1 ou son sel ou son ester acceptable du point de vue pharmaceutique dans lequel X est l'oxygène.

6. Composé tel que défini dans la revendication 1 ou son sel ou son ester acceptable du point de vue pharmaceutique dans lequel X est le soufre.

7. Composé tel que défini dans la revendication 2 choisi dans le groupe constitué par
la 5-brome-6-chlore-3-(2-(S)-azétidinylméthoxy)-pyridine ;
la 5-bromo-6-chloro-3-(1-méthyl-2-(S)-azétidinylméthoxy)pyridine ;
la 5,6-dichloro-3-(2-(S)-azétidinylméthoxy)-pyridine ;
la 5,6-dichloro-3-(1-méthyl-2-(S)-azétidinylméthoxy)pyridine ;
la 5,6-dichloro-3-(2-(R)-azétidinylméthoxy)pyridine ;
la 5,6-dichloro-3-(1-méthyl-2-(R)-azétidinylméthoxy)pyridine ;
la 5-éthyl-6-chloro-3-(2-(S)-azétidinylméthoxy)pyridine ;
la 5-cyano-6-chloro-3-(2-(S)-azétidinylméthoxy)pyridine ;
la 5-cyano-6-chloro-3-(1-méthyl-2-(S)-azétidinylméthoxy)pyridine ;
la 5-carbamoyl-6-chloro-3-(1-méthyl-2-(S)azétidinylméthoxy)pyridine;
la 6-fluoro-5-méthyl-3-(2-(S)-azétidinylméthoxy)pyridine ;
la 6-chloro-5-méthyl-3-(2-(S)-azétidinylméthoxy)pyridine ;
la 5-bromo-6-fluoro-3-(2-(S)-azétidinylméthoxy)pyridine ;
la 5-méthoxy-6-chloro-3-(2-(S)-azétidinylméthoxy)pyridine ;
la 5-éthoxy-6-chloro-3-(2-(S)-azétidinylméthoxy)pyridine ;
la 5-nitro-6-méthyl-3-(2-(S)-azétidinylméthoxy)pyridine ;
la 5,6-diméthyl-3-(2-(S)-azétidinylméthoxy)pyridine ;
la 5-nitro-6-méthyl-3-(2-(R)-azétidinylméthoxy)pyridine ;
la 5-nitro-6-méthyl-3-(1-méthyl-2-(S)-azétidinylméthoxy)pyridine ;
la 5-nitro-6-méthyl-3-(1-méthyl-2-(R)-azétidinylméthoxy)pyridine ;
la 5-amino-6-méthyl-3-(2-(S)-azétidinylméthoxy)pyridine ;
la 5-amine-6-méthyl-3-(2-(R)-azétidinylméthoxy)pyridine ;
la 5-amino-6-méthyl-3-(1-méthyl-2-(S)-azétidinylméthoxy)pyridine ;
la 5-amino-6-méthyl-3-(1-méthyl-2-(R)-azétidinylméthoxy)pyridine ;
la 5-bromo-6-méthyl-3-(2-(S)-azétidinylméthoxy)pyridine ;
la 5-bromo-6-méthyl-3-(2-(R)-azétidinylméthoxy)pyridine ;
la 5-bromo-6-méthyl-3-(1-méthyl-2-(R)-azétidinylméthoxy)pyridine ;
la 5-bromo-6-fluoro-3-(2-(R)-azétidinylméthoxy)pyridine ;
la 5-bromo-6-fluoro-3-(1-méthyl-2-(S)-azétidinylméthoxy)pyridine ;
la 5-bromo-6-fluoro-3-(1-méthyl-2-(R)-azétidinylméthoxy)pyridine ;
la 5-bromo-6-chloro-3-(2-(R)-azétidinylméthoxy)pyridine ;
la 5-bromo-6-chloro-3- (1-méthyl-2- (R)-azétidinylméthoxy)pyridine ;
la 5-cyano-6-méthyl-3-(2-(S)-azétidinylméthoxy)pyridine ;
la 5-cyano-6-méthyl-3-(2-(R)-azétidinylméthoxy)pyridine ;
la 5-cyano-6-méthyl-3-(1-méthyl-2-(S)-azétidinylméthoxy)pyridine ;
la 5-cyano-6-méthyl-3-(1-méthyl-2-(R)-azétidinylméthoxy)pyridine ;
la 5-cyano-6-fluoro-3-(2-(S)-azétidinylméthoxy)pyridine ;
la 5-cyano-6-fluoro-3-(2-(R)-azétidinylméthoxy)pyridine ;
la 5-cyano-6-f]uoro-3-(1-méthyl-2-(S)-azétidinylméthoxy)pyridine ;
la 5-cyano-6-fluoro-3-(1-méthyl-2-(R)-azétidinylméthoxy)pyridine ;
la 5-cyano-6-chloro-3-(2-(R)-azétidinylméthoxy)pyridine ;
la 5-cyano-6-chloro-3-(1-méthyl-2-(R)-azétidinylméthoxy)pyridine ;
la 5-carbamoyl-6-méthyl-3-(2-(S)-azétidinylméthoxy)pyridine ;
la 5-carbamoyl-6-méthyl-3-(2-(R)-azétidinylméthoxy)pyridine ;
la 5-carbamoyl-6-méthyl-3-(1-méthyl-2-(S)azétidinylméthoxy)pyridine;
la 5-carbamoyl-6-méthyl-3-(1-méthyl-2-(R)azétidinylméthoxy)pyridine;
la 5-carbamoyl-6-fluoro-3-(2-(S)-azétidinylméthoxy)pyridine ;
la 5-carbamoyl-6-fluoro-3-(2-(R)-azétidinylméthoxy)pyridine ;
la 5-carbamoyl-6-fluoro-3-(1-méthyl-2-(S)azétidinylméthoxy)pyridine ;
la 5-carbamoyl-6-fluoro-3-(1-méthyl-2-(R)azétidinylméthoxy)pyridine;
la 5-carbamoyl-6-chloro-3-(2-(S)-azétidinylméthoxy)pyridine ;
la 5-carbamoyl-6-chloro-3-(2-(R)-azétidinylméthoxy)pyridine ;
la 5-carbamoyl-6-chloro-3-(1-méthyl-2-(R)azétidinylméthoxy)pyridine ;
la 5-amino-6-fluooro-3-(2-(S)-azétidinylméthoxy)pyridine ;
la 5-amino-6-fluoro-3-(2-(R)-azétidinylméthoxy)pyridine ;
la 5-amino-6-fluoro-3-(1-méthyl-2-(S)-azétidinylméthoxy)pyridine ;
la 5-amino-6-fluoro-3-(1-méthyl-2-(R)-azétidinylméthoxy)pyridine ;
la 5-amino-6-chloro-3-(2-(S)-azétidinylméthoxy)pyridine ;
la 5-amino-6-chloro-3-(2-(R)-azétidinylméthoxy)pyridine ;
la 5-amino-6-chloro-3-(1-méthyl-2-(S)-azétidinylméthoxy)pyridine ;
la 5-amino-6-chloro-3-(1-méthyl-2-(R)-azétidinylméthoxy)pyridine ;
la 5-formamido-6-méthyl-3-(2-(S)-azétidinylméthoxy)pyridine ;
la 5-formamido-6-méthyl-3-(2-(R)-azétidinylméthoxy)pyridine ;
la 5-formamido-6-méthyl-3-(1-méthyl-2-(S)-azétidinylméthoxy)pyridine ;
la 5-formamido-6-méthyl-3-(1-méthyl-2-(R)-azétidinylméthoxy)pyridine ;
la 5-acétamido-6-fluoro-3-(2-(S)-azétidinylméthoxy)-pyridine ;
la 5-acétamido-6-fluoro-3-(2-(R)-azétidinylméthoxy)-pyridine ;
la 5-acétamido-6-fluoro-3-(1-méthyl-2-(S)azétidinylméthoxy)pyridine ;
la 5-acétamido-6-fluoro-3-(1-méthyl-2-(R)azétidinylméthoxy)pyridine ;
la 5-acétamido-6-chloro-3-(2-(S)-azétidinylméthoxy)pyridine ;
la 5-acétamido-6-chloro-3-(2-(R)-azétidinylméthoxy)pyridine ;
la 5-acétamido-6-chloro-3-(1-méthyl-2-(S)azétidinylméthoxy)pyridine ;
la 5-acétamido-6-chloro-3-(1-méthyl-2-(R)azétidinyiméthoxy)pyridine ;
la 5-méthylamino-6-méthyl-3-(2-(S)-azétidinylméthoxy)pyridine ;
la 5-méthylamino-6-méthyl-3-(2-(R)-azétidinylméthoxy)pyridine ;
la 5-méthylamino-6-méthyl-3-(1-méthyl-2-(S)azétidinylméthoxy)pyridine ;
la 5-méthylamino-6-méthyl-3-(1-méthyl-2-(R)azétidinylméthoxy)pyridine ;
la 5-éthylamino-6-fluoro-3-(2-(S)-azétidinylméthoxy)pyridine ;
la 5-éthylamino-6-fluoro-3-(2-(R)-azétidinylméthoxy)pyridine ;
la 5-éthylamino-6-fluoro-3-(1-méthyl-2-(S)azétidinylméthoxy)pyridine ;
la 5-éthylamino-6-fluoro-3-(1-méthyl-2-(R)azétidinylméthoxy)pyridine ;
la 5-éthylamino-6-chloro-3-(2-(S)-azétidinylméthoxy)pyridine ;
la 5-éthylamino-6-chloro-3-(2-(R)-azétidinylméthoxy)pyridine ;
la 5-éthylamino-6-chloro-3-(1-méthyl-2-(S)-azétidinylméthoxy)pyridine ;
la 5-éthylamino-6-chlore-3-(1-méthyl-2-(R)-azétidinylméthoxy)pyridine ;
la 5-éthyl-6-méthyl-3-(2-(S)-azétidinylméthoxy)pyridine ;
la 5-éthyl-6-méthyl-3-(2-(R)-azétidinylméthoxy)pyridine ;
la 5-éthyl-6-méthyl-3-(1-méthyl-2-(S)-azétidinylméthoxy)pyridine ;
la 5-éthyl-6-méthyl-3-(1-méthyl-2-(R)-azétidinylméthoxy)pyridine ;
la 5-éthyl-6-fluoro-3-(2-(S)-azétidinylméthoxy)pyridine ;
la 5-éthyl-6-fluoro-3-(2-(R)-azétidinylméthoxy)pyridine ;
la 5-éthyl-6-fluoro-3-(1-méthyl-2-(S)-azétidinylméthoxy)pyridine ;
la 5-éthyl-6-fluoro-3-(1-méthyl-2-(R)-azétidinylméthoxy)pyridine ;
la 5-propyl-6-chloro-3-(2-(S)-azétidinylméthoxy)pyridine ;
la 5-propyl-6-chloro-3-(2-(R)-azétidinylméthoxy)pyridine ;
la 5-propyl-6-chloro-3-(1-méthyl-2-(S)-azétidinylméthoxy)pyridine ;
la 5-propyl-6-chloro-3-(1-méthyl-2-(R)-azétidinylméthoxy)pyridine ;
la 5-méthyl-6-fluoro-3-(2-(R)-azétidinylméthoxy)pyridine ;
la 5-méthyl-6-fluoro-3-(1-méthyl-2-(S)-azétidinylméthoxy)pyridine ;
la 5-méthyl-6-fluoro-3-(1-méthyl-2-(R)-azétidinylméthoxy)pyridine ;
la 5-méthyl-6-chloro-3-(2-(R)-azétidinylméthoxy)pyridine ;
la 5-méthyl-6-chloro-3-(1-méthyl-2-(S)-azétidinylméthoxy)pyridine ;
la 5-métbyl-6-chloro-3-(1-méthyl-2-(R)-azétidinylméthoxy)pyridine ;
la 5,6-diméthyl-3-(2-(R)-azétidinylméthoxy)pyridine ;
la 5,6-diméthyl-3-(1-méthyl-2-(S)-azétidinylméthoxy)pyridine ;
la 5,6-diméthyl-3-(1-méthyl-2-(R)-azétidinylméthoxy)pyridine ;
la 6-fluoro-5-méthoxy-3-(2-(S)-azétidinylméthoxy)pyridine ;
la 6-fluoro-5-méthoxy-3-(2-(R)-azétidinylméthoxy)pyridine ;
la 6-fluoro-5-méthoxy-3-(1-méthyl-2-(S)-azétidinylméthoxy)pyridine ;
la 6-fluoro-5-méthoxy-3-(1-méthyl-2-(R)-azétidinylméthoxy)pyridine ;
la 6-fluoro-5-éthoxy-3-(2-(S)-azétidinylméthoxy)pyridine ;
la 6-fluoro-5-éthoxy-3-(2-(R)-azétidinylméthoxy)pyridine ;
la 6-fluoro-5-éthoxy-3-(1-méthyl-2-(S)-azétidinylméthoxy)pyridine ; et
la 6-fluoro-5-éthoxy-3-(1-méthyl-2-(R)-azétidinylméthoxy)pyridine ; ou son sel ou son ester acceptable du point de vue pharmaceutique.

8. Composé tel que défini dans la revendication choisi dans le groupe constitué par
la 5-bromo-6-chloro-3-(2-(S)-pyrrolidinylméthoxy)pyridine ;
la 5-bromo-6-chloro-3-(1-méthyl-2-(S)-pyrrolidinylméthoxy)pyridine ;
la 5-bromo-6-chloro-3-(2-(R)-pyrrolidinylméthoxy)pyridine ;
la 5-bromo-6-chloro-3-(1-méthyl-2-(R)-pyrrolidinylméthoxy)pyridine ;
la 5,6-dichloro-3-(2-(R)-pyrrolidinylméthoxy)pyridine ;
la 5,6-dichloro-3-(1-méthyl-2-(R)-pyrrolidinylméthoxy)pyridine ;
la 5,6-dichloro-3-(2-(S)-pyrrolidinylméthoxy)pyridine ;
la 5,6-dichloro-3-(1-méthyl-2-(S)-pyrrolidinylméthoxy)pyridine ;
la 5-éthyl-6-chloro-3-(2-(S)-pyrrolidinylméthoxy)pyridine ;
la 5-nitro-6-méthyl-3-(2-(S)-pyrrolidinylméthoxy)pyridine ;
la 5-nitro-6-méthyl-3-(2-(R)-pyrrolidinylméthoxy)pyridine ;
la 5-nitro-6-méthyl-3-(1-méthyl-2-(S)-pyrrolidinylméthoxy)pyridine ;
la 5-nitro-6-méthyl-3-(1-méthyl-2-(R)-pyrrolidinylméthoxy)pyridine ;
la 5-amino-6-méthyl-3-(2-(S)-pyrrolidinylméthoxy)pyridine ;
la 5-amino-6-méthyl-3-(2-(R)-pyrrolidinylméthoxy)pyridine ;
la 5-amino-6-méthyl-3-(1-méthyl-2-(S)-pyrrolidinylméthoxy)pyridine ;
la 5-amino-6-méthyl-3-(1-méthyl-2-(R)-pyrrolidinylméthoxy)pyridine ;
la 5-bromo-6-méthyl-3-(2-(S)-pyrrolidinylméthoxy)pyridine ;
la 5-bromo-6-méthyl-3-(2-(R)-pyrrolidinylméthoxy)pyridine ;
la 5-bromo-6-méthyl-3-(1-méthyl-2-(S)-pyrrolidinylméthoxy)pyridine ;
la 5-bromo-6-méthyl-3-(1-méthyl-2-(R)-pyrrolidinylméthoxy)pyridine ;
la 5-bromo-6-fluoro-3-(2-(S)-pyrrolidinylméthoxy)pyridine ;
la 5-bromo-6-fluoro-3-(2-(R)-pyrrolidinylméthoxy)pyridine ;
la 5-bromo-6-fluoro-3-(1-méthyl-2-(S)-pyrrolidinylméthoxy)pyridine ;
la 5-bromo-6-fluoro-3-(1-méthyl-2-(R)-pyrrolidinylméthoxy)pyridine ;
la 5-cyano-6-méthyl-3-(2-(S)-pyrrolidinylméthoxy)pyridine ;
la 5-cyano-6-méthyl-3-(2-(R)-pyrrolidinylméthoxy)pyridine ;
la 5-cyano-6-méthyl-3-(1-méthyl-2-(S)-pyrrolidinylméthoxy)pyridine ;
la 5-cyano-6-méthyl-3-(1-méthyl-2-(R)-pyrrolidinylméthoxy)pyridine ;
la 5-cyano-6-fluoro-3-(2-(S)-pyrrolidinylméthoxy)pyridine ;
la 5-cyano-6-fluoro-3-(2-(R)-pyrrolidinylméthoxy)pyridine ;
la 5-cyano-6-fluoro-3-(1-méthyl-2-(S)-pyrrolidinylméthoxy)pyridine ;
la 5-cyano-6-fluoro-3-(1-méthyl-2-(R)-pyrrolidinylméthoxy)pyridine ;
la 5-cyano-6-chloro-3-(2-(S)-pyrrolidinylméthoxy)pyridine ;
la 5-cyano-6-chloro-3-(2-(R)-pyrrolidinylméthoxy)pyridine ;
la 5-cyano-6-chloro-3-(1-méthyl-2-(S)-pyrrolidinylméthoxy)pyridine ;
la 5-cyano-6-chloro-3-(1-méthyl-2-(R)-pyrrolidinylméthoxy)pyridine ;
la 5-carbamoyl-6-méthyl-3-(2-(S)-pyrrolidinylméthoxy)pyridine ;
la 5-carbamoyl-6-méthyl-3-(2-(R)pyrrolidinylméthoxy)pyridine ;
la 5-carbamoyl-6-méthyl-3-(1-méthyl-2-(S)-pyrrolidinylméthoxy)pyridine ;
la 5-carbamoyl-6-méthyl-3-(1-méthyl-2-(R)-pyrrolidinylméthoxy)pyridine ;
la 5-carbamoyl-6-fluoro-3-(2-(S)-pyrrolidinylméthoxy)pyridine ;
la 5-carbamoyl-6-fluoro-3-(2-(R)pyrrolidinylméthoxy)pyridine ;
la 5-carbamoyl-6-fluoro-3-(1-méthyl-2-(S)-pyrrolidinylméthoxy)pyridine ;
la 5-carbamoyl-6-fluoro-3-(1-méthyl-2-(R)-pyrrolidinylméthoxy)pyridine ;
la 5-carbamoyl-6-chloro-3-(2-(S)-pyrrolidinylméthoxy)pyridine ;
la 5-carbamoyl-6-chloro-3-(2-(R)pyrrolidinylméthoxy)pyridine ;
la 5-carbamoyl-6-chloro-3-(1-méthyl-2-(S)-pyrrolidinylméthoxy)pyridine ;
la 5-carbamoyl-6-chloro-3-(1-méthyl-2-(R)-pyrrolidinylméthoxy)pyridine ;
la 5-amino-6-fluoro-3-(2-(S)-pyrrolidinylméthoxy)pyridine ;
la 5-amino-6-fluoro-3-(2-(R)pyrrolidinylméthoxy) pyridine ;
la 5-amino-6-fluoro-3-(1-méthyl-2-(S)-pyrrolidinylméthoxy)pyridine ;
la 5-amino-6-fluoro-3-(1-méthyl-2-(R)-pyrrolidinylméthoxy)pyridine;
la 5-amino-6-chloro-3-(2-(S)-pyrrolidinylméthoxy)pyridine ;
la 5-amino-6-chloro-3-(2-(R)pyrrolidinylméthoxy)pyridine ;
la 5-amino-6-chloro-3-(1-méthyl-2-(S)-pyrrolidinylméthoxy)pyridine ;
la 5-amino-6-chloro-3-(1-méthyl-2-(R)-pyrrolidinylméthoxy)pyridine;
la 5-formamido-6-méthyl-3-(2-(S)-pyrrolidinylméthoxy)pyridine ;
la 5-formamido-6-méthyl-3-(2-(R)pyrrolidinylméthoxy)pyridine ;
la 5-formamido-6-méthyl-3-(1-méthyl-2-(S)-pyrrolidinylméthoxy)pyridine ;
la 5-formamido-6-méthyl-3-(1-méthyl-2-(R)-pyrrolidinylméthoxy)pyridine ;
la 5-acétamido-6-fluoro-3-(2-(S)-pyrrolidinylméthoxy)pyridine ;
la 5-acétamido-6-fluoro-3-(2-(R)pyrrolidinylméthoxy)pyridine ;
la 5-acétamido-6-fluoro-3-(1-méthyl-2-(S)-pyrrolidinylméthoxy)pyridine ;
la 5-acétamido-6-fluoro-3-(1-méthyl-2-(R)-pyrrolidinylméthoxy)pyridine;
la 5-acétamido-6-chloro-3-(2-(S)-pyrrolidinylméthoxy)pyridine ;
la 5-acétamido-6-chloro-3-(2-(R)pyrrolidinylméthoxy)pyridine ;
la 5-acétamido-6-chloro-3-(1-méthyl-2-(S)-pyrrolidinylméthoxy)pyridine ;
la 5-acétamido-6-chloro-3-(1-méthyl-2-(R)-pyrrolidinylméthoxy)pyridine ;
la 5-méthylamino-6-méthyl-3-(2-(S)-pyrrolidinylméthoxy)pyridine ;
la 5-méthylamino-6-méthyl-3- (2-(R)pyrrolidinylméthoxy)pyridine ;
la 5-méthylamino-6-méthyl-3-(1-méthyl-2-(S)-pyrrolidinylméthoxy)pyridine ;
la 5-méthylamino-6-méthyl-3-(1-méthyl-2-(R)-pyrrolidinylméthoxy)pyridine ;
la 5-éthylamino-6-fluoro-3-(2-(S)-pyrrolidinylméthoxy)pyridine ;
la 5-éthylamino-6-fluoro-3-(2-(R)pyrrolidinylméthoxy)pyridine ;
la 5-éthylamino-6-fluoro-3-(1-méthyl-2-(S)-pyrrolidinylméthoxy)pyridine ;
la 5-éthylamino-6-fluoro-3-(1-méthyl-2-(R)-pyrrolidinylméthoxy)pyridine ;
la 5-éthylamino-6-chloro-3-(2-(S)-pyrrolidinylméthoxy)pyridine ;
la 5-éthylamino-6-chloro-3-(2-(R)pyrrolidinylméthoxy) pyridine ;
la 5-éthylamino-6-chloro-3-(1-méthyl-2-(S)-pyrrolidinylméthoxy)pyridine ;
la 5-éthylamino-6-chloro-3-(1-méthyl-2-(R)-pyrrolidinylméthoxy)pyridine ;
la 5-éthyl-6-méthyl-3-(2-(S)-pyrrolidinylméthoxy)pyridine ;
la 5-éthyl-6-méthyl-3-(2-(R)pyrrolidinylméthoxy)pyridine ;
la 5-éthyl-6-méthyl-3-(1-méthyl-2-(S)-pyrrolidinylméthoxy)pyridine ;
la 5-éthyl-6-méthyl-3-(1-méthyl-2-(R)-pyrrolidinylméthoxy)pyridine ;
la 5-éthyl-6-fluoro-3-(2-(S)-pyrrolidinylméthoxy)pyridine ;
la 5-éthyl-6-fluoro-3-(2-(R)-pyrrolidinylméthoxy)pyridine ;
la 5-éthyl-6-fluoro-3-(1-méthyl-2-(S)-pyrrolidinylméthoxy)pyridine ;
la 5-éthyl-6-fluoro-3-(1-méthyl-2-(R)-pyrrolidinylméthoxy)pyridine ;
la 5-propyl-6-chloro-3-(2-(S)-pyrrolidinylméthoxy)pyridine ;
la 5-propyl-6-chloro-3-(2-(R)-pyrrolidinylméthoxy)pyridine ;
la 5-propyl-6-chloro-3-(1-méthyl-2-(S)-pyrrolidinylméthoxy)pyridine ;
la 5-propyl-6-chloro-3-(1-méthyl-2-(R)-pyrrolidinylméthoxy)pyridine ;
la 5-méthyl-6-fluoro-3-(2-(S)-pyrrolidinylméthoxy)pyridine ;
la 5-méthyl-6-fluoro-3-(2-(R)-pyrrolidinylméthoxy)pyridine ;
la 5-méthyl-6-fluoro-3-(1-méthyl-2-(S)-pyrrolidinylméthoxy)pyridine ;
la 5-méthyl-6-fluoro-3-(1-méthyl-2-(R)-pyrrolidinylméthoxy)pyridine ;
la 5-méthyl-6-chloro-3-(2-(S)-pyrrolidinylméthoxy)pyridine ;
la 5-méthyl-6-chloro-3-(2-(R)-pyrrolidinylméthoxy)pyridine ;
la 5-méthyl-6-chloro-3-(1-méthyl-2-(S)-pyrrolidinylméthoxy)pyridine ;
la 5-méthyl-6-chloro-3-(1-méthyl-2-(R)-pyrrolidinylméthoxy)pyridine ;
la 5,6-diméthyl-3-(2-(S)-pyrrolidinylméthoxy)pyridine ;
la 5,6-diméthyl-3-(2-(R)-pyrrolidinylméthoxy)pyridine ;
la 5,6-diméthyl-3-(l-méthyl-2-(S)-pyrrolidinylméthoxy)pyridine ;
la 5,6-diméthyl-3-(1-méthyl-2-(R)-pyrrolidinylméthoxy)pyridine ;
la 6-fluoro-5-méthoxy-3-(2-(S)-pyrrolidinylméthoxy)pyridine ;
la 6-fluoro-5-méthoxy-3-(2-(R)-pyrrolidinylméthoxy)pyridine ;
la 6-fluoro-5-méthoxy-3-(1-méthyl-2-(S)-pyrrolidinylméthoxy)-pyridine ;
la 6-fluoro-5-méthoxy-3-(1-méthyl-2-(R)-pyrrolidinylméthoxy)pyridine ;
la 6-fluoro-5-éthoxy-3-(2-(S)-pyrrolidinylméthoxy)pyridine ;
la 6-fluoro-5-éthoxy-3-(2-(R)-pyrrolidinylméthoxy)pyridine ;
la 6-fluoro-5-éthoxy-3-(1-méthyl-2-(S)-pyrrolidinylméthoxy)-pyridine ;
la 6-fluoro-5-éthoxy-3-(1-méthyl-2-(R)-pyrrolidinylméthoxy)pyridine ; ou son sel ou son ester acceptable du point de vue pharmaceutique.

9. Composition pharmaceutique comprenant une quantité d'un composé tel que défini dans la revendication 1 efficace pour réguler la transmission synaptique chez un mammifère en combinaison avec un véhicule acceptable du point de vue pharmaceutique.

10. Composé selon la revendication 1 pour l'utilisation comme agent thérapeutique.

11. Utilisation d'un composé tel que défini dans la revendication 1 pour la fabrication d'un médicament pour réguler la transmission synaptique chez un mammifère.
